(19) Europäisches Patentamt
European Patent Office
Office européen des brevets

(11) Publication number: **0 496 836 B1**

(12) # EUROPEAN PATENT SPECIFICATION

(45) Date of publication of patent specification: **14.06.95**

(51) Int. Cl.[6]: **C07K 1/08**, C07D 209/16, C07D 209/20, C07C 323/14, C07C 321/14, C07C 317/14, C07C 317/04, C07C 271/10, C07C 271/34, C07C 317/10, C07C 317/18, C07C 321/20

(21) Application number: **91900571.0**

(22) Date of filing: **22.10.90**

(86) International application number: **PCT/US90/06061**

(87) International publication number: **WO 91/05564 (02.05.91 91/10)**

(54) **SYNTHESIS AND USE OF AMINO ACID FLUORIDES AS PEPTIDE COUPLING REAGENTS.**

(30) Priority: **23.10.89 US 426121**

(43) Date of publication of application: **05.08.92 Bulletin 92/32**

(45) Publication of the grant of the patent: **14.06.95 Bulletin 95/24**

(84) Designated Contracting States: **AT BE CH DE DK ES FR GB IT LI NL SE**

(56) References cited:
**US-A- 4 575 541**
**US-A- 4 743 612**

**TETRAHEDRON LETTERS. vol. 28, no. 43, 1987, OXFORD GB pages 5099 - 5102 S RAJESWARI ET AL. 'a new synthesis of amides from acyl fluorides and n-silylamines'**

(73) Proprietor: **RESEARCH CORPORATION TECHNOLOGIES, INC.**
**101 N. Wilmot Road, Suite 600**
**Tucson**
**Arizona 85711 (US)**

(72) Inventor: **CARPINO, Louis, A.**
**11 Mount Pleasant**
**Amherst, MA 01002 (US)**
Inventor: **SADAT-AALAEE, Dean**
**27 Squire Village**
**Sunderland, MA 01375 (US)**

(74) Representative: **Hansen, Bernd, Dr.rer.nat. et al**
**Hoffmann, Eitle & Partner**
**Patentanwälte**
**Postfach 81 04 20**
**D-81904 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 496 836 B1

SYNTHESIS. August 1973, STUTTGART DE pages 487 - 488 G A OLAH ET AL. 'synthetic methods and reactions ; IV. Fluorination of carboxylic acids with cyanuric fluoride'

TETRAHEDRON, (INCL. TETRAHEDRON REPORTS) vol. 42, no. 13, 1986, OXFORD GB pages 3503 - 3519 C PICARD ET AL. 'Synthése de dilactones macrocycliques assistée par les organostanniques. Application aux macrocycles soufrés (sulfures, sulfoxides, disulfures) complexation selective par l ion Ca++'

JOURNAL OF THE AMERICAN CHEMICAL SOCIETY. vol. 112, no. 26, 19 December 1990, GASTON, PA US pages 9651 - 9652 L A CARPINO ET AL. '((9-fluorenylmethyl)oxy)carbonyl (FMOC) amino acid fluorides. Convenient new peptide coupling reagents applicable to the FMOC/tert-butyl strategy for solution and solid-phase syntheses'

J. Org. Chem.Vol.55, No.2 issue 1990,Carpino et al.,"Rapid Continuous Peptide Synthesis via FMOC Amino Acid Chloride Coupling and 4-(Aminoethyl) piperidine Deblocking",pages 721-728, SEE ENTIRE DOCUMENT

Chemical Abstracts,Vol. 100,issued 1984,Carpino et al., "Peptide synthesis and amino acid blocking agents", see entire document,abstract no. 103899n.

Chemical Abstracts. Vol. 102 issued 1985,Carpino et al.,"Peptides utilising thioxanthylmethyloxycarbonyl dioxides " see entire document,abstract no.102:25040a.

J. Org. Chem; Volume 51, No. 19,issued 1986.Carpino et al."((9-FluoroenylMethyl) Oxy) Carbonyl (FMOC) Amino Acid Chlorides.Synthesis Characterisation, and Application to the Rapid Synthesis of Short Peptide Segments.", see entire document.

## Description

The present invention relates to novel amino acid fluorides and protected amino acid fluorides and their use in synthetic biochemistry, including peptide syntheses. More particularly, this invention is directed to the N-protected amino acid fluorides, and free amino acid fluorides and the hydrogen fluoride salts thereof, the side chain of which may be unprotected or protected with a blocking group and their use thereof in peptide synthesis.

As more and more polypeptides become of medicinal importance, there is an increasing incentive to improve the methods by which they may be synthesized. In recent years, peptides which have been found to be of possible pharmacological importance include those active against various diseases, such as cancers, diabetes, and plant toxins, etc. Others have shown specific activity as growth promoters or suppressants, antibiotics, insecticides, contraceptives, anti-hypertensives, sleep-inducers, anti-depressants, analgesics, etc. The list is long and varied.

Currently, syntheses of peptides in solution by classic or various repetitive methods or on a solid support (Merrifield) are popular techniques. Solution methods have the advantages of being easily monitored and allowing purification of intermediates, if necessary, at any stage. A major drawback is the relative slow pace of the synthesis with each step being carried out manually.

The major advantage of the Merrifield Method is its easy automation so that unattended, computed-controlled machine syntheses is possible. Unfortunately, this method suffers from an inherent deficiency due to the insoluble nature of the support on which the synthesis proceeds. Unless each acylation step occurs with 100% efficiency, mixtures will inevitably be built up on the polymer. The longer the chain, the greater will be the contamination due to undesired side reactions. Products produced in such reactions remain to contaminate the desired product when at the end of the cycle it is removed from the polymeric matrix. The properties of these peptides will not differ sufficiently for peptides of greater than about 30-40 residues to make efficient separation feasible.

For very long segments (50 or more amino acids), therefore, current methods are not satisfactory. Often mixtures are obtained of such forbidding complexity that it may be difficult or impossible to isolate the desired peptide.

The problems enumerated hereinabove could be eliminated if the proper derivatives of the underlying amino acids and the proper reaction conditions could be found.

For example, FMOC, (Nα-(9-fluorenylmethyl)oxycarbonyl), protected amino acid chlorides, which are described by Carpino, et al. in J. Org. Chem. 51, 3732 (1986) have been used as acylating agent for stepwise peptide syntheses for both solution and solid phase techniques.

However, the amino acid chlorides have major drawbacks associated therewith. First, the acid chlorides react with trace amounts of water, such as moisture in the air, to give the corresponding amino acid. Therefore, they are not so stable, and as such, they are not a prime candidate for long term storage. Consequently, an objective was to find an amino acid derivative which was stable to moisture.

Moreover, another problem associated with amino acid chlorides is that it has not been possible to date to synthesize amino acid chlorides in which the protecting groups on the side chains of the amino acids can be removed under extremely mild conditions. As one skilled in the art is well aware, many of the amino acids have functional groups on the side chains which can interfere with peptide formation unless otherwise protected. In peptide synthesis, only the mildest conditions should be used to remove these protecting groups. For example, one of the easiest protecting groups to remove from the side chains containing amino, hydroxyl or carboxyl functions, such as lysine, tyrosine, threonine, serine, aspartic acid, glutamic acid and the like, is t-butyl or t-butyl containing moieties. For example, trifluoracetic acid can easily remove the t-butyl group from a serine side chain; on the other hand, a benzyl protecting group on the side chain can not be removed by said treatment but instead requires a more potent acid such as HF or trifluoromethanesulfonic acid. Therefore, the conditions for removing the benzyl group from the side chain are much harsher relative to the t-butyl groups. Furthermore, the mild catalytic hydrogenolysis of benzyl groups is not generally applicable to long chain peptides or resin attached peptides.

Although benzyl groups on the side chains of N-protected amino acid chloricles, can be prepared, such as FMOC-cysteine-S-benzyl chloride, FMOC-lysine-α-carbobenzoxy chloride, FMOC-tyrosine-O-benzyl chloride, FMOC-serine-O-benzyl chloride and FMOC aspartic acid -benzyl ester, these molecules suffer from the disadvantages described hereinabove. Consequently, an investigation was commenced to determine if t-butyl or "t-butyl like" containing groups can be used to protect the side chain of amino acid chlorides. Unfortunately, efforts in this area were unsuccessful. None of the above compounds could be synthesized if the t-butyl group was used in place of the benzyl substitution.

This was not unusual since it is well known that t-butyl-based protecting groups are readily deblocked by hydrogen chloride which is an inevitable by-product of acid chloride formation and/or long term storage (hydrolysis by trace amounts of water). For example, in the case of the FMOC-tyrosine derivative 1, the acid chloride could be obtained, but after several days it was noted to lose the t-butyl group slowly.

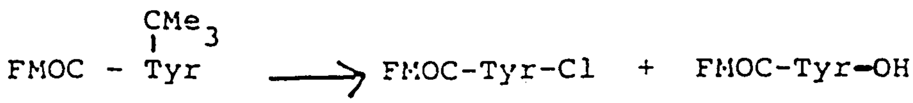

1 2 3

Furthermore, compound 1 as well as the analogous serine and threonine derivatives could be obtained only as oils which could not be crystallized and were therefore difficult, if not impossible, to purify.

In the case of the FMOC aspartic acid derivative 4, treatment with thionyl chloride gave only the aspartic acid anhydride 6, presumably via the unstable acid chloride 5 which undergoes intramolecular loss of t-butyl chloride.

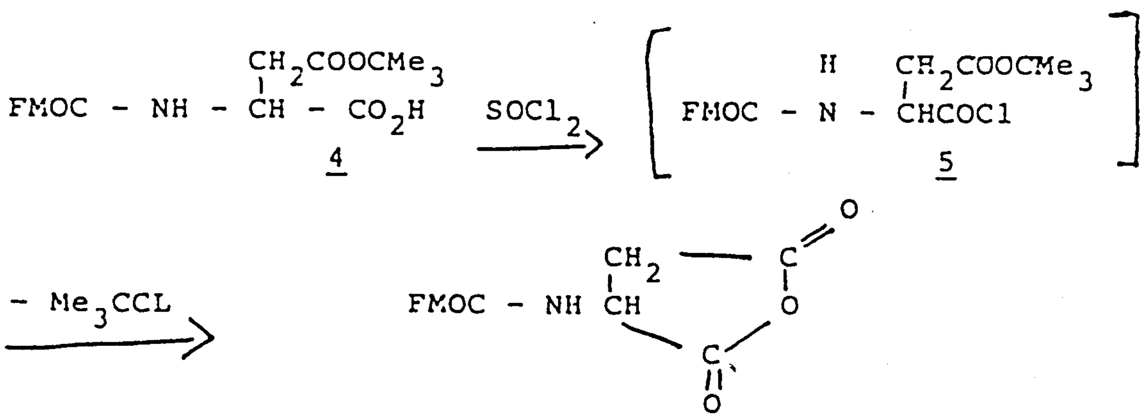


6

Lysine derivative 7 could not be converted to an acid chloride because of the marked sensitivity of the BOC function.

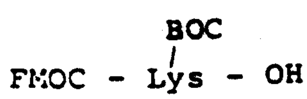

7

Similar problems arise in the cases of Arg, His, Asn, Gln, and Trp. The net result of these problems is that only about one half of the commonly occurring amino acids can be converted to stable amino acid chlorides.

Therefore, a search was undertaken to find an amino acid candidate for use in peptide synthesis which is inexpensive, stable to moisture, and which shows great potential for long-term storage. Moreover, it was hoped that a candidate could be found where the protecting groups on the amino acid side chain could be removed under milder conditions then those used to remove the benzyl group. Preferably, it was hoped that a t-butyl containing group or a group as easily removable as t-butyl could be placed on the side chain of these amino acid candidates.

The present invention circumvents the difficulties experienced with respect to the acid chlorides and accomplishes the goals described hereinabove. The compounds of the present invention are effective in

coupling with amino acids or peptides to form new peptide bonds. Moreover, the compounds of the present invention are more stable to moisture then the acid chlorides and therefore can be used for long term storage. Furthermore, t-butyl containing protecting groups and other protecting groups can be placed on the side chains of those amino acid compounds and removed under milder conditions than those required for the removal of benzyl groups. Finally, the compounds of the present invention are potent acylating agents in peptide bond formation.

These compounds are, much to our surprise, the corresponding amino acid fluorides.

The present invention is directed to an amino acid of the formula:

$$\mathrm{BLK-}\overset{\mathrm{X}}{\overset{|}{\mathrm{AA}}}\mathrm{\ -\ F}$$

or the hydrogen fluoride salts thereof
wherein

BLK is hydrogen or an N-amino protecting group;

AA is an amino acid residue; and

X is - or a protecting group.

The present invention is also directed to a method for preparing a peptide which comprises reacting the amino acid fluoride described hereinabove with an amino acid or peptide having a free amino group and removing the protecting groups therefrom.

As used herein, the term "amino acid" refers to an organic acid containing both a basic amino group ($NH_2$) and an acidic carboxyl group (COOH). Therefore, said molecule is amphoteric and exists in aqueous solution as dipole ions. (See, "The Condensed Chemical Dictionary", 10th ed. edited by Gessner G. Hawly, Van Nostrand Reinhold Company, London, Eng. p.48 (1981)). The preferred amino acids are the $\alpha$-amino acids. They include but are not limited to the 25 amino acids that have been established as protein constituents. They must contain at least one carboxyl group and one primary or secondary amino group on the amino acid molecule. They include such proteinogenic amino acids as alanine, valine, leucine, isoleucine, norleucine, proline, hydroxyproline, phenylalanine, tryptophan, methionine, glycine, serine, threonine, cysteine, cystine, tyrosine, asparagine, glutamine, aspartic acid, glutamic acid, lysine, hydroxylysine, ornithine, arginine, histidine, penicillamine and the like.

An "amino acid residue", as defined herein, is an amino acid minus an amine hydrogen on the amino end of the molecule and the OH group on the carboxy end of the molecule (i.e., it includes the acyl group

$$\underset{\mathrm{O}}{\underset{\|}{\mathrm{C}}}$$

on the carboxy end of the molecule). Therefore, unless designated to the contrary, the group "AA" signifies an amino acid residue. For example, the amino acid residues of various amino acids are represented below:

| AA Symbol | AA Residue |
|---|---|
| Gly | 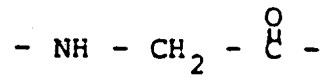 |
| Ala | 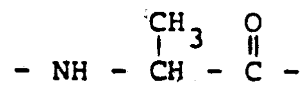 |
| Leu | 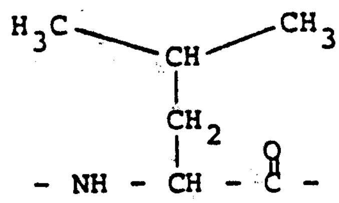 |
| Ile | 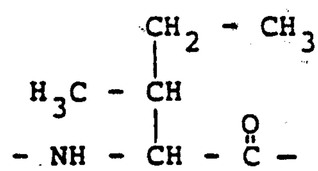 |

Pro

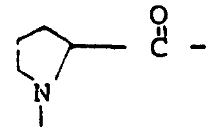

Phe

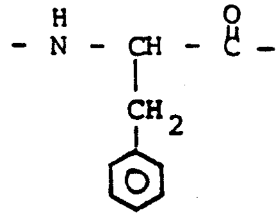

Trp

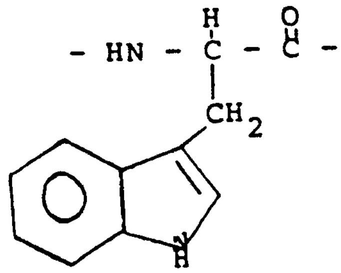

Met

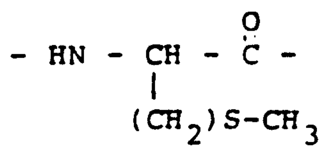

Ser

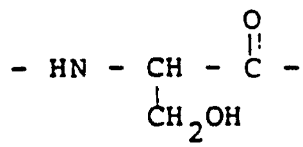

Thr

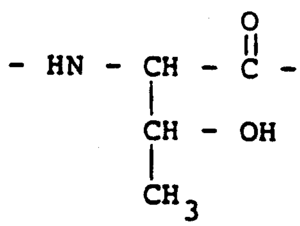

Cys

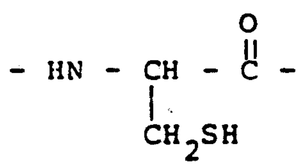

Tyr

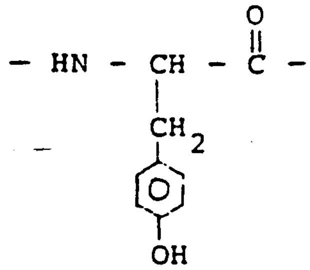

Asn

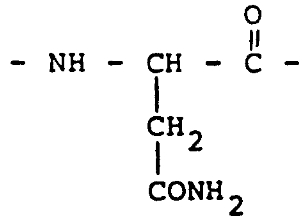

Gln

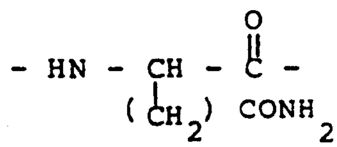

Asp

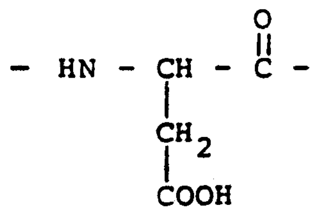

Glu

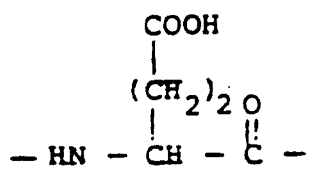

Lys

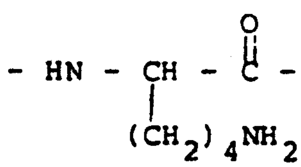

Arg

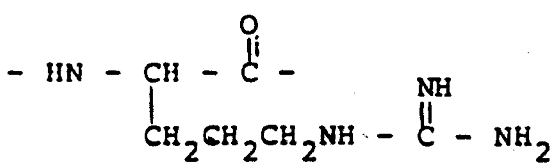

His

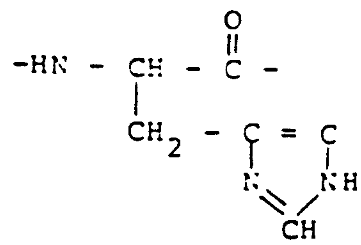

Nor

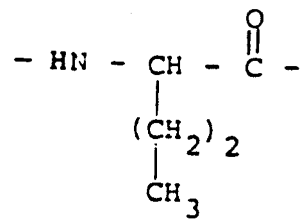

Therefore, the symbol "AA-F" refers to an amino acid fluoride, i.e., a compound having a fluoro group attached to the acyl group

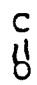

of the amino acid. When BLK is hydrogen, then the structure becomes the amino acid fluoride of an amino acid having a free amino group. However, in view of the synthesis of the amino acid fluoride described hereinbelow, the free amino acid fluoride may be isolated as the hydrogen fluoride salt thereof.

It will be apparent to one skilled in the art, shown by exemplification in the table hereinabove that in the course of protein synthesis, it may be necessary to protect certain side chains of the amino acids to prevent unwanted side reactions. For example, it may be necessary to protect the hydroxyl group on the side chain of tyrosine, serine, or threonine in order to prevent these groups from interfering with the desired reactions. This is a common problem in peptide synthesis and many procedures are available for protecting the functional groups on the side chains of the amino acids. Such procedures for protecting various functional groups are known to one skilled in the art and are described in the treatise entitled "The PEPTIDES", Vol. 2, Edited by E. Gross and J. Meienhoffer, Academic Press, NY, NY, pp. 160-251 (1980), and the boor entitled "Reagents for Organic Synthesis", by T.W. Green, John Wiley and Sons, New York, 1981, the contents of both being incorporated herein by reference.

For example, then the functional side chain contains an hydroxy group, such as threonine or serine, it can be protected by such groups as methyl, methoxymethyl(MOM), 2-methoxyethoxymethyl(MEM), tetrahydropyranyl, β-trimethylsilylethyl, 4-methoxytetrahydropryanyl, 1-ethoxyethyl, t-butyl, p-methoxybenzyl, p-halobenzyl, o-nitrobenzyl, p-nitrobenzyl, o-chlorobenzyl, adamantyl, diphenylmethyl, triphenylmethyl, cyclohexyl, cyclopentyl, 1-benzyloxycarbonyl, tri-substituted silyl, wherein the substituents are independently aryl, alkyl or aralkyl, 2,2,2-trifluoroethyl, and the like. The preferred groups for the protection of the hydroxyl side chain are adamantyl, t-butyl, 4-methoxybenzyl, cyclopentyl and cyclohexyl.

When the side chain contains a phenol, such as in tyrosine, it may be protected by such groups as methyl, methoxymethyl(MOM), methoxyethoxymethyl(MEM), -trimethylsilylethyl, methylthiomethyl, tetrahydropyranyl, isopropyl, cyclohexyl, cyclopentyl, t-butyl, adamantyl, 4-methoxysilyl, o-nitrobenzyl, 2,4-dinitrophenyl, m-bromobenzyl, 2,6-dichlorobenzyl, trisubstituted-silyl wherein the substituents are independently alkyl, aryl or aralkyl, ethoxycarbonyl, carbamoyl and the like. The most preferred protecting groups are adamantyl, 4-methoxybenzyl, t-butyl, cyclopentyl, and cyclohexyl. An especially preferred protecting group is t-butyl.

A carboxy side chain, such as that found in aspartic acid or glutamic acid, can be protected by the following groups: 1-or 2-adamantyl, methoxymethyl, methythiomethyl, t-butyl, methyl, ethyl, phenyl, tetrahydropyranyl, cyclopentyl, cyclohexyl, cycloheptyl, 4-picolyl, trisubstituted-silyl wherein the substituents are independently alkyl, aryl or aralykl, N-piperidinyl, N-succinimidoyl, β-trimethylsilylethyl, 4-methoxybenzyl, benzyl, p-bromobenzyl, p-chlorobenzyl, p-nitrobenzyl, phenacyl, N-phthalimidoyl, 4-alkyl-5-oxo-1,3-oxazolidinyl, trisubstituted-stannyl wherein the substituents are independently alkyl, aryl or aralkyl, and the like. The most preferred protecting group is t-butyl.

If the functional group on the side chain is mercapto, e.g., cysteine, such groups as triphenylmethyl, benzyl, 4-methylbenzyl, 3,4-dimethylbenzyl, 4-methoxybenzyl, β-trimethylsilylethyl, p-nitrobenzyl, 4-picolyl, diphenylmethyl, triphenylmethyl, bis(4-methoxyphenyl)methyl, diphenyl-4-pyridylmethyl, 2,4-dinitrophenyl, t-butyl, t-butylthio, adamantyl, isobutoxymethyl, benzylthiomethyl, thiazolidinyl, acetamidomethyl, benzamidomethyl, 2-nitro-1-phenylethyl, 2,2-bis(carboethoxy)ethyl, 9-fluorenemethyl, acetyl, benzoyl, and the like can be used to protect said group. In this case, it is preferred that the protecting groups are t-butyl, t-butylthio, 4-methoxybenzyl, and triphenylmethyl.

If the side chain contains an amino group, such as the ε-amino group of lysine and ornithine, the following groups may be used: 9-fluorenylmethyloxycarbonyl, 9-(2-sulfo)fluorenylmethyloxycarbonyl, β-trimethylsilylethyloxycarbonyl, 2-furanylmethyloxycarbonyl, adamantyloxycarbonyl, carbobenzoxy, t-butyloxycarbonyl, t-amyloxycarbonyl, cyclobutyloxycarbonyl, 1-methylcyclobutyloxycarbonyl, cyclopentyloxycarbonyl, cyclohexyloxycarbonyl, 1-methylcyclohexyloxycarbonyl, isobornyloxycarbonyl, CBZ, p-methoxybenzyloxycarbonyl, p-nitrobenzyloxycarbonyl, chlorobenzyloxycarbonyl, isonicotinyloxycarbonyl, p-toluenesulfonylamidocarbonyl, methylsulfonylethyloxycarbonyl, β,β,β-trichloroethyloxycarbonyl, dithiasuccinoyl, phthaloyl, 4,5-diphenyl-4-oxazoline-2-one, piperidino oxycarbonyl trifluoroacetyl, chloroacetyl, p-toluenesulfonyl and the like. Preferred groups for the protection of this amino side chain are carbobenzoxycarbonyl, t-butyloxycarbonyl, and adamantyloxycarbonyl.

If the amino acid has an imidazole group, such as in histidine, the following groups may be used to protect the side chain: benzyloxymethyl, piperdinylcarbonyl, phenacyl, pivaloyloxymethyl, 1-(alkoxycarbonylamino)- 2,2,2-trifluoroethyl, 1-trifluoromethyl-1-(p-chlorophenoxymethoxy)-2,2,2-trifluoroethyl, 2,4-dinitrophenyl, toluenesulfonyl, FMOC, triphenylmethyl, t-butyloxycarbonyl, t-butyloxymethyl and the like. The preferred groups are FMOC, t-butyloxycarbonyl, triphenylmethyl, and t-butyloxymethyl.

When the amino acid has a guanidine side chain, such as in arginine, the following protecting groups can be used to protect the ω nitrogen on the guanidine moiety: methoxytrimethylbenzenesulfonyl, pentamethylchromanesulfonyl, mesitylenesulfonyl, tolunesulfonyl, 2,4,6-trimethylbenzenesulfonyl, trimethoxybenzensulfonyl, bisadamantyloxylcarbonyl, nitro, tosyl, and the like. The preferred protecting groups are methoxytrimethylsulfonyl, pentaamethylchromanesulfonyl, bisadamantyloxycarbonyl, and mesitylenesulfonyl.

For side chains containing an amide group such as in glutamine and asparagine, the following groups can be used to protect the side chain: dimethoxybenzyhydryl, 9-xanthenyl, 2,4,6-trimethoxybenzyl, and the like.

As used herein, the term "alkyl", when used alone or in combination with other groups refers to a carbon chain containing from 1 to 6 carbon atoms. They may be straight chains or branched and include such groups as methyl, ethyl, propyl, isopropyl, n-butyl, sec-butyl, isobutyl, t-butyl, n-pentyl, amyl, n-hexyl and the like. The preferred alkyl group contains from 1-3 carbon atoms. The term aryl as used herein refers to an aromatic ring system containing from 6-10 ring carbon atoms and up to a total of 15 carbon atoms. It includes such groups as phenyl, α-naphthyl, β-naphthyl, and the like. The preferred group is phenyl.

Aralkyl groups are aryl groups attached to the main chain through an alkylene bridge. Such groups include benzyl, phenethyl and the like. The preferred aralkyl group is benzyl.

The aryl and aralkyl groups herein may be unsubstituted or may be substituted with an electron donating groups in situations wherein the protecting group is cleaved by acid. An electron donating group as defined herein shall be interpreted as a group that will release or donate electrons more than hydrogen would if it occupied the same position in the molecule. See, J. Marsh, Advance Organic Chemistry, 2nd Ed., McGraw Hill, Inc. (1977). These types of groups are well known in the art. Examples of electron donating groups include alkyl, lower alkoxy, aralkoxy; and the like. These electron donating groups, e.g., alkoxy, may be present on the aryl moiety of the following groups: DMB, TMB, Mtr, Pmc, Bz, Trt, CBZ and the like.

The protecting groups described hereinabove are well known to one skilled in the art. They can be removed under very mild acidic or basic conditions. The preferred protecting groups are those which can be cleaved by acid or base under conditions which are milder than those used to cleave the benzyl group. These include groups which can be cleaved by trifluoroacetic acid at room temperature within one to four hours. The especially preferred protecting groups are groups which can be cleaved by trifluoroacetic acid at room temperature within 1-2 hours.

These protecting groups include such groups as tetrahydropyranyl, β-trimethylsilylethyl, 1-ethoxyethyl, t-butyl, p-methoxybenzyl, 1-adamantyl, diphenylmethyl, triphenylmethyl, trialkylsilyl, (e.g. trimethylsilyl, triethylsilyl, and the like) trialkylstannyl, (e.g trimethylstannyl, triethylstannyl, and the like), bis (4-methoxyphenyl)methyl, 2-furanylmethyloxycarbonyl, t-amyloxycarbonyl, 1-methylcyclohexyloxycarbonyl, isobornyloxy carbonyl, methoxytrimethylbenzenesulfonyl, pentamethylchromanesulfonyl, 2,4,6-trimethoxybenzyl, 9-xantheneyl and the like.

However, not all of the amino acids have side-chain functional groups. For example, many amino acids have hydrogen, alkyl or aralkyl side chains. These include glycine, alanine, valine leucine, norleucine, phenylalanine, isoleucine and the like. Therefore, these amino acids do not require protecting groups thereon.

To differentiate between those amino acids having protecting groups and those not having protecting groups thereon,
the term

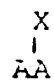

is used. As used herein, if no protecting group is present on the amino acid side chain, such as, e.g., in alanine, (which doesn't have a functional group and therefore no blocking group is required) "X" is -. Moreover, if the amino acid side chain has a functional group, such as in tyrosine, but is unprotected, then this also is indicated by X being defined as -. In other words, in both instances, when X is -, the side group is unprotected.

Although the term functional group is understood by one skilled in the art, it is defined as a group which could react with the reactants used or products formed under peptide forming condition if not protected by a blocking group. These functional groups include amino, carboxy, hydroxy, guanidino, imidazole, amino and the like.

On the other hand, if X is a blocking group, then this signifies that the functional group on the side chain is protected. For example, if AA is serine and X is t-butyl, then the residue

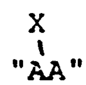

becomes

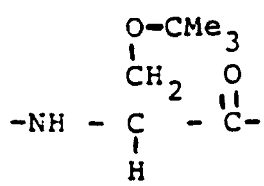

These protecting groups include the protecting groups described hereinabove.

Abbreviations have been used in the specification and claims with respect to these blocking groups and are listed hereinbelow:

| Protecting group | Abbreviation |
|---|---|
| dimethoxybenzhydryl | DMB |
| 2,4,6-trimethoxybenzyl | TMB |
| 2,3,6-trimethyl-4-methoxybenzenesulfonyl | Mtr |
| 9-fluorenylmethyloxycarbonyl | FMOC |
| t-butoxycarbonyl | BOC |
| t-butoxymethyl | Bom |
| pentamethylchromanesulfonyl | Pmc |
| adamantyl | ada |
| β-trimethylethyl | TMSE |
| β-trimethylethyloxycarbonyl | TEOC |
| t-butyl | t-bu |
| benzyl | Bz |
| cyclopentyl | Cp |
| cyclohexyl | Ch |
| triphenylmethyl | Trt |
| benzyloxycarbonyl | Cbz |
| adamantyloxycarbonyl | Adoc |
| formyl | CHO |
| trifluoroacetyl | TFA |

The term amino acid protecting group, as used herein, refers to blocking groups which are known in the art and which have been utilized to block the amino ($NH_2$) group of the amino acid. Blocking groups such as 9-lower alkyl-9-fluorenyloxycarbonyl,2-chloro-1-indanylmethoxycarbonyl (CLIMOC) and benz [f] indene-3-methyloxycarbonyl (BIMOC) and dbd-TMOC are discussed in U. S. Patent Nos. 3,835,175, 4,508,657, 3,839,396, 4,581,167, 4,394,519, 4,460,501 and 4,108,846, and the contents thereof are incorporated herein by reference as is fully set forth herein.

Moreover, other amino protecting groups can be used. These protecting groups are derived from 2-propenyloxycarbonyl compounds having the general formula I :

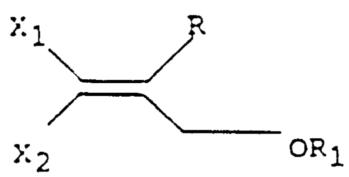

I

wherein R is an electron withdrawing group, $R_1$ is COZ, $X_1$ and $X_2$ are independently H, lower alkyl, aryl, aryl lower alkyl or a solid support or R and $X_1$ taken together with the carbon atoms to which they are attached form a ring containing from 4 to 15 ring carbon atoms and may contain up to 2 heteroatoms, wherein the heteroatoms are O, S or N; and Z is a leaving group.

Compounds or Formula I can be derived from the corresponding alcohols of the formula II:

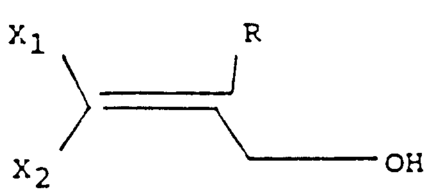

II

wherein R, $X_1$, and $X_2$ are as hereinbefore defined.

These 2-propenyloxycarbonyl compounds of formula I protect the primary or secondary amino group during chemical syntheses thereby protecting said amine from further reaction. Thus, a portion of the

organic molecule other than the protected amine is modified by chemical reaction; and, (c) subsequently are easily cleaved from the amino group.

In a preferred embodiment, the protecting group can be rapidly and cleanly deblocked via treatment with a nucleophile. The nucleophile can also act as a scavenger for the protecting group. The preferred nucleophile is a primary or secondary amine.

An electron withdrawing group as defined herein shall be interpreted as a group that will draw electrons to itself more than a hydrogen atom would if it occupied the same position in the molecule. See, J. March, Advanced Organic Chemistry, 3rd Ed., John Wiley & Sons, P.17 (1985). These types of groups are well-known to one skilled in the art.

Examples of electron withdrawing groups include $SO_2R_2$, $SOR_2$, $COOR_2$, $COR_2$, CHO, $CONR_2R_3$, CN, $CF_3$, $NO_2$, aryl, pyridyl and the like, wherein $R_2$ and $R_3$ are independently lower-alkyl having from 1 to 6 carbon atoms, aryl, aryl lower-alkyl, hetero-aryl or a solid support and the alkyl, aryl or hetero-aryl groups are unsubstituted, or mono- or di-substituted with halides, $SO_2R_2$, $SOR_2$, $COOR_2$, $COR_2$, CHO, CN, $CF_3$ or $NO_2$.

As used herein, the terms lower alkyl, when used singly or in combination, refer to alkyl groups containing one to six carbon atoms. They may be straight chain or branched and include such groups as methyl, ethyl, propyl, isopropyl, butyl, sec-butyl, isobutyl, t-butyl, pentyl, isopentyl, neopentyl, hexyl and the like. The preferred alkyl groups contain one to four carbon atoms.

The term aryl, when used alone or in combination, refer to an aromatic ring containing six to ten ring carbon atoms. The aryl group includes phenyl, and 1 or 2-naphthyl. The preferred aryl group is phenyl.

The most preferred aralkyl group is benzyl.

The term hetero-aryl when used alone or in combination refers to an hetero-aromatic ring having from four to nine carbon ring atoms and one or two hetero ring atoms. The heterocyclic rings may contain sulfur, nitrogen or oxygen. The hetero-aryl groups include quinolyl, isoquinolyl, furyl, thienyl, or pyridyl.

The preferred heteroaryl groups are 2- or 4-pyridyl.

The preferred R groups are $SO_2R_2$, $SOR_2$, $COOR_2$, $COR_2$, $CONR_2R_3$, aryl, or pyridyl, wherein $R_2$ and $R_3$ are as defined hereinabove.

Especially preferred R groups are $SO_2C(CH_3)_3$,$SOC(CH_3)_3$, $SO_2C_6H_5$, $SOC_6H_5$, 2-pyridyl, or 4-pyridyl or COOEt.

As used herein the term solid support when used singly or in combination refers to polymer resins typically used in peptide synthesis. These polymers are generally employed in the form of beads. The polymer resins preferred for peptide synthesis are polystyrenes, polyacrylamides and the like. The preferred polystyrene resin is a copolymer of styrene-divinylbenzene.

As noted hereinabove, R and $X_1$ taken together with the carbons to which they are attached may be cyclic containing one, two, or three rings. Said cyclic structure may contain from four to fifteen ring carbon atoms and up to two heteroatoms. These heterocyclic ring atoms may be sulfur, oxygen or nitrogen. For example, when R and $X_1$ taken together form a ring, the compounds of Formula I may have the formula III or its isomer IIIa

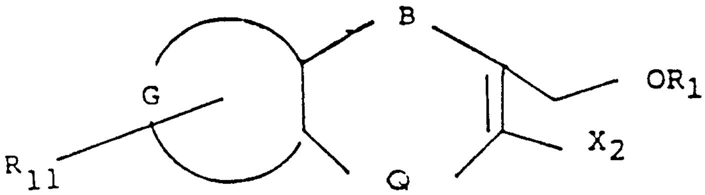

III

or

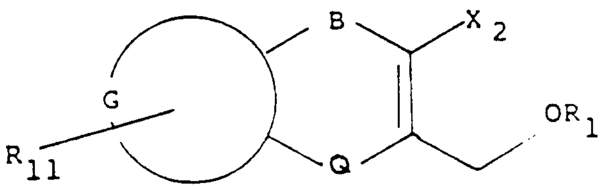

IIIa

wherein B is a chemical bond, $CR_8R_9$,

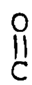,

$SO_2$, SO, RP(O), or S, R is lower alkyl or $OR_{10}$, $R_{10}$ is lower alkyl, $R_8$, $R_9$, $R_{12}$ and $R_{13}$ are independently hydrogen or lower alkyl, G is a mono or bicyclic fused ring system containing 5 to 10 carbon atoms and may contain up to 2 heteroatoms, Q is a chemical bond, $CR_{12}$, $R_{13}$, $SO_2$, SO, RP(O) or S, $R_{11}$ is hydrogen or lower alkyl and $X_2$ and $R_1$ are as defined hereinabove. G may be completely saturated, partially unsaturated or fully aromatic (e.g. a benzo system). Examples of G include cyclopentyl, cyclohexyl, cyclopentenyl, cyclohexenyl, decalinyl, phenyl, naphthyl, pyridyl azaindenyl and the like.

As defined hereinabove, when Q is a chemical bond, then III or IIIa has the structure

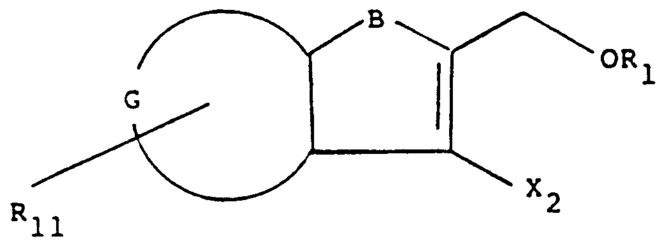

or

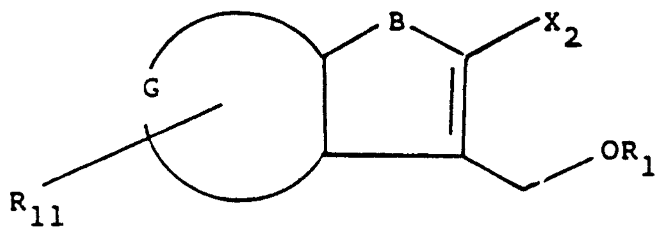

Similarly, when B is a chemical bond, then 3 or 3a has the structure

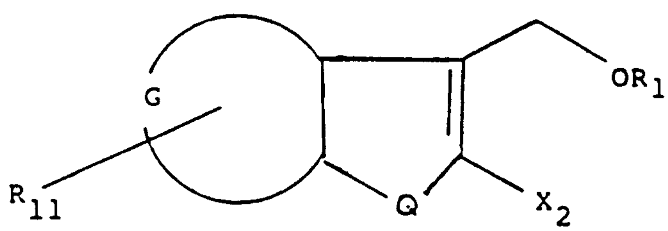

or

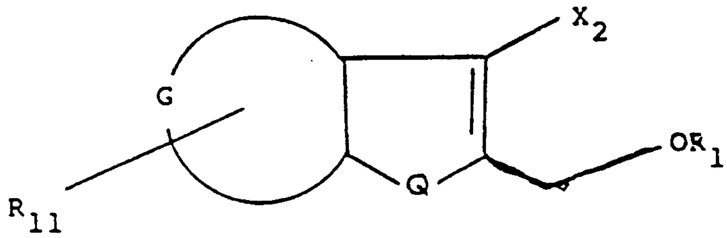

However, in order to be effective, either B or Q is an electron withdrawing group or ring G is itself electron withdrawing. In other words, if ring G is heterocyclic, i.e., contains ring heteroatoms, then B and Q are independently any of the values described hereinabove. However, if ring G is not heterocyclic (e.g., is aromatic or cycloalkyl), then at least one of B or Q is

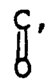

$SO_2$, SO, RP(O) or S wherein R is as defined hereinabove.

A preferred class of III or IIIa has the formula

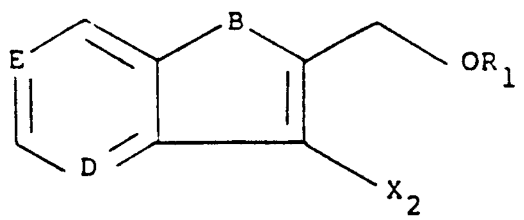

IIIb

or

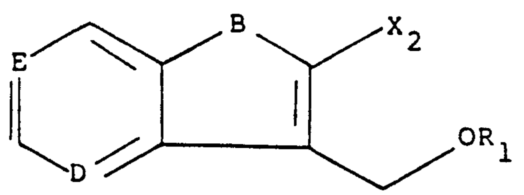

IIIc

wherein B is $CR_8R_9$ or $SO_2$ and E and D are independently CH or N, provided that when B is $CR_8R_9$, then E or D is N.

A preferred embodiment has the formula IV:

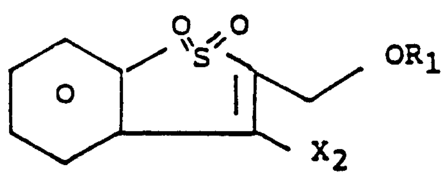

IV

or the formula IVa or IVb:

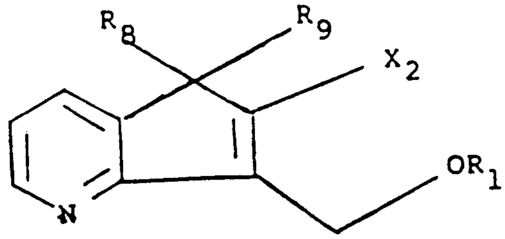

IVa

or

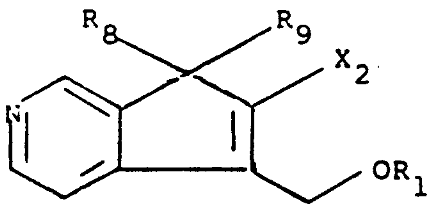

IVb

wherein $X_2$ and $R_1$ are as defined hereinabove and $R_8$ and $R_9$ are independent.

When compounds of Formula I are used to protect a primary or secondary amino group and introduce the 2-propenyloxy carbamate compound of Formula I, Z is a leaving group. As is generally known in the art and for the purposes of the present invention "a leaving group" is defined as a group which is readily broken away from its union with the carbon atoms. It is one which readily joins, for example, with an active hydrogen atom to split out a compound containing the hydrogen atom and the leaving group. Leaving groups are generally electron attracting groups either because of their electronegativity or because they have an inductive effect. Leaving groups are defined in U.S. Patent No. 4,394,519 to Carpino, et al. which is incorporated herein by reference.

The preferred leaving groups Z are halo, CN, $SR_4$, SAr, $N_3$, OAr,

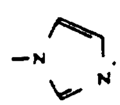
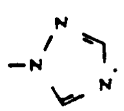
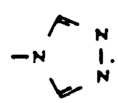
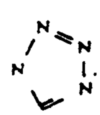
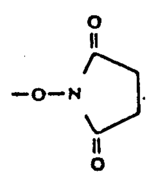
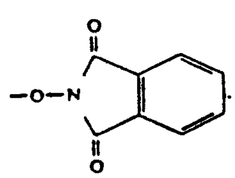
or
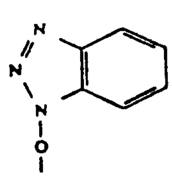

wherein $R_4$ is lower-alkyl, aryl or aryl lower-alkyl, wherein the alkyl or aryl groups are unsubstituted, or mono- or di-substituted with halides, $SO_2R_4$, $SOR_4$, $COOR_4$, $COR_4$, CHO, CN, $CF_3$ or $NO_2$.

The most preferred leaving groups are halo, especially Cl and Br.

The preferred groups in the vinyl position, $X_1$ and $X_2$, are independently H, phenyl, lower alkyl having from 1 to 4 carbon atoms or a solid support. Especially preferred groups for $X_1$ and $X_2$ are independently H or phenyl. The $X_1$, and $X_2$ positions may also be used as the site of attachment to a polymer resin, on Z, when, Z is an amino acid or peptide residue.

The preferred compounds of Formula I have the formula :

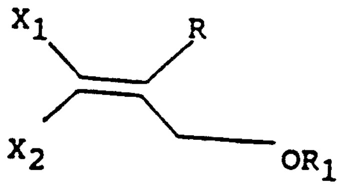

wherein R is $SO_2R_2$, $SOR_2$, $COOR_2$, $CONR_2R_3$, aryl or 2- or 4-pyridyl, wherein $R_2$ and $R_3$ are as defined hereinabove or R and $X_1$ taken together with the carbon atoms to which they are attached form a ring, the ring having from 4 to 15 ring carbon atoms and up to a total of 3 rings, often forming a fused benzo-system; $R_1$ is H or COZ and Z is halo; and $X_1$ and $X_2$ are independently H, phenyl or lower alkyl.

The preferred groups in compounds wherein R and $X_1$ are taken together with the carbon atoms to which they are attached are $SO_2C_6H_4$ or $C_5H_3NCH_2$ thereby describing compounds of the formulae IV or IVa or IVb described hereinabove:

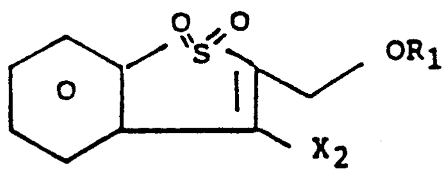

IV

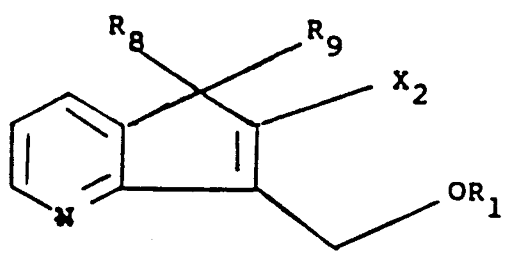

IVa

or

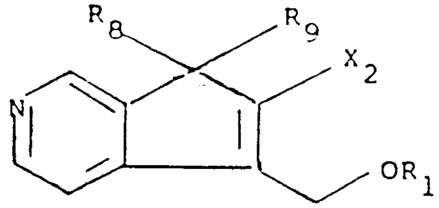

IVb

wherein $R_1$ and $X_2$, $R_8$ and $R_9$ are as defined hereinabove, and $R_8$ and $R_9$ are independently hydrogen or lower alkyl.

The most preferred compounds of Formula I are the 2-propenyloxy chloroformates wherein R is $SO_2C(CH_3)_3$, $SOC(CH_3)_3$, $SO_2C_6H_6$, $SOC_6H_6$, COOEt, 2-pyridyl or 4- pyridyl; Z is Cl or Br; and $X_1$ and $X_2$ are H or phenyl.

The preferred alcohols of Formula II are:

2-(t-butylsulfonyl)-2-propenyl alcohol,
2-carboethoxy-2-propenyl alcohol,
2-(phenylsulfonyl)-2-propenyl alcohol,
(E)-3-phenyl-2-(phenylsulfonyl)-2-propenyl alcohol,
(Z)-3-phenyl-2-(phenylsulfonyl)-2-propenyl alcohol,
3,3-diphenyl-2-(phenylsulfonyl)-2-propenyl alcohol,
Benzothiophenesulfone-2-methanol,
3,3-dimethyl-2-(phenylsulfonyl)-2-propenyl alcohol.
(Z)-3-phenyl-2-(methylsulfonyl)-2-propenyl alcohol

The preferred chloroformates of Formula I are:

2-(t-butylsulfonyl)-2-propenyl chloroformate,
2-carboethoxy-2-propenyl chloroformate,
2-(phenylsulfonyl)-2-propenyl chloroformate,
(E)-3-phenyl-2-(phenylsulfonyl)-2-propenyl chloroformate,
(Z)-3-phenyl-2-(phenylsulfonyl)-2-propenyl chloroformate, or
3,3-diphenyl-2-(phenylsulfonyl)-2-propenyl chloroformate,
Benzothiophenesulfone-2-methyl chloroformate,
3,3-dimethyl-2-(phenylsulfonyl)-2-propenylchloroformate,
(Z)-(3-phenyl)-2-methylsulfonyl-2-propenylchloroformate.

The 2-propenyloxy carbonyl compounds outlined hereinabove can be formed by art-recognized techniques known to one skilled in the art. Exemplary procedures are outlined hereinbelow.

The compounds having the formula Ia:

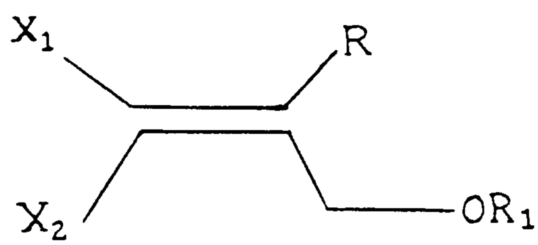

Ia

can be prepared from the corresponding alcohol II:

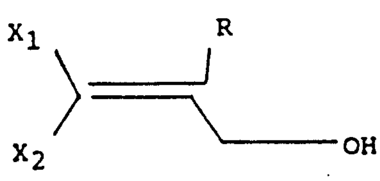

II

as illustrated in the following equations:

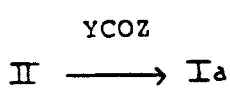


(1)

wherein

Z = Y = Cl, Br, Fl, CN;
Z = Cl, Y = $SR_4$, SAr, OAr, Fl;

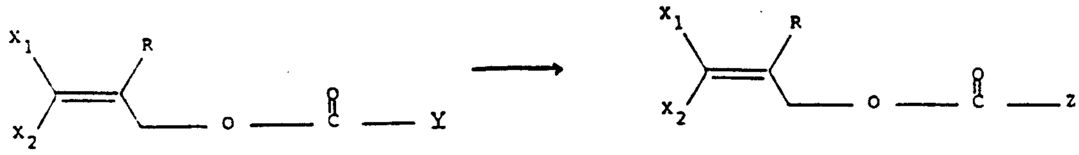

(2)

wherein Z = Cl, Br; Y = Fl, $N_3$, CN.

In the above equations, $X_1$, $X_2$, R, and $R_4$ are as defined herein.

Typically, reactions such as indicated by Equation (1) are carried out in an inert organic solvent. As defined herein an inert solvent is a reaction inert solvent, i.e., one which will not react with the reagents or reactants under the given reaction conditions. Suitable solvents are halogenated or non-halogenated hydrocarbons containing up to about eight carbon atoms, e.g., methylene chloride, ethylene dichloride, benzene, isooctane and the like. Reactions are conducted at temperatures of from about 0 °C to about 25 °C during a reaction period of from about 1 to about 6 hours. Suitable yields are obtained with equimolar quantities of reactants, although the yield may often be appreciably increased by utilizing an excess of either one of them, for example, up to about a 20% molar excess. Generally speaking, the halogen substituted compounds are prepared under less rigorous reaction conditions than are required for the preparation of those compounds wherein the substituent is of higher molecular weight. The presence of a weak base, may increase the rate of reaction.

Reactions of Equation (2) in which the substituent placed on the carbonyl carbon atom is initially present in an ionic form are carried out in inert polar organic solvents which will enhance ionization, including, for example, acetonitrile, dimethylformamide, dimethylsulfoxide, tetrahydrofuran, dioxane and the like. The reaction is normally carried out at a temperature of from about 0 °C to about 25 °C during a period of from about 1 to 5 hours. Preferably equimolar quantities of reactants are employed to minimize side reactions but a moderate excess of either reactant would not introduce appreciable difficulties.

Compounds of Formula II can be prepared by reacting an aldehyde or ketone of Formula V:

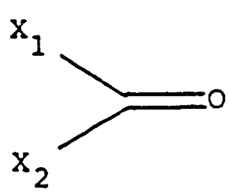

V

with a Wittig reagent, $\emptyset_3PCHR$,

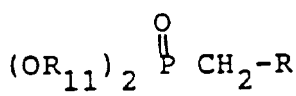

or $(R_{11})_3S_iCH_2$-R such as,

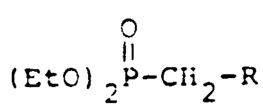

or $Me_3Si$-$CH_2$-R

where R is defined hereinabove, and $R_{11}$ is lower alkyl under Wittig reaction conditions; followed by the addition of formaldehyde under Prins reaction conditions, wherein $X_1$, $X_2$ and R are as hereinbefore described and $R_{11}$ is lower alkyl.

Compounds of Formula I wherein R contains a sulfur atom can be prepared by an alternative route. The following procedures as depicted in Scheme I is illustrative for the preparation of these compounds:

Scheme I

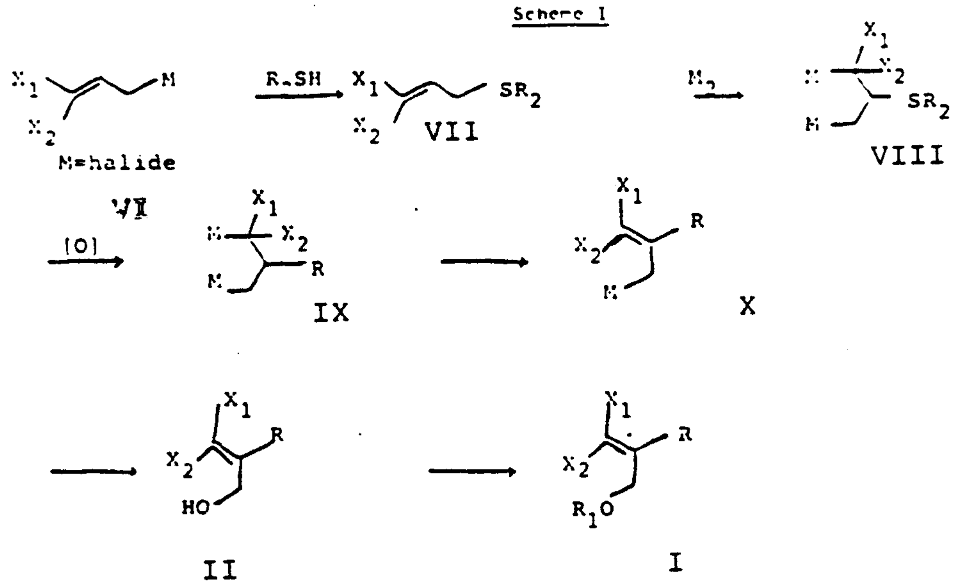


An allylic halide such as allyl bromide VI , is treated with a thiol, $R_2SH$, such as t-butylthiol in strong base to form the corresponding thioether VII. Halogenation of the thioether forms the dihalo thioether VIII which in turn is oxidized with an oxidizing agent to form the sulfonyl or sulfinyl compound IX. Various oxidizing agents can be used to effect said reaction, such as MCPBA. Compound IX is then reacted with a strong base, such as lutidine, to form the corresponding unsaturated compound X. Substitution of the halide in compound X with hydroxide forms the compound of Formula II. The compound of Formula I can be readily prepared from II according to the procedure described hereinabove.

Typically, the reactions for synthesis of the compounds described in Scheme 1 hereinabove are carried out in an inert organic solvent. Suitable solvents include alcohols, such as methanol, ethanol, isopropanol, t-butanol and the like, ethers such as diethyl ether, 1,4-dioxane, tetrahydrofuran (THF) and the like, hydrocarbons, such as benzene, hexane, cyclohexane, toluene, Skelly solvents and the like, and halogenated hydrocarbons such as $CHCl_3$, $CCl_4$, $CF_2Cl_2$ and the like.

Temperatures for these reactions range from about -78°C to the reflux temperature of the solvent being employed. Unless indicated to the contrary in the discussion of the various reaction schemes described hereinabve and hereinafter, the preferred temperatures are from about 0°C to about 100°C.

The compounds of Formula I described herein wherein Z is a leaving group protect the amino group in an amino acid during peptide synthesis. Thus, a method for the preparation of a peptide comprises:

a) reacting an amino acid having a free amino group with a compound of the formula:

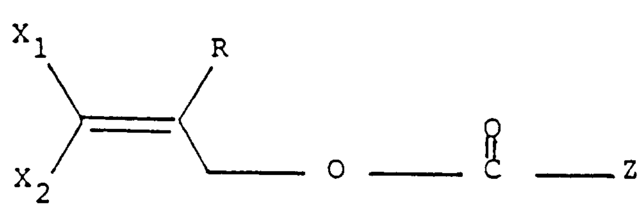

wherein

Z is a leaving group;

$X_1$ and $X_2$ are independently H, lower alkyl, aryl, aryl lower-alkyl or a solid support;

R is an electron withdrawing group; or

R and $X_1$ with the carbon atoms to which they are attached form a ring containing from 4 to 15 ring carbon atoms;

b) reacting the product of (a) with an amino acid-fluoride or peptide having a free amino group; and

c) removing the protecting group.

Thus, the present invention can be used as blocking groups for amino acids during peptide synthesis. The preferred amino acids are alpha-amino acids.

More specifically, the compounds of Formula Ia, wherein Z is a leaving group, can react with a carboxyprotected amino acid as indicated hereinbelow in Scheme II:

Scheme II

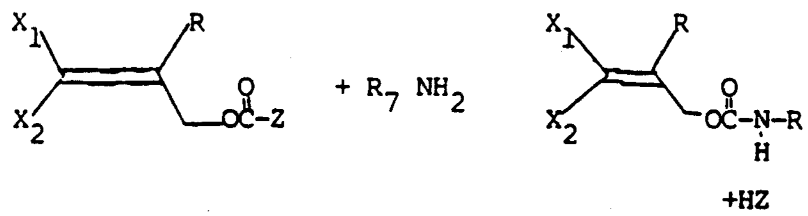

Ia XI XII

In the above scheme, $X_1$, $X_2$, R, Z are as defined above, and $R_7NH_2$ (XI) is an alpha-amino acid fluoride. The alpha-amino acids are the alpha-amino acids described herein above and include phenylalanine, glycine, valine, and the like. Often the amino acids are protected by a carboxy protecting group known in the art.

A variety of carboxy protecting groups known in the art may be employed. Examples of many of these possible groups may be found in "Protective Groups in Organic Synthesis" by T.W. Green, John Wiley and Sons, 1981. The preferred carboxy protecting group is the t-butyl ester.

In the above scheme, a compound of Formula Ia is reacted with a carboxy protected amino acid to form XII which is then hydrolyzed in acid to form a compound of Formula I wherein Z is an amino acid adduct. The most preferred protecting group are Bspoc; i.e., 2-(t-butylsulfonyl)-2-propenyloxycarbonyl and Bsmoc, i.e, Benzothiophenesulfone-2-methyloxycarbonyl, and 2-methylsulfonyl-3-phenyl-2-propenyloxycarbonyl (Mspoc).

If the leaving group is a halogen, especially chlorine, the reaction may be effected in an inert, polar organic solvent such as dioxane, tetrahydrofuran, dimethylformamide, pyridine or other solvent containing, for example, up to eight carbon atoms. The reaction is run under alkaline conditions, typically dilute aqueous alkali metal base such as sodium or potassium hydroxide or carbonate, at low temperatures, for example, from about 0°C to 25°C during a period of from about 2 to 3 hours. Usually the protected amino acid or peptide will precipitate upon acidification of the mixture, and may be purified by any appropriate method such as recrystallization. Excess blocking reagent may be employed, even up to 0.5 molar excess, but equimolar quantities of reactants generally give better results.

The protected amines can also be prepared by reaction of the 2-propenyl alcohols with isocyanates. This reaction will form a 2-propenyl carbamate directly without requiring the conversion of the alcohols to chloroformate.

Preferred protecting groups of Formula I are 2-t-butylsulfonyl-2-propenyloxycarbonyl (Bspoc), benzothiophene sulfone-2-methyloxycarbonyl (Bsmoc) and 2-methylsulfonyl-3-phenyl-2-propenyloxycarbonyl (Mspoc).

Other amino protecting groups are described in an article entitled "Solid Phase Peptide Synthesis" by G. Barany and R. B. Merifield in Peptides, Vol. 2, edited by E. Gross and J. Meienhoffer, Academic Presser, New York, New York, pp. 100-118 (1980), the contents of which are incorporated herein by reference. These N-amino protecting groups include such groups as the FMOC, Bspoc, Bsmoc, t-butyloxycarbonyl (BOC), t-amyloxycarbonyl (Aoc), β-trimethyl-ethyloxycarbonyl (TEOC), adamantyloxycarbonyl (Adoc), 1-methyl-cyclobutyloxycarbonyl (Mcb), 2-(p-biphenylyl)propyl-2-oxycarbonyl (Bpoc), 2-(p-phenylazophenyl)-propyl-2-oxycarbonyl (Azoc), 2,2-dimethyl-3,5-dimethyloxybenzyloxycarbonyl (Ddz), 2-phenylpropyl-2-oxycarbonyl (Poc), benzyloxycarbonyl (Cbz), p-toluenesulfonyl aminocarbonyl (Tao) o-nitrophenylsulfenyl (Nps), dithiasuccinoyl (Dts), phthaloyl, piperidinooxycarbonyl, formyl, trifluoroacetyl and the like.

These protecting groups can be placed into four categories:

1) a base labile N α-amino acid protecting group such as FMOC, and the like.

2) protecting groups removed by acid, such as Boc, TEOC, Aoc, Adoc, Mob, Bpoc, Azoc, Ddz, Poc, Cbz, 2-furanmethyloxycarbonyl (Foc), p-methoxybenzyloxycarbonyl (Moz), Nps, and the like.

3) protecting groups removed by hydrogenation such as Dts, Cbz.

4) protecting groups removed by nucleophiles, such as Bspoc, Bsmoc and Nps and the like.
5) protecting groups derived from carboxylic acids, such as formyl, acetyl, trifluoroacetyl and the like, which are removed by acid, base or nucleophiles.

As defined herein, a nucleophile is an electron-rich atom, i.e., an atom which can donate an electron pair, which tends to attack a carbon nucleus but does not act as a Bronsted Lowry base. For example, a nucleophile, as defined herein, includes those molecules which are used for nucleophilic addition across a double bond and behaves in a manner similar to that described in the schemes herein below.

The general mechanism for cleavage of Bspoc and Bsmoc groups are similar in that the nucleophile is believed to react through a Michael-type addition across a double bond. Although the following schemes are shown for Bspoc, it is also illustrative of Bsmoc:

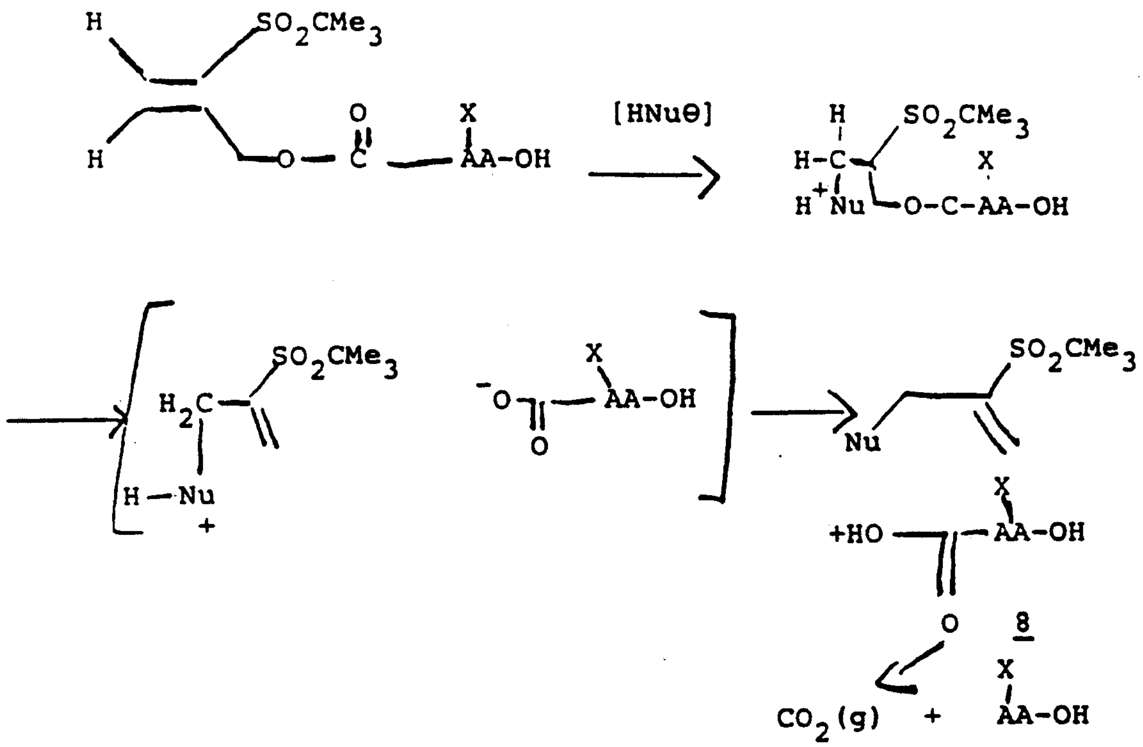


The nucleophile is believed to attack at the terminal carbon atoms of the propenyl group (Michael acceptor) forming a zwitterion which can eliminate the OCOAA(X)OH anion and $H^+$ to form an alkene-amine and the amide (8) after protonation. Loss of $CO_2$ will furnish the free amino acid.

The nucleophiles which will function in concert with this invention must have an active hydrogen atom, i.e., a hydrogen atom attached to the nucleophilic atom.

It is preferred that the nucleophile is a simple amine. It is especially preferred that the simple amine is a primary or secondary amine of the formula $HNR_5R_6$ wherein $R_5$ and $R_6$ are independently hydrogen, lower alkyl or substituted lower alkyl, the lower alkyl being substituted with OH, $CH_3$, or $CH_2CH_3$ or $R_5$ and $R_6$ taken together form a mono or bicyclic ring containing from 4 to 10 ring carbon atoms and 1 or 2 heteroatoms selected from the group consisting of nitrogen, sulfur or oxygen.

Typical examples of useful amines include ethanolamine, morpholine, piperidine, diethylamine, 2,6-dimethylpiperidine, piperazine, diethylamine, ethylamine and the like.

An organo mercaptan can also be used as a nucleophile, e.g., alkyl mercaptans, cycloalkyl mercaptans, aryl mercaptan or aralkyl mercaptans. The most preferred mercaptan is benzyl mercaptan.

The nucleophile can be added as a free compound or as an insoluble reagent attached to a solid support i.e., polystyrene or silica dioxide. These are represented by the formula:

p-[alk]-NuH

wherein p is an organic polymer as defined hereinabove or a silica gel polymer; alk is a chemical bond, alkyl or aroyl chain having from about one to about ten carbon atoms and Nu-H is a nucleophile as defined

hereinabove.

A preferred insoluble reagent is the silica based piperazine reagent 9:

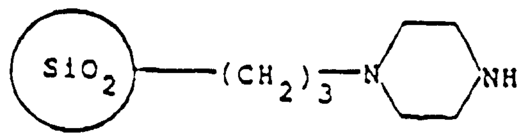

9

Another useful nucleophile is benzylmercaptan as shown in the following scheme.

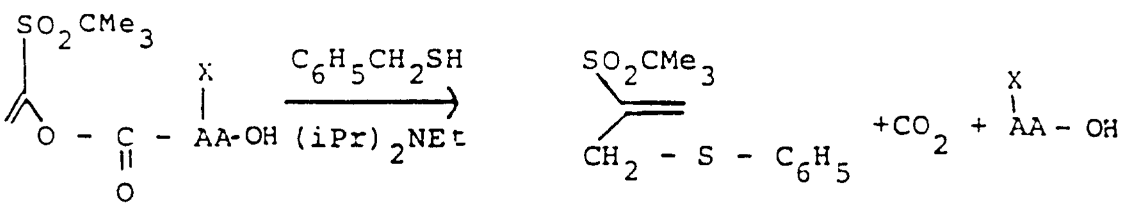


In this scheme the thio-group reacts in a Michael fashion to remove the Bspoc protecting group.

The amino acid fluorides of the present invention can be prepared by art recognized techniques. More specifically, it can be prepared by reacting an N-protected amino acid with the reagent cyannuric fluoride according to the following equation:

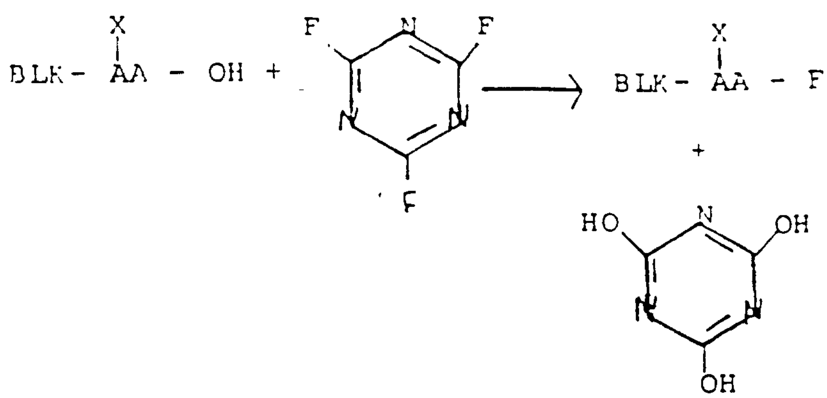

wherein BLK is an amino protecting group as defined herein and X is defined herein. It is preferred that BLK is the FMOC CLIMOC, BIMOC, DBD-TMOC, Bspoc, Bsmoc, Mspoc, or related base sensitive group. This reaction can be run at temperatures as low as 0° and up to the refluxing temperature of the solvent, but it is preferred that reaction is run at room temperature. It also can be run in an inert solvent such as pyridine/$CH_2Cl_2$ and the like.

The cyanuric fluoride can be prepared from the corresponding chloride in the presence of potassium flouride at elevated temperatures ranging from 150° to 250°C, according to the following equation:

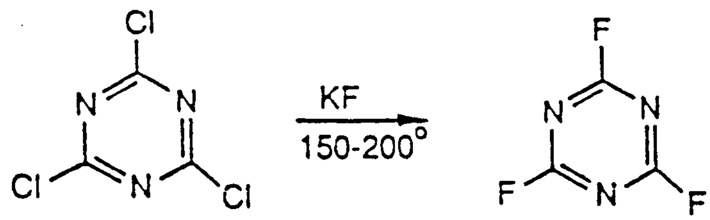


Other fluorinating agents well known in the art, such as thionyl fluoride, 2,4,6-trinitrofluorobenzene, N-methyl-2-fluoropyridinium salts, and the like may be used in place of KF to effect the formation of cyanuric fluoride.

The amino acid fluorides of the present invention are useful in peptide bond formation. The scope is quite broad, as the amino acid fluorides of the present invention can be coupled with an amino acid, a dipeptide, tripeptide, or higher peptide, having a free terminal amino group. As used herein, the term first amino acid is meant to include amino acids as well as dipeptides, and the higher peptides.

The synthesis of peptides according to the present invention requires the following steps:

1) protection of the carboxyl group on a first amino acid.
2) formation of the amino acid fluorides of the present invention in accordance with the procedure herein.
3) formation of the peptide bond by coupling the amino acid fluoride with the first amino acid.
4) removal of the protecting groups.

A variety of carboxy protecting groups known in the art may be employed. Examples of many of these possible groups may be found in "Protective Groups in Organic Synthesis", by T.W. Green, John Wiley & Sons, 1981, the contents of which is incorporated herein by reference.

The following sequence is illustrative of the coupling of an amino acid fluoride of the present invention with an amino acid having a free amino group:

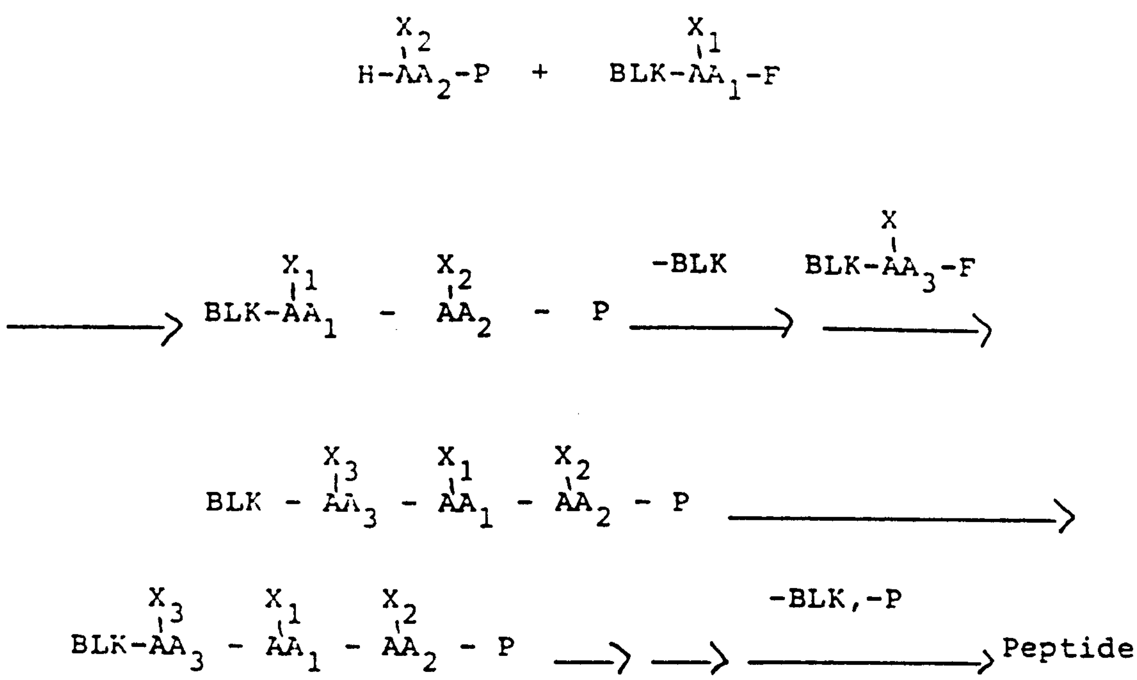


In the above scheme, BLK is as defined hereinabove, $X_1$, $X_2$ and $X_3$ are independently defined as X hereinabove, and P is a carboxy protecting group, e.g., methyl ester, t-butylester, β-trimethylsilylethyl ester, benzyl ester and the like.

As shown by the above scheme, the N α-amino protected amino acid fluoride is reacted with a second amino acid in which the carboxy group is protected. A peptide is formed between the first amino acid and the second amino acid. The peptide chain can be increased by removing the alpha amino protecting group by techniques known to one skilled in the art, and then reacting the corresponding di-peptide with another N

α-amino protected amino acid fluoride to form the corresponding tri-peptide. The N α-amino protecting group of the tri-peptide is removed and the above cycle is repeated until the desired peptide has been obtained.

The coupling of the N-α protected amino acid fluoride with the carboxy protected amino acid by the normal two phase techniques takes place without racemization.

The present invention can readily be utilized in solid phase peptide synthesis. Solid phase peptide synthesis is based on the stepwise assembly of a peptide chain while it is attached at one end to a solid support or solid phase peptide resin. Two methods are generally well known in the art.

One, the Merrifield method, employs a solid support for attachment of the amino acid or peptide residues. This method employs N-protected amino acids as building blocks which are added to an amino acid or peptide residue attached to the solid support at the acyl (acid) end of the molecule. After the peptide bond has been formed, the protected group is removed and the cycle repeated. When a peptide having the desired sequence has been synthesized, it is then removed from the support.

The second method, the inverse Merrifield method, employs reagents attached to solid supports in a series of columns. The amino acid or peptide residue is passed through these columns in a series to form the desired amino acid sequence.

These methods are well known in the art as discussed in U.S. Patent Nos. 4,108,846, 3,839,396, 3,835,175, 4,508,657, 4,623,484, 4,575,541, 4,581,167, 4,394,519 as well as in Advances in Enzymology, 32, 221 (1969) and in PEPTIDES, VOL, 2, edited by Erhard Gross and Johannes Meienhoffer, Academic Press, New York, New York pp. 3-255 (1980) and are incorporated herein by reference and is fully set forth herein.

The amino acid fluorides of the present invention can exist in various stereoisomeric forms, viz., the D or L stereoisomers. Moreover, the amino acid fluorides may be present in mixture of the D and L forms such as in racemic mixtures. All of these forms are comtemplated to be within the scope of the present invention. It is preferred that the stereoisomer of the amino acid fluorides of the present invention exist in the L form.

## EXAMPLES

The invention will now be illustrated by examples. The examples are not intended to be limiting of the scope of the present invention. In conjunction with the general and detailed descriptions above, the examples provide further understanding of the present invention.

## I PREPARATIVE EXAMPLES: PREPARATION OF CYANURIC FLUORIDE

A 250 mL three necked round bottomed flask equipped with a thermometer, stirring bar, distilling apparatus and powder dropping funnel was charged with oven-dried sodium fluoride (53.g, 1.25 mole) and tetramethylene sulfone (sulfolane, TMS) (145 g, 115 mL, 1.20 mole, d = 1.26). To the suspension was added cyanuric chloride (62 g. 0.33 mole) in small portions by use of the powder dropping funnel over a 10-min period. The reaction mixture was gradually heated to 250 °C by means of a heating mantle. The product distilled from the flask as formed and was collected in glass traps cooled in a Dry Ice-acetone bath. Collection of the distillate (29 mL) continued until distillation stopped (bp 72-78 °C). Redistillation gave 24 mL (86.19 g, 86,2% d = 1.6 g/mL) of the fluoride as a clear colorless liquid, bp 72-73 °C, lit bp 74 °C.

## II GENERAL PROCEDURE FOR PREPARATION OF N-(9-FLUORENYL-METHOXYCARBONYL)AMINO ACID FLUORIDES:

A solution (or suspension) of FMOC-amino acid (1 mmole) in dry $CH_2Cl_2$ (5 mL) was kept under nitrogen and treated with cyanuric fluoride (1.08 g, 8 mmol, 700 uL, d = 1.6) and pyridine (81 uL, 1 mmole). A clear solution was obtained which was refluxed (or stirred at room temperature) for 45-120 min. Completion of reaction was checked by TLC. During the reaction a white precipitate separated. The mixture was extracted with ice-water (2 x 15 mL) which caused the precipitate to dissolve. The organic layer was dried over $MgSO_4$. Filtration and solvent removal gave a residue (solid or oil) which was crystallized from $CH_2Cl_2$/hexane or $Et_2O$/hexane to give the corresponding FMOC-N-protected amino acid fluoride as a white crystalline solid. The crude and recrystallized acid fluorides were analyzed by HPLC following the same technique described for the corresponding chlorides except that it was necessary to wait for 15-300 min following addition of the fluoride to dry methanol in order to allow time for complete conversion to the methyl ester. For example, a mixture initially analyzing for 93.45% FMOC-Gly-F (as methyl ester) came to complete conversion after 50 min with a measured content of 98.89% FMOC-Gly-OMe and 1.00% FMOC-Gly-OH. In case of FMOC-Val-F, initially analyzing for 82.70% FMOC-Val-F (as methyl ester) complete conversion occurred after 5 hours with a measured content of 98.19% FMOC-Val-OMe and 0.94% FMOC-Val-OH. Fischer esterification of the free acid in the methanolic HF solution did not occur. For example, a mixture initially containing 52.27% of FMOC-Gly-OH showed no significant change after 15 hours (52.19% acid) and the same results were observed in case of FMOC-Val-OH.

EXAMPLE 1

N-(9-Fluorenylmethoxycarbonyl)glycine Fluoride:

The reaction was used in accordance with the procedure described above.

Reaction was completed after 2 hours of reflux, the fluoride being obtained in 80.5% yield as pale yellow needles, mp 140.1 °C, (98.9% pure according to HPLC analysis); IR (KBr) 3337 (NH), 1843 (COF), 1680 (OCON) $cm^{-1}$; $^1H$ NMR ($CDCl_3$) $\delta$ 4.1-4.3 (m, 3, C<u>H</u>$CH_2$), 4.5 (d, 2, $NCH_2CO$), 5.3 (bs, 1, NH), 7.15 - 7.8 (m, 8, aryl).

| | | | |
|---|---|---|---|
| Anal. Calcd for $C_{17}H_{14}FNO_3$: | C, 68.22; | H, 4.71; | N, 4.67. |
| Found: | C, 68.25, | H, 4.63; | N, 4.79. |

EXAMPLE 2

N-(9-Fluorenylmethoxycarbonyl)alanine Fluoride:

The reaction was run in accordance with the procedure described hereinabove.

Reaction was complete after 2 hours of reflux, the fluoride being obtained in 75.4% yield as a white solid, mp 111-2 °C, (98.66% pure according to HPLC analysis); $[\alpha]D^{23}$ + 3.6 ° (c 0.5, EtOAc); IR (KBr) 3326 (NH), 1845 (COF), 1690 (OCON) $cm^{-1}$; $^1H$ NMR ($CDCl_3$) $\delta$ 1.6 (d, 3, $CH_3$), 4.2 (t, 1, C<u>H</u>$CH_2$), 4.5 (m, 3, $CH_2O$, NCHCO), 5.2 (d, 1, NH), 7.2-7.8 (m, 8, aryl).

| | | | |
|---|---|---|---|
| Anal. Calcd for $C_{18}H_{16}FNO_3$: | C, 69.00; | H, 5.14; | N, 4.47. |
| Found: | C, 69.16; | H, 5.30; | N, 4.30. |

EXAMPLE 3

N-(9-Fluorenylmethoxycarbonyl)valine Fluoride:

The reaction was run in accordance with the procedure described hereinabove.

Reaction was complete after 2 hours of reflux, the fluoride being obtained in a yield of 70.2% as a white solid, mp 113-4 °C, (98.62% pure according to HPLC analysis); $[\alpha]D^{24}$ + 10.7 ° (c 1, $CH_2Cl_2$); IR (KBr) 3312 (NH), 1843 (COF), 1688 (OCON) $cm^{-1}$; $^1H$ NMR ($CDCl_3$) $\delta$ 1.0 (d, 6, $CH_3$), 2.3 (m, 1, C<u>H</u>$CH_2$), 4.2 (t, 1, C<u>H</u>$CH_3$), 4.5 (m, 3, $CH_20$, NCHCO), 5.15 (d, 1, NH), 7.2-7.8 (m, 8, aryl).

| | | | |
|---|---|---|---|
| Anal. Calcd for $C_{20}H_{20}FNO_3$: | C, 70.36; | H, 5.90; | N, 4.10. |
| Found: | C, 70.27; | H, 5.92; | N, 4.19. |

EXAMPLE 4

N-(9-Fluorenylmethoxycarbonyl)leucine Fluoride:

The reaction was run in accordance with the procedure described above.

Reaction was complete after 1 hour of reflux, the fluoride being obtained in a yield of 75.2% as a white solid, mp 95.5-6.5 °C, (98.58% pure according to HPLC analysis); $[\alpha]D^{23}$ - 5.8 ° (c 1, EtOAc); IR (KBr) 3336

(NH), 1838 (COF), 1699 (OCON) $cm^{-1}$; $^1H$ NMR ($CDCl_3$) $\delta$ 1.00 (d, 6, $CH_3$), 1.6-1.8 (m, 3, $CH_2CH$), 4.2 (t, 1, C<u>H</u>$CH_2O$), 4.5 (m, 3, $CH_2O$, NCHCO), 5.1 (d, 1, NH), 7.2-7.8 (m, 8, aryl).

| | | | |
|---|---|---|---|
| Anal. Calcd for $C_{21}H_{22}FNO_3$: | C, 70.96; | H, 6.23; | H, 3.94. |
| Found: | C, 70.70; | H, 6.48; | N, 4.15. |

EXAMPLE 5

N-(9-Fluorenylmethoxycarbonyl)isoleucine Fluoride:

The reaction was run in accordance with the procedure described above.

Reaction was complete after 1.5 hours of reflux, the fluoride being obtained in a yield of 73.3% as a white solid, mp 115-6°C, (97.13%) pure according to HPLC analysis); $[\alpha]D^{23}$ + 15.6° (c 0.5, EtOAc); IR (KBr) 3304 (NH), 1840 (COF), 1696 (OCON) $cm^{-1}$; $^1H$ NMR ($CDCl_3$) $\delta$ 1.00 (m, 6, CH(<u>Me</u>)$CH_2$<u>Me</u>), 1.1-1.6 (m, 2, CHC<u>H$_2$</u>$CH_3$), 2.00 (m, 1, CH), 4.2 (t, 1, C<u>H</u>$CH_2O$), 4.5 (m, 3, $CH_2O$, NCHCO), 5.2 (d, 1, NH), 7.2-7.8 (m, 8, aryl).

| | | | |
|---|---|---|---|
| Analy. Calcd for $C_{21}H_{22}FNO_3$: | C, 70.96; | H, 6.23; | N, 3.94. |
| Found: | C, 68.30; | H, 6.06; | N, 3.87. |

EXAMPLE 6

N-(9-Fluorenylmethoxycarbonyl)proline Fluoride:

The reaction was run in accordance with the procedure described above.

Reaction was complete after 12 hours of stirring at room temperature, the fluoride being obtained in a yield of 78.2% as a white solid mp, 88-9°C; $[\alpha]D^{30}$ - 28.6° (c 0.5, EtOAc).

| | | | |
|---|---|---|---|
| Anal. Calcd for $C_{20}H_{18}FNO_3$ : | C, 70.78; | H, 5.34; | N, 4.12. |
| Found: | C, 70.86; | H, 5.43, | N, 4.21. |

EXAMPLE 7

N-(9-Fluorenylmethoxycarbonyl)phenylalanine Flouride:

The reaction was run in accordance with the procedure described above.

Reaction was complete after 1 hour of reflux, the fluoride being obtained in a yield of 63.9% as white crystals, mp 118-20°C, (99.3% pure according to HPLC analysis); $[\alpha]D^{23}$ + 35.5° (c 1, $CH_2Cl_2$); IR (KBr) 3318 (NH), 1843 (COF), 1700 (OCON) $cm^{-1}$; $^1H$ NMR ($CDCl_3$) $\delta$ 3.2 (d, 2, <u>CH$_2$</u>$C_6H_5$), 4.2 (t, 1, <u>CH</u>$CH_2O$), 4.45 (m, 2, $CH_2O$), 4.85 (m, 1, NCHCO), 5.1 (d, 1, NH), 7.1-7.8 (m, 13 aryl).
Anal. Calcd for $C_{24}H_{20}FNO_3$: C, 74.03; H, 5.14; N, 3.59; F, 4.88. Found: C, 74.03; H, 5.13; N, 3.69; F, 4.68.

EXAMPLE 8

N-(9-Fluorenylmethoxycarbonyl)tryptophan Fluoride:

The reaction was run in accordance with the procedure described above.

Reaction was complete after 1 hour of stirring at room temperature, the fluoride being obtained in a yield of 70.7% as a white solid, mp 125-8°C (dec.) (98.2% pure according to HPLC analysis); $[\alpha]D^{24}$ - 5.2° (c 1, EtOAc); IR (KRr) 3390 and 3360 (NH), 1845 (COF), 1697 (OCON) $cm^{-1}$, $^1H$ NMR ($CDCl_3$) $\delta$ 3.4 (d, 2, <u>CH$_2$</u>CHCO), 4.2 (t, 1, C<u>H</u>$CH_2O$), 4.4 (d, 2, CHC<u>H$_2$</u>O), 4.9 (m, 1, NCHCO), 5.3 (d, 1, NH), 7.0-8.2 (m, 14, NH + aryl).

| | | | |
|---|---|---|---|
| Anal. Calcd for $C_{33}H_{31}FNO_3$: | C, 72.88; | H, 4.94; | N, 6.53. |
| Found: | C, 72.83, | H, 5.01; | N, 6.43. |

EXAMPLE 9

N-(9-Fluorenylmethoxycarbonyl)-O-(t-Butyl)serine Fluoride

The reaction was run in accordance with the procedure described above.

Reaction was completed after 1 hour of stirring at room temperature, the fluoride being obtained in a yield of 72.7% as white crystals, mu 89-91 °C, (98.26% pure according to HPLC analysis); $[\alpha]D^{26}$ + 28.8 ° (c 0,5 EtOAc); IR (KBr) 3444 (NH), 1868 (COF), 1733 (OCON) $cm^{-1}$; $^1H$ NMR ($CDCl_3$) $\delta$ 1.2 (s, 9, $CMe_3$), 3.6 (q, 1, CHHOC$Me_3$), 3.9 (q, 1, CHHOC$Me_3$), 4.22 (t, 1, CH$CH_2$OCO), 4.45 (m, 2, $CH_2$OCO), 4.7 (m, 1, NCHCO), 5.65 (d, 1, NH), 7.25-7.8 (m, 8, aryl).

| | | | |
|---|---|---|---|
| Anal. Calcd for $C_{22}H_{24}FNO_4$: | C, 68.55; | H, 6.27; | N, 3.63. |
| Found: | C, 68.49; | N, 6.32; | N, 3.67. |

EXAMPLE 10

N-(9-Fluorenylmethoxycarbonyl)-O-(t-Butyl)threonine Fluoride:

The reaction was run in accordance with the procedure described hereinabove:

Reaction was complete after 1 1/2 hours of stirring at room temperature, the fluoride being obtained in a yield of 72.6% as white crystals, mp 53-5 °C, (98.03% pure according to HPLC analysis); $[\alpha]D^{27}$ + 12.3 ° (c 0.4 EtOAc); IR (KBr) 3320 (NH), 1857 (COF), 1726 (OCON) $cm^{-1}$; $^1H$ NMR ($CDCl_3$) $\delta$ 1.15 (s, 9, $CMe_3$), 1.3 (d, 3, $CH_3$), 4.3-4.5 (m, 5, CH$CH_2$OCO, NCH(CHO-)CO), 5.6 (d, 1, NH), 7.2-7.8 (m, 8, aryl).

| | | | |
|---|---|---|---|
| Anal. Calcd for $C_{23}H_{26}FNO_4$: | C, 69.15; | H, 6.56; | N, 3.50. |
| Found: | C, 69.11; | H, 6.83; | N, 4.00. |

EXAMPLE 11

N-α-(9-Fluorenylmethoxycarbonyl)-Nε-(t-Butyloxycarbonyl)lysine Fluoride:

The reaction was run in accordance with the procedure described above.

Reaction was completed after 1 hour of stirring at room temperature, the fluoride being obtained in a yield of 65.9% as white crystals, mp 128-30 °C (99.5% pure according to HPLC analysis); $[\alpha]D^{23}$ - 2.2 ° (c 0.5, $CH_2Cl_2$); IR (KBr) 2254 (NH), 1854 and 1836 (COF), 1693 (OCON) $cm^{-1}$; $^1H$ ($CDCl_3$) 1.4 (s, 9, $CMe_3$), 1.5-2 (m, 6, $CH_2$-$CH_2$-$CH_2$), 3.15 (m, 2, $CH_2$NH), 4.2 (t, 1, CH$CH_2$O), 4.4-4.6 (m, 4, $CH_2$O, NCHCO, $CH_2$NH), 5.7 (d, 1, NH), 7.2-7.8 (m, 8, aryl).

| | | | |
|---|---|---|---|
| Anal. Calcd For $C_{36}H_{31}FN_2O_5$: | C, 66.36; | H, 6.64; | H, 5.95. |
| Found: | C, 66.16; | H, 6.50; | N, 5.92. |

EXAMPLE 12

N-(9-Fluorenylmethoxycarbonyl)aspartic Acid Fluoride-β-(t-Butyl) Ester:

The reaction was run in accordance with the procedure described above.

Reaction was complete after 30 minutes of stirring at room temperature, the fluoride being obtained in a yield of 67.8% a white crystals, mp 74-5°C, (97.97% pure according to HPLC analysis); $[\alpha]D^{23}$ + 4.00° (c 0.5, EtOAc); IR (KBr) 3320 (NH), 1856 (COF), 1725 (COO), 1695 (OCON) $cm^{-1}$, $^1H$ NMR ($CDCl_3$) δ 1.45 (s, 9, $CMe_3$), 2.9 (dq, 2, $CH_2COO$), 4.3 (t, 1, $\underline{CH}CH_2OCO$), 4.5 (m, 2, $CH\underline{CH_2}O$), 4.85 (m, 1, HCHCO), 5.85 (d, 1, NH), 7.2-7.8 (m, 8, aryl).

| | | | |
|---|---|---|---|
| Anal. Calcd for $C_{23}H_{24}FNO_5$: | C, 66.81; | H, 5.85; | N, 3.38. |
| Found: | C, 67.03; | H, 5.95; | N, 3.70. |

The results of the above synthesis are summarized in the Table hereinbelow:

Table 1

| Synthesis of FMOC-Amino Acid Fluorides | | | |
|---|---|---|---|
| Compound | Yield % | mp(°C) | $[\alpha]_D^{t°C}$ |
| FMOC-Gly-F | 80.5 | 140-1 | |
| FMOC-Ala-F | 75.4 | 111-2 | +3.6° (c 0.5, EtOAc, 23) |
| FMOC-Val-F | 70.2 | 113-4 | +10.7° (c 1, $CH_2Cl_2$, 24) |
| FMOC-Leu-F | 75.2 | 95-6 | -5.8° (c 1, EtOAc, 23) |
| FMOC-Ile-F | 73.3 | 115-6 | +15.6° (c 0.5, EtOAc, 23) |
| FMOC-Phe-F | 63.9 | 118-20 | +35.5° (c 1, $CH_2Cl_2$, 24) |
| FMOC-Trp-F | 70.7 | 125-8 | -5.2° (c 1, EtOAc, 24) |
| FMOC-Ser(tBu)-F | 72.7 | 89-91 | +28.8° (c 0.5, EtOAc, 26) |
| FMOC-Thr(tBu)-F | 72.6 | 53-5 | +12.3° (c 0.4, EtOAc, 27) |
| FMOC-Lys(BOC)-F | 65.9 | 128-30 | -2.2° (c .5, $CH_2Cl_2$, 24) |
| FMOC-Asp(OtBu)-F | 67.8 | 74-5 | +4.0° (c 0.5, EtOAc, 23) |

These results indicate that cyanuric fluoride is suitable not only for the preparation of simple FMOC-amino acid fluorides but also for those containing t-BOC, t-Bu or CBZ-groups on the side chain. The amino acid fluorides described hereinabove were obtained in crystalline formed.

The use of amino acid fluorides of the present invention being used as peptide coupling agents is illustrated hereinbelow.

EXAMPLE 13

t-Butyl Allyl Sulfide. To a solution of 350 mL of anhydrous ethanol maintained under nitrogen was slowly added 22.99 g (1 mol) of sodium spheres. The sodium dissolved within 90 minutes, and to the resulting sodium ethoxide solution was added 90.19 g (1 mol) of t-butyl mercaptan with mechanical stirring. Allyl bromide (120.98 g; 1 mol) was then added dropwise to the mechanically stirred sodium t-butyl thiolate solution. After the addition was complete, the mixture was refluxed for 10 minutes, the solution allowed to cool, the precipitated sodium bromide filtered, and the ethanol removed by distillation at atmospheric pressure. The residue was diluted with 200 mL of water, and the layers separated. The aqueous layer was extracted with five 40-mL portions of ether. The combined organic layers were extracted with 150 mL of water, the organic layer dried over $MgSO_4$, filtered, and the solvent removed in vacuo from a water bath at 45°C to give a yellow liquid. Distillation through a 0.8 x 15-cm fractionating column gave 56.16 g (45%) of the sulfide as a colorless liquid, bp 139-141°C; IR (neat), $cm^{-1}$ 3090, 2960 1635, 1455, 1360, 1160, 985, 915; $^1H$ NMR ($CDCl_3$) 1.35 (s, 9H, t-butyl), 3.20 (d, 2H, $CH_2$), 4.90-6.25 (m 3H, vinyl).

EXAMPLE 14

1,3-Dibromo-2-(t-butylsulfonyl) Propane. To a stirred solution of 17.37 g (0.13 mol) of t-butyl allyl sulfide in 133 mL of $CCl_4$ at -24°C ($CCl_4$/dry ice) was added dropwise a solution of 21.33 g (0.13 mol) of $Br_2$ in 67 mL of $CCl_4$. A yellow solid precipitated during the addition. The mixture was warmed to room temperature and stirred for 10 minutes following complete solution of the yellow solid. The resulting solution was poured into a mixture of 55.50 g (0.27 mol) of 85% m-chloroperbenzoic acid in 490 mL of $CH_2Cl_2$ kept at -24°C, and the mixture stirred for 30 minutes at this temperature. The cooling bath was then removed and the mixture starred at room temperature overnight. The precipitated m-chlorobenzoic acid was filtered and the filtrate washed with three 200-mL portions of saturated $NaHCO_3$, followed by 200 mL of water. The organic layer was dried over $MgSO_4$, filtered, and the solvent removed in vacuo from a water bath at 45°C. The crude product was recrystallized from 20% EtOAc/Skelly B to give 32.02 g (75%) of the dibromide, mp 139-140°C; IR (KBr), $cm^{-1}$ 2980, 1470, 1365, 1285, 1105, 860, 840, 685; $^1H$ NMR ($CDCl_3$) 1.45 (S, 9H, t-butyl), 3.80-4.00 (m, 5H, CH and $CH_2$).

EXAMPLE 15

2-(Phenylsulfinyl)-2-propenyl N-p-Chlorophenyl Carbamate. A solution of 0.57 g (3.1 mmol) of 2-(phenylsulfinyl)-2-propenyl alcohol and 0.48 g (3.1 mmol) of p-chlorophenyl isocyanate in 10 mL of benzene was refluxed overnight. The solvent was removed in vacuo from a water bath at 45°C to give a brown oil which was recrystallized from $CCl_4$/Skelly B to give 0.72 g (69%) of the urethane, mp 106-107.5°C; IR (KBr) $cm^{-1}$ 3240, 1730, 1600, 1545, 1490, 1310, 1220, 1030, 745, 680; $^1H$ NMR ($CDCl_3$) $\delta$ 4.65 (s, 1H, allylic), 4.70 (s, 1H, allylic), 6.00 (s, 1H, vinyl), 6.30 (s, 1H, vinyl), 7.10-7.85 (m, 10H, phenyl and NH).

Anal. Calcd for $C_{16}H_{14}ClNO_3S$: C, 57.23; H, 4.20; N, 4.17. Found: C, 56.95; H, 4.14; N, 4.10.

EXAMPLE 16

t-Butyl 2-(t-Butylsulfonyl)-2-propenyloxycarbonyl-L-phenylalaninate. To a stirred solution of 0.185 g (0.768 mmol) of 2-(t-butylsulfonyl)-2-propenyl chloroformate in 2 mL of benzene at 0°C was added dropwise a solution of 0.34 g (1.53 mmol) of t-butyl L-phenylalaninate in 5 mL of benzene. Upon addition, a white precipitate began to separate. Upon completion of the addition, the slurry was stirred at room temperature for 30 minutes, poured into 15 mL of ether and washed with three 15-mL portions of 5% HCl, and 15 Ml of water. The organic layer was dried over $MgSO_4$, filtered, and the solvent removed in vacuo from a water bath at 45°C. The resulting oil was recrystallized from 20% ether/pentane to give 0.23 g (70%) of the colorless ester, mp 67-69°C; IR (KBr) $cm^{-1}$ 3410, 2980, 1715, 1510, 1365, 1290, 1155, 1095, 1060, 940, 750, 700, 625; $^1H$ NMR ($CDCl_3$) $\delta$ 1.45 (s, 9H, t-butyl), 1.50 (s, 9H, t-butyl), 3.12 (m, 2H, benzyl), 4.56 (q, 1H, CH), 4.90 (s, 2H, $CH_2O$), 5.38 (d, 1H, NH), 6.14 (s, 1H, vinyl), 6.29 (s, 1H, vinyl), 7.15-7.40 (m, 5H, Phenyl); $[\alpha]_D^{28}$ +25.4° (c = 1, $CH_2Cl_2$), also,

$$[\alpha]^{28}_{546}$$

+30.9° (c = 1, $CH_2Cl_2$).

Anal. Calcd for $C_{21}H_{31}NO_6S$: C, 59.27; H, 7.34; N, 3.29. Found: C, 59.31; H, 7.45; N, 3.23.

EXAMPLE 17

2-(t-Butylsulfonyl)-2-propenyloxycarbonyl-L-phenylalanine. A solution of 4.57 g (19.0 mmol) of 2-(t-butylsulfonyl)-2-propenyl chloroformate and 5.64 g (18.6 mmol) of t-butyl L-phenylalaninate hydrophosphite in 90 mL of $CH_2Cl_2$ was stirred in the presence of 165 mL of 5% $NaHCO_3$ at room temperature for 2 hours. The aqueous phase was separated, and the organic phase washed with three 75-mL portions of 5% HCl. The organic phase was dried over $MgSO_4$, filtered, and the solvent removed in vacuo from a water both at 45°C. The resulting oil was dissolved in 36 mL of 50% $CH_2Cl_2$/triflouroacetic acid, and the solution stirred at room temperature for (91%) of the colorless acid, mp 88.0-89.5°C; IR (KBr) $cm^{-1}$ 3270, 1760 1690, 1520, 1290, 1200, 1100, 1060, 960, 755, 700, 630; $^1H$ NMR ($CDCl_3$) 1.40 (s, 9H, t-butyl), 3.20 (m, 2H, benzyl), 4.75 (q, 1H, CH), 4.90 (s, 2H, $CH_2O$), 5.35 (d, 1H, NH), 6.15 (s, 1H, vinyl), 6.32 (s, 1H, vinyl), 7.15-7.40 (m, 5H, phenyl); $[\alpha]_D^{24}$ -31.0° (c = 0.5, DMF), also

$$[\alpha]^{24}_{546}$$

-37.5° (c=0.5 DMF).

Anal. Calcd for $C_{17}H_{23}NO_6S$: C, 55.27; H, 6.27; N, 3.79. Found: C, 55.02; H, 6.47; N, 3.71.

EXAMPLE 18

2-(t-Butylsulfonyl)-2-propenyloxycarbonyl-L-phenylalanyl fluoride. The compound of Example 17 is reacted with cyanuric fluoride as described herein to prepare the above product.

EXAMPLE 19

2-(t-Butylsulfonyl)-2-propenyloxycarbonyl Glycerine. A mixture of 3.34 g (13.9 mmol) of 2-(t-butylsulfonyl)-2-propenyl chloroformate and 2.95 g (13.8 mmol) of t-butyl glycinate hydrophosphite in 85 mL of $CH_2Cl_2$ was stirred in the presence of 100 mL of 5% $NaHCO_3$ at room temperature for 4 hours. The aqueous phase was separated, and the organic phase washed with three 33-mL portions of 5% HCl. The organic phase was dried over $MgSO_4$, filtered, and the solvent removed in vacuo from a water bath at 45°C. The resulting oil was dissolved in 20 mL of 50% $CH_2Cl_2$/triflouroacetic acid, and the solution stirred at room temperature for 2 hours. Excess triflouroacetic acid and solvent were removed in vacuo from a water bath at 45°C. The resulting oil was recrystallized from 30% EtOAc/Skelly B to give 3.25 g (84%) of the acid, mp 105.0-105.5°C; IR (KBr) $cm^{-1}$ 3355, 1765, 1695, 1560, 1290, 1190, 1100, 1050, 770; $^1H$ NMR ($CDCl_3$) $\delta$ 1.41 (s, 9H, t-butyl), 4.05 (d, 2H, $CH_2$), 4.94 (s, 2H, $CH_2O$), 5.44 (t, 1H, NH), 6.26 (s, 1H, vinyl), 6.33 (s, 1H, vinyl).

Anal. Calcd for $C_{10}H_{17}NO_6S$: C, 43.00; H, 6.13; N, 5.01. Found: C, 43.00; H, 6.03; N, 4.97.

EXAMPLE 20

2-t-butylsulfonyl-2-propenyloxycarbonyl glycine fluoride. The product of Example 19 is reacted with cyanuric fluoride as described herein to prepare the above product.

EXAMPLE 21

2-(t-Butylsulfonyl)-2-propenyloxycarbonyl-D-valine. A solution of 1.01 g (4.15 mmol) of 2-(t-butylsulfonyl)-2-propenyl chloroformate and 0.87 g (4.15 mmol) of t-butyl D-valinate hydrochloride in 22 mL of $CH_2Cl_2$ was stirred in the presence of 45 mL of saturated $NaHCO_3$ for 90 minutes. The aqueous phase was separated, and the organic phase was washed with two 20-mL portions of 5% HCl. The organic phase was dried over $MgSO_4$, filtered, and the solvent removed in vacuo from a water bath at 45°C. The resulting oil was dissolved in 10 mL of 50% $CH_2Cl_2$/trifluoroacetic acid, and the solution stirred at room temperature for 90 minutes. Excess triflouroacetic acid and solvent were removed in vacuo from a water bath at 45°C. The resulting oil was recrystallized from ether/Skelly F to give 1.01 g (75%) of the colorless acid, mp 113-114°C; IR (KBr) $cm^{-1}$ 3380, 3160, 1750, 1700, 1540, 1400, 1295, 1100, 1025, 760, 665; $^1H$ NMR ($CDCl_3$) $\delta$ 0.9 (d, 3H, $CH_3$), 1.05 (d, 3H, $CH_3$), 1.4 (s, 9H, t-butyl), 2.2 (m, 1H, CH), 4.3 (d of d, 1H, CHN), 4.95 (s, 2H, $CH_2O$), 5.5 (d, 1H, NH), 6.25 (s, 1H, vinyl), 6.35 (s, 1H, vinyl), 11.6 (s, 1H, $CO_2H$); $[\alpha]^{25}_D$ +3.2° (c=1, $CHCl_3$), also

$$[\alpha]^{25}_{546} +3.7°$$

(c=1, $CHCl_3$).

Anal. Calcd for $C_{13}H_{23}NO_6S$: C, 48.58; H, 7.21; N, 4.36. Found: C, 48.70; H, 6.99; N, 4.29.

EXAMPLE 22

2-t-butylsulfonyl-2-propenyloxycarbonyl fluoride. The product of Example 21 is reacted with cyanuric fluoride as described herein to prepare the above product.

EXAMPLE 23

2-(t-Butylsulfonyl)-2-propenyl Bromide. A mixture of 16.78 g (0.052 mol) of 1,3-dibromo-2-(t-butylsulfonyl) propane and 14 mL (0.12 mol) of 2,6-lutidine in 55 mL of $CH_2Cl_2$ was refluxed for 75 minutes. The solution was allowed to cool to room temperature and extracted with three 80-mL portions of 5% HCl followed by 80 mL of water. The organic layer was dried over $MgSO_4$, filtered, and the solvent removed in vacuo from a water bath at 45 °C to give 11.46 g (91%) of the allyl bromide as a white solid, mp 40.5-42.0 °C, which was used without further purification; IR (KBr) 2980 $cm^{-1}$, 1480, 1395, 1230, 1100, 970, 730, 640; $^1H$ NMR ($CDCl_3$) $\delta$ 1.40 (s, 9H, t-butyl), 4.30 (s, 2H $CH_2Br$), 6.50 (s, 2H, vinyl).

EXAMPLE 24

2-(t-Butylsulfonyl-2-propenyl Alcohol. A mixture of 8.55 g (35.3 mmol) of 2-(t-butylsulfonyl)-2-propenyl bromide and 5.31 g (78.1 mmol) of sodium formate in 150 mL of methanol was refluxed overnight. The solution was allowed to cool and concentrated to 50 mL with the aid of a water aspirator, resulting in the precipitation of excess sodium formate. The residue was diluted with 150 mL of water and extracted with five 50-mL portions of $CH_2Cl_2$. The organic layer was dried over $MgSO_4$, filtered, and the solvent removed in vacuo from a water bath at 45 °C. The crude product was recrystallized from 15% EtOAc/Skelly F to give 4.30 g (68%) of the alcohol as a colorless solid, mp 53.5-54.5 °C; IR (KBr), $cm^{-1}$ 3470, 3120, 3000, 1450, 1370, 1270, 1095, 1050, 960, 900, 800, 750, 630; $^1H$ NMR ($CDCl_3$) $\delta$ 1.39 (s, 9H, t-butyl), 2.57 (t, 1H, OH), 4.56 (d, 2H $CH_2O$), 6.30 (s, 1H, vinyl), 6.31 (s, 1H, vinyl).

Anal. Calcd for $C_7H_{14}O_3S$: C, 47.17; H, 7.92; S. 17.99. Found: C, 47.07; H, 7.95; S, 17.70.

EXAMPLE 25

2-(t-Butylsulfonyl)-2-propenyl Chloroformate. To a solution of 6.67 g (37.4 mmol) of 2-(t-butylsulfonyl)-2-propenyl alcohol in 27 mL of dry THF at 0 °C was added in one portion 27 mL of phosgene. The solution was stirred for 1 hour at 0 °C and allowed to stand at room temperature overnight. Excess phosgene and solvent were removed under reduced pressure. The crude product was recrystallized from 25% ether/Skelly B to give 8.23 g (91%) of the chloroformate as a colorless solid, mp 56.5-57.7; IR (KBr) $cm^{-1}$ 2980, 1755, 1430, 1380, 1290, 1140, 1100, 965, 915, 810, 750, 680, 630; $^1H$ NMR ($CDCl_3$) $\delta$ 1.41 (s, 9H, t-butyl), 5.11 (s, 2H, $CH_2O$), 6.37 (s, 1H, vinyl), 6.47 (s, 1H, vinyl).

Anal. Calcd for $C_8H_{13}ClO_4S$: C, 39.92; H, 5.44; S, 3.32. Found: C, 40.10; H, 5.40; S, 13.07.

EXAMPLE 26

2-(t-Butylsulfonyl)-2-propenyl N-p-Chlorophenyl Carbamate. To a solution of 0.24 g (1.0 mmol) of 2-(t-butylsulfonyl)-2-propenyl chloroformate in 3 mL of benzene at 0 °C was added dropwise 0.26 g (2.0 mmol) of p-chloroaniline 3 mL of benzene. A white precipitate separated almost immediately. After the addition was complete the mixture was stirred at 0 °C for 10 minutes and for 2 hours at room temperature. The mixture was then diluted with 15 mL of benzene and extracted with two 15-mL portions of 5% HCl, followed by 15 mL of water. The organic layer was dried over $MgSO_4$, filtered, and the solvent removed in vacuo from a water bath at 45 °C. The crude product was recrystallized from 20% EtOAc/Skelly B to give 0.26 g (79%) of the urethane, mp 124-125 °C. The same compound was obtained in 85% yield by treatment of 2-(t-butylsulfonyl)-2-propenyl alcohol with p-chlorophenyl isocyanate in refluxing benzene; IR (KBr) $cm^{-1}$ 3340, 3120, 2980, 1730, 1600, 1490, 1430, 1280, 1210, 1070, 975, 915, 830, 750, 620; $^1H$ NMR ($CDCl_3$) $\delta$ 1.4 (s 9H, t-butyl), 4.0 (t, J = 1 Hz, 2H, $CH_2O$), 6.3 (s 1H, vinyl), 6.35 (s, 1H, vinyl), 7.15-7.5 (m, 5H, phenyl and NH).

Anal. Calcd. for $C_{14}H_{18}ClNO_4S$: C, 50.68; H, 5.47; N, 4.22. Found: C, 50.46; H, 5.47; N, 4.28.

EXAMPLE 27

2-Phenylthio-2-propenyl Alcohol. To a stirred solution of 36.0 g (0.64 mol) of propargyl alcohol and 0.12 g (1.8 mmol) of powdered potassium hydroxide, was added dropwise at 125 °C over 30 minutes, 60.0 g (0.54 mol) of thiophenol. The mixture was stirred for an additional 90 minutes, and allowed to cool to room temperature. The brown solution was diluted with 200 mL of ether, and extracted with three 100-mL portions of 2 N sodium hydroxide, and two 100-mL portions of water. The organic phase was dried over $MgSO_4$, filtered, and the solvent removed in vacuo from a water bath at 45 °C to give 90.4 g of a brown liquid,

which, according to NMR analysis, contained 34% of the desired isomer. The mixture was fractionally distilled through a 0.8 x 20-cm column packed with glass helices to give 29.5 g of a colorless liquid, which by NMR analysis contained 67% of the desired isomer, bp 109-116 °C/1.2 Torr. This liquid was chromatographed on silica gel (100-200 mesh, 50 g/gram of compound) with 20% EtOAc/Skelly B as eluant, to give 16.4 g (18%) of the sulfide as a colorless liquid; IR (neat) $cm^{-1}$ 3360, 1610, 1580, 1470, 1430, 1040, 740, 690; $^1H$ NMR ($CDCl_3$) δ 3.70 (t, 1H, OH), 4.10 (d, 2H, $CH_2$), 5.2 (t, J = 1 Hz, 1H, vinyl), 5.55 (t, J = 1 Hz, 1H, vinyl), 7.15-7.55 (m, 5H, phenyl).

## EXAMPLE 28

2-(Phenylsulfonyl)-2-propenyl Alcohol. A mixture of 5.03 g (30.3 mmol) of 2-(phenylthio)-2-propenyl alcohol and 12.74 g (62.7 mmol) of 85% m-chloroperbenzoic acid in 250 mL of $CH_2Cl_2$ was stirred overnight at room temperature. The mixture was extracted with three 100-mL portions of saturated $NaHCO_3$, followed by 100 mL of water. The organic layer was dried over $MgSO_4$, filtered, and the solvent removed in vacuo from a water bath at 45 °C. The residue was chromatographed on silica gel (100-200 mesh, 50 g/gram of compound) with 40% EtOAc/Skelly B as eluant, to give 4.30 g (72%) of the sulfone as a colorless oil; IR (neat) $cm^{-1}$ 3500, 1580, 1440, 1300, 1170, 1130, 1050, 950, 900, 750, 690; $^1H$ NMR ($CDCl_3$) δ 3.70 (bs, 1H, OH), 4.20 (bs, 2H, $CH_2$) 6.05 (bs, 1H, vinyl), 6.35 (bs, 1H, vinyl) 7.30-7.95 (m, 5H, phenyl).

Anal. Calcd for $C_9H_{10}O_3S$: C, 54.53; H, 5.08; S. 16.17. Found: C, 54.57; H, 5.13; S. 16.02.

## EXAMPLE 29

2-(Phenylsulfonyl)-2-propenyl N-p-Chlorophenyl Carbamate. A solution of 3.38 g (17.1 mmol) of 2-(phenylsulfonyl)-2-propenyl alcohol and 2.62 g (17.1 mmol) of p-chlorophenyl isocyanate in 15 mL of benzene was refluxed overnight. The solvent was removed in vacuo from a water bath at 45 °C to give a solid which was recrystallized twice from $CCl_4$ to give 4.25 g (71%) of the urethane, mp 104-106 °C; IR (KBr) $cm^{-1}$ 3320, 1730, 1600, 1530, 1300, 1215, 1065, 960, 820, 750, 680; $^1H$ NMR ($CDCl_3$) δ 4.85 (bs, 2H, $CH_2$), 6.16 (bs, 1H, vinyl), 6.50 (bs, 1H, vinyl), 7.05-8.00 (m, 10H, phenyl and NH).

Anal. Calcd for $C_{16}H_{14}ClNO_4S$: C, 54.63; H, 4.01; N, 3.98. Found: C, 54.54; H, 3.99; N, 4.05.

## EXAMPLE 30

2-(Methylsulfonyl)ethyl N-p-Chlorophenyl Carbamate. A solution of 2.06 g (16.6 mmol) of 2-(methylsulfonyl)ethyl alcohol and 2.55 g (16.6 mmol) of p-chlorophenyl isocyanate in 15 mL of benzene was refluxed for 30 minutes. The solvent was removed in vacuo from a water bath at 45 °C to give a solid which was recrystallized from $CHCl_3$ to give 2.98 g (65%) of the urethane, mp 147-147.5 °C; IR (KBr) 3360 $cm^{-1}$, 1720, 1600, 1490, 1310, 1230, 1130, 1070, 830, 760; $^1H$ NMR (DMSO-$d_6$-$CDCl_3$) δ 3.0 (s, 3H, $CH_3$), 3.40 (t, 2H, $CH_2SO_2$), 4.60 (t, 2H, $CH_2O$), 7.15-7.55 (m, 4H, phenyl), 9.20 (bs, 1H, NH).

Anal. Calcd for $C_{10}H_{12}ClNO_4S$: C, 43.25; H, 4.36; N, 5.04. Found: C, 43.16; H, 4.14; N, 4.99.

## EXAMPLE 31

2-Carboethoxy-2-propenyl Alcohol. To a stirred solution of 12.07 g (53.8 mmol) of triethyl phosphonacetate and 21 mL of 35-40% aqueous formaldehyde at room temperature was added dropwise a solution of 13.2 g (95.5 mmol) of $K_2CO_3$ in 50 mL of water. The temperature of the reaction mixture rose to 48 °C over the course of the addition. After the addition was complete, the mixture was stirred for 1 hour, quenched with 22 mL of saturated $NH_4Cl$ solution, and extracted with three 25-mL portions of ether. The organic layer was dried over $Na_2SO_4$, filtered, and the solvent removed in vacuo from a water bath at 45 °C to give a colorless liquid which was distilled to give 4.57 g (65%) of the colorless alcohol, bp 60-65 °C/0.9 Torr; IR (neat) $cm^{-1}$ 3450, 1710, 1635, 1450, 1400, 1300, 1260, 1160, 1050, 945, 820; $^1H$ NMR ($CDCl_3$) δ 1.30 (t, 3H, $CH_3$), 3.5-4.5 (m, 5H, $CH_2OH$ and $CO_2CH_2$), 5.90 (bs, 1H, vinyl), 6.25 (bs, 1H, vinyl).

## EXAMPLE 32

2-Carboethoxy-2-propenyl N-p-Chlorophenyl Carbamate. A solution of 2.12 g (16.3 mmol) of 2-(carboethoxy)-2-propenyl alcohol and 2.50 g (16.3 mmol) of p-chlorophenyl isocyanate in 15 mL of benzene was refluxed overnight. The solvent was removed in vacuo from a water bath at 45 °C to give a solid which was recrystallized from $CCl_4$ to give 2.99 g (65%) of the urethane, mp 93.0-93.5 °C; IR (KBr) $cm^{-1}$ 3360,

1720, 1600, 1525, 1390, 1310, 1220, 1170, 1060; $^1H$ NMR (DMSO-$d_6$/$CDCl_3$) δ 1.25 (t, 3H, $CH_3$), 4.25 (q, 2H, $CO_2CH_2$), 4.90 (s, 2H, $CH_2O$), 5.90 (bs, 1H, vinyl), 6.35 (bs, 1H, vinyl), 7.15-7.50 (m, 4H, phenyl), 8.0 (bs, 1H, NH).

Anal. Calcd. for $C_{13}H_{14}ClNO_4$: C, 55.04; H, 4.97; N, 4.94. Found: C, 54.89; H, 4.69; N, 4.89.

EXAMPLE 33

2-(Phenylsulfinyl)-2-propenyl Alcohol. To a stirred solution of 0.91 g (5.5 mmol) of 2-(phenylthio)-2-propenyl alcohol in 60 mL of $CH_2Cl_2$ at -78°C was added 1.11 g (5.5 mmol) of 85% m-chloroperbenzoic acid. The reaction mixture was stirred for 1 hour at -78°C. The cooling bath was removed and the mixture allowed to warm to room temperature. The mixture was filtered, and then extracted with three 50-mL portions of saturated $NaHCO_3$. The organic layer was dried over $MgSO_4$, filtered, and the solvent removed in vacuo from a water bath at 45°C. The residue was flash chromatographed on silica gel (230-400 mesh, 4 x 12-cm packed column) with 80% EtOAc/Skelly B as eluant, to give 0.57 g (57%) of the sulfoxide as a colorless oil; IR (neat) $cm^{-1}$ 3360, 1440, 1030, 990, 930, 750, 690; $^1H$ NMR ($CDCl_3$) δ 4.00 (s, 1H, allylic), 4.15 (s, 1H, allylic), 4.40 (bs, 1H, OH), 5.90 (t, J=1 Hz, 1H, vinyl), 6.05 (s, 1H, vinyl), 7.35-7.80 (m, 5H, phenyl).

EXAMPLE 34

trans-Phenyl β-Styryl Sulfide. To a stirred solution of 14.0 g (0.137 mol) of freshly distilled phenylacetylene at 0°C was added dropwise 15.1 g (0.137 mol) of thiophenol. The solution was stirred at room temperature overnight. The reaction mixture was distilled to give 22.2 g (76%) of a colorless liquid, bp 177-181°C/5 Torr. NMR analysis showed a trans/cis-ratio of 80/20. $^1H$ NMR ($CDCl_3$) δ 6.53 (d, J=1, Hz, 1H, cis-vinyl), 6.81 (d, J=2 Hz 1H, trans-vinyl).

EXAMPLE 35

trans-Phenyl β-Styryl Sulfone. To a slightly frozen mixture of 8.17 g (38.5 mmol) of an 80/20 trans-cis-mixture of phenyl β-styryl sulfide in 160 mL of acetic acid was added dropwise 16 mL of 30% hydrogen peroxide. The solution was refluxed for 1 hour, poured on crushed ice to give an oil which soon solidified, and was filtered. The crude solid was dissolved in 100 mL of $CH_2Cl_2$, dried over $MgSO_4$, filtered, and the solvent removed in vacuo from a water bath at 45°C to give 6.64 g (71%) of a solid which contained approximately 15% of the cis-isomer as determined by $^1H$-NMR analysis. Recrystallization from 30% EtOAc/Skelly B gave 3.2 g (34%) of the pure colorless sulfone, mp 72.5-73.5°C; $^1H$ NMR ($CDCl_3$) 6.8 (d, J=15 Hz; 1H, vinyl), 7.2-8.1 (m, 11H, vinyl and phenyl).

EXAMPLE 36

(E)-3-Phenyl-2-(phenylsulfonyl)-2-propenyl Alcohol. To a stirred solution of 2.0 g (8.2 mmol) of trans-phenyl β-styryl sulfone in 50 mL of dry THF at -78°C was added dropwise under a nitrogen atmosphere 6.0 mL (8.4 mmol) of 1.4 M n-BuLi within 5 minutes. The reddish-purple solution was stirred at -78°C for 30 minutes. Gaseous formaldehyde, generated by heating paraformaldehyde at 170°C, was passed through a 5-mm tube into the reaction mixture with the aid of a slow stream of nitrogen at -78°C for 45 minutes. The mixture was allowed to come to room temperature over a period of 30 minutes while continuing to pass formaldehyde through the reaction mixture until a pale yellow solution was obtained. The reaction was quenched with 75 mL of 5% HCl and extracted with 50 mL of ether. The organic layer was washed with two 50-mL portions of water, dried over $MgSO_4$, filtered, and the solvent removed in vacuo from a water bath at 45°C to give 2.1 g (93%) of the crude alcohol as a brown oil. The oil was flash chromatographed in two 1.05 g batches on silica gel (230-400 mesh, 4 x 19-cm column) with 50% ether/Skelly B as eluant to give 0.99 g (44%) of a light yellow solid, mp 85-87°C. Recrystallization from 40% ether/Skelly B gave the pure colorless alcohol, mp 88-89°C; IR (KBr) $cm^{-1}$ 3470, 1625, 1445, 1285, 1145, 1020, 770, 755, 735, 700, 680; $^1H$ NMR ($CDCl_3$) δ 2.63 (t, 1H, OH), 4.37 (d, 2H, $CH_2O$), 7.27-8.13 (m, 11H, phenyl and vinyl).

Anal. Calcd for $C_{15}H_{14}O_3S$: C, 65.67; H, 5.14; S, 11.69. Found: C, 65.60; H, 5.12; S, 11.31.

EXAMPLE 37

(E)-3-Phenyl-2-(phenylsulfonyl)-2-propenyl N-p-Chlorophenyl Carbamate. A solution of 0.56 (2.04 mmol) of (E)-3-phenyl-2-(phenylsulfonyl)-2-propenyl alcohol and 0.32 g (2.08 mmol) of p-chlorophenyl isocyanate in 10 mL of benzene was refluxed for 120 hours. The solvent was removed in vacuo from a water bath at 45 °C. The crude solid was recrystallized from 20% EtOAc/Skelly B to give 0.41 g (47%) of the colorless urethane, mp 138.5-139.5 °C; IR (KBr) $cm^{-1}$ 3320, 1695, 1590, 1520, 1305, 1230, 1150, 1045; $^1H$ NMR ($CDCl_3$) δ 5.07 (s, 2H, $CH_2O$), 6.52 (bs, 1H, NH), 7.25-8.08 (m, 14H, phenyl), 8.15 (s, 1H, vinyl).

Anal. Calcd for $C_{22}H_{18}ClNO_4S$: C, 61.75; H, 4.24; N, 3.27. Found: C, 61.54; H, 4.27; N, 3.11.

EXAMPLE 38

Thiophenyl Trimethylsilyl Methane. To a stirred solution of 143 mL of 1.4 M n-BuLi (0.20 mol) in 55 mL of dry THF, was added dropwise at room temperature under a nitrogen atmosphere 24.8 g (0.20 mol) of thioanisole. Upon completion of the addition, the yellow mixture began to reflux spontaneously. The mixture was stirred at room temperature for 3 hours. Upon addition of 21.84 g (0.20 mol) of chlorotrimethylsilane, the mixture again began to reflux spontaneously. After stirring at room temperature overnight, the mixture was quenched with 100 mL of 5% of HCl. The aqueous layer was separated, and the organic layer was dried over $MgSO_4$, filtered, and the solvent removed in vacuo from a water bath at 45 °C. The crude product was distilled to give 19.22 g (49%) of the colorless sulfide, bp 84-86 °C/0.3 Torr; $^1H$ NMR ($CDCL_3$) δ 0.17 (s, 9H, $Si(CH_3)_3$), 2.13 (s, 2H, $CH_2S$), 7.0-7.42 (m, 5H, phenyl).

EXAMPLE 39

Benzenesulfonyl Trimethylsilyl Methane. To a stirred mixture of 14.1 g (69.4 mmol) of 85% m-chloroperbenzoic acid in 300 mL of $CH_2Cl_2$ at 0 °C was added dropwise 6.82 g (34.7 mmol) of thiophenyl trimethylsilyl methane. The mixture was stirred at 0 °C for 3 hours and then at room temperature overnight. The mixture was extracted with three 75-mL portions of saturated $NaHCO_3$. The organic layer was dried over $MgSO_4$, filtered, and the solvent removed in vacuo from a water bath at 45 °C. The crude product was Kugelrohred at 115 °C/0.1 Torr to give 6.83 g (86%) of the clear sulfone; $^1H$ NMR ($CDCl_3$) δ 0.27 (s, 9H, $Si(CH_3)_3$), 2.8 (s, 2H, $CH_2SO_2$), 7.13-8.05 (m, 5H, phenyl).

EXAMPLE 40

2,2-Diphenyl-1-(phenylsulfonyl) Ethene. To a stirred solution of 2.0 g (8.76 mmol) of benzenesulfonyl trimethylsilyl methane in 20 mL of dry THF at 0 °C, was added dropwise under a nitrogen atmosphere 6.25 mL of 1.4 M n-BuLi (8.75 mmol). The reddish-orange solution was stirred at 0 °C for 30 minutes. To the solution was added 1.60 g (8.78 mmol) of benzophenone. The solution was stirred at 0 °C for 2 hours, then at room temperature overnight. The reaction mixture was diluted with 50 mL of 5% HCl, and extracted with three 25-mL portions of water, dried over $MgSO_4$, filtered, and the solvent removed in vacuo from a water bath at 45 °C. The crude product was recrystallized from 15% EtOAc/Skelly B to give 1.25 g (44%) of the slightly yellow sulfone, mp 111.0-112.5 °C; $^1H$ NMR ($CDCl_3$) δ 7.02 (s, 1H, vinyl), 7.12-7.72 (m, 15H, phenyl).

EXAMPLE 41

3,3-Diphenyl-2-(phenylsulfonyl)-2-propenyl Alcohol. To a stirred solution of 1.25 g (3.90 mmol) of 2,2-diphenyl-1-(phenylsulfonyl) ethene in 20 mL of dry THF at -78 °C, was added dropwise under a nitrogen atmosphere 2.8 mL of 1.4 M n-BuLi (3.90 mmol). The dark black solution was stirred at -78 °C for 30 minutes. Gaseous fomaldehyde, generated by heating paraformaldehyde at 170 °C, was passed through a 5-mm tube into the reaction mixture with the aid of a slow stream of nitrogen at -78 °C for 30 minutes. The mixture was allowed to come to room temperature over a period of 30 minutes while continuing to pass formaldehyde through the reaction mixture until a pale yellow color was observed. The mixture was stirred overnight at room temperature. The mixture was quenched with 50 mL of 5% HCl, and extracted with three 25-mL portions of $CH_2Cl_2$. The organic layer was dried over $MgSO_4$, filtered, and the solvent removed in vacuo from a water bath at 45 °C. The resulting crude oil was flash chromatographed on silica gel (230-400 mesh, 5 x 16-cm column) with 30% ether/Skelly B as eluant to give 0.47 g (34%) of the colorless alcohol. In a melting point capillary the compound does not melt but decomposes above 290 °C with blackening; IR

(KBr) cm$^{-1}$ 3480, 3040, 1590, 1480, 1440, 1370, 1280, 1130, 1020, 960, 790, 730, 700, 680, 610; $^1$H NMR ($CDCl_3$) δ 3.47 (t, 1H, OH), 4.61 (d, 2H, $CH_2O$), 6.75-7.58 (m, 15H, phenyl).

Anal. Calcd for $C_{12}H_{18}O_3S$: C, 71.98; H, 5.18; S, 9.15. Found: C. 71.97; H, 5.22; S, 9.02.

EXAMPLE 42

3,3-Diphenyl-2-(phenylsulfonyl)-2-propenyl N-p-Chlorophenyl Carbamate. A solution of 0.35 g (1.0 mmol) of 3,3-diphenyl-2-(phenylsulfonyl)-2-propenyl alcohol and 0.15 g (1.0 mmol of p-chlorophenyl isocyanate in 2 mL of benzene was refluxed for 18 hours. The solvent was removed in vacuo from a water bath at 45°C. Recrystallization from 95% EtOAc/EtOH gave 0.38 g (78%) of the colorless urethane. In a melting point capillary the compound does not melt but decomposes above 250°C; IR (KBr) cm$^{-1}$ 3310, 1730, 1590, 1530, 1490, 1300, 1205, 1140, 1050, 825, 700, 680; $^1$H NMR ($CDCl_3$) δ 5.13 (s, 2H, $CH_2O$), 6.80 (bs, 1H, NH), 6.91-7.58 (m, 19H, phenyl).

Anal. Calcd for $C_{28}H_{22}ClNO_4S$: C, 66.73; H, 4.40; N, 2.78. Found: C, 66.51; H, 4.44; N, 2.54.

EXAMPLE 43

cis-Phenyl β-Styryl Sulfide. To a solution of 150 mL of anhydrous ethanol maintained under nitrogen was added 3.45 g (0.15 mol) of sodium. The sodium dissolved within 25 minutes, and to the resulting sodium ethoxide solution was added 16.5 g (0.15 mol) of thiophenol. The solution was brought to reflux, and 15.0 g (0.15 mol) of phenylacetylene was added dropwise. The solution was refluxed overnight, cooled, and poured over crushed ice. The precipitate was filtered, dissolved in 100 mL of $CH_2Cl_2$, dried over $MgSO_4$, filtered, and the solvent removed in vacuo from a water bath at 45°C. The crude product was recrystallized from Skelly F to give 22.61 g (71%) of the sulfide, mp 43.0-44.5°C; $^1$H NMR ($CDCl_3$) δ 6.51 (d, 2H, vinyl), 7.13-7.67 (m, 10H, phenyl).

EXAMPLE 44

(Z)-3-Phenyl-2-(thiophenyl)-2-propenyl Alcohol. To a solution of 1.0 g (4.7 mmol) of cis-phenyl β-styryl sulfide in 20mL of dry THF at -78°C was added dropwise under a nitrogen atmosphere 5.0 mL (7.0 mmol) of 1.4 M n-BuLi within 5 minutes. The light yellow solution was stirred at -78°C for 30 minutes. Gaseous formaldehyde, generated by heating paraformaldehyde at 170°C, was passed through 5-mm tube into the reaction mixture with the aid of a slow steam of nitrogen at -78°C for 3 hours. The mixture was allowed to come to room temperature over a period of 30 minutes and starred overnight. The mixture was quenched with 30 mL of 5% HCl and extracted with three 25-mL portions of $CH_2Cl_2$. The organic layer was dried over $MgSO_4$, filtered, and the solvent removed in vacuo from a water bath at 45°C to give a brown oil. The oil was flash chromatographed on silica gel (230-400 mesh, 4 x 18-cm packed column) with 20% EtOAc/Skelly B as eluant to give 0.50 g (45%) of the alcohol, mp 64.0-65.0°C; IR (KBr) cm$^{-1}$ 3240, 3140, 1580, 1470, 1090, 1070, 1010, 740, 695; $^1$H NMR ($CDCl_3$) δ 1.82 (broad, 1H, OH), 4.17 (d, 2H, $CH_2O$), 7.15-7.75 (m, 11H, phenyl and vinyl).

Anal. Calcd for $C_{15}H_{14}OS$: C, 74.35; H, 5.82; S, 13.23. Found: C, 74.35; H, 5.90; S, 13.18.

EXAMPLE 45

(Z)-3-Phenyl-2-(phenylsulfonyl)-2-propenyl Alcohol. A mixture of 0.47 g (1.94 mmol) of (Z)-3-phenyl-2-(thiophenyl) -2-propenyl alcohol and 0.79 g (3.89 mmol) of 85% m-chloroperbenzoic acid in 10 mL of $CH_2Cl_2$ was stirred at room temperature overnight. The mixture was diluted with 40 mL of $CH_2Cl_2$ and extracted with three 50-mL portions of saturated $NaHCO_3$. The organic layer was dried over $MgSO_4$, filtered, and the solvent removed in vacuo from a water bath at 45°C to give a clear oil. The oil was recrystallized from 20% EtOAc/Skelly B to give 0.27 g (51%) of the sulfone, mp 67.5-69.0°C; IR (KBr) cm$^{-1}$ 3280, 3180, 1445, 1300, 1150, 1080, 1025, 990, 750, 730, 650, 610; $^1$H NMR ($CDCl_3$) 2.85 (s, 1H, OH), 4.67 (s, 2H, $CH_2O$), 7.21-7.83 (m, 11H, phenyl and vinyl).

Anal. Calcd for $C_{15}H_{14}O_3S$: C, 65.67; H, 5.14; S, 11.69. Found: C, 65.45; H, 4.97; S, 11.82.

EXAMPLE 46

(Z)-3-Phenyl-2-(phenylsulfonyl)-2-propenyl N-p-Chlorophenyl Carbamate. A solution of 0.22 g (0.80 mmol) of (Z)-3-phenyl-2-(phenylsulfonyl)-2-propenyl alcohol and 0.12 g (0.78 mmol) of p-chlorophenyl

isocyanate in 4 mL of benzene was refluxed overnight. The solvent was removed in vacuo from a water bath at 45 °C. The crude product was recrystallized from 15% EtOAc/Skelly B to give 0.23 g (70%) of the urethane, mp 124.0-125.0 °C; IR (KBr) $cm^{-1}$ 3320, 1740, 1590, 1525, 1300, 1210, 1120, 1050, 830, 740, 690; $^1H$ NMR ($CDCl_3$) δ 5.23 (s, 2H, $CH_2O$), 7.05 (bs, 1H, NH), 7.22-7.83 (m, 15H, phenyl and vinyl).

Anal. Calcd for $C_{22}H_{18}ClNO_4S$: C, 61.75; H, 4.24; N, 3.27. Found: C, 61.70; H, 4.09; N, 3.21.

EXAMPLE 47

2-(Phenylsulfonyl)-2-propenyl chloroformate. Using the procedure from Example 5, 2-(phenylsulfonyl)-2-propenyl alcohol can be readily converted to 2-(phenylsulfonyl)-2-propenyl chloroformate.

EXAMPLE 48

2-Carbethoxy-2-propenyl chloroformate. Using the procedure from Example 5, 2-carbethoxy-2-propenyl alcohol can be readily converted to 2-carbethoxy-2-propenyl chloroformate.

EXAMPLE 49

2-(Phenylsulfonyl)-2-propenyl chloroformate. Using the procedure from Example 5,2-(phenylsulfonyl)-2-propenyl alcohol can be readily converted to 2-(phenylsulfonyl)-2-propenyl chloroformate.

EXAMPLE 50

(E)-3-Phenyl-2-(phenylsulfonyl)-2-propenyl chloroformate. Using the procedure from Example 5, (E)-3-phenyl-2-(phenylsulfonyl)-2-propenyl alcohol can be readily converted to (E)-3-phenyl-2-(phenylsulfonyl)-2-propenyl chloroformate.

EXAMPLE 51

(Z)-3-Phenyl-2-(phenylsulfonyl)-2-propenyl chloroformate. Using the procedure from Example 5, (Z)-3-phenyl-2-(phenylsulfonyl)-2-propenyl alcohol can be readily converted to (Z)-3-phenyl-2-(phenylsulfonyl)-2-propenyl chloroformate.

EXAMPLE 52

3,3-Diphenyl-2-(phenylsulfonyl)-2-propenyl chloroformate. Using the procedure from Example 5, 3,3-diphenyl-2-(phenylsulfonyl)-2-propenyl alcohol can be readily converted to 3,3-diphenyl-2-(phenylsulfonyl)-2-propenyl chloroformate.

EXAMPLE 53

Benzothiophenesulfone-2-methanol

Oxidation of 0.5 g of benzothiophene-2-methanol [F.F. Blicke and D.G. Sheets, J. Am. Chem. Soc., 71, 2856 (1949)] with 2 eqs. of m-chloroperbenzoic acid in 20 mL methylene dichloride gave in 51% yield the sulfone alcohol, mp 112-113 °C.

Anal. Calcd for $C_9H_8O_3S$: C, 55.10; H, 4.08. Found: C, 54:81; H, 4.10.

The corresponding urethane derived from p-chlorophenyl isocyanate had mp 154-56 °C.

Anal. Calcd for $C_{16}H_{12}ClNO_4S$:
C, 54.94; H, 3.43; N, 4.01; Cl, 10.16. Found: C, 54.87; H, 3.48; N, 3.94; Cl, 10.35.

EXAMPLE 54

Similarly, by using the procedures described herein and the appropriate starting materials, the following compounds are prepared:

3,3-dimethyl-2-(phenylsulfonyl)-2-propenyl alcohol
3,3-dimethyl-2-(phenylsulfonyl)-2-propenyl chloroformate
Benzothlophenesulfone-2-methyl chloroformate.

EXAMPLE 55

(Z)-3-phenyl-2-thiomethyl-2-propenyl alcohol

Using the procedure of Example 44 and substituting cis-methyl-B-stryl sulfide for cis-phenyl B-stryl sulfide, the above compound can be prepared.

EXAMPLE 56

Z-3-phenyl-2-(methysulfonyl)-2-propenyl alcohol

Using the procedure of Example 45 and substituting the Z-3-phenyl-2-thiomethyl-2-propenyl alcohol for (Z)-3-phenyl-2-thiophenyl-2-propenyl alcohol, the above compound can be prepared.

EXAMPLE 57

Z-3-phenyl-2-(methylsulfonyl)-2-propenyl chloroformate. The product of Example 56 is reacted with phosgene in accordance with the procedure described in Example 25 to prepare the above product.

EXAMPLE 58

The products of Examples 25,26,28, and 29, 30,37, 42, 46, 47, 48, 49, 50, 51, 52, 54 and 58 can react with a carboxy protected amino acid, such as L-Phe-$O^+$, in accordance with the procedure described hereinabove in Example 17 to prepare the corresponding protected amino acid. These amino acids are in turn hydrolyzed with acid, e.g., triflouoracetic acid, and then reacted with cyanuric fluoride as described herein to prepare the corresponding protected amino acid fluoride. For example, when the product of Example 57 is reacted with a carboxy protected amino acid, such as L-Phe-$O^+$ in accordance with the procedure described in Example 17, the product obtained is Mspoc-L-Phe. This product is then reacted with cyanuric fluoride in accordance with the procedure described herein to provide Mspoc-L-Phe-F.

EXAMPLE 59

USE OF FMOC-AA-F IN PEPTIDE SYNTHESIS

I General Method for Executing Rapid FMOC/4-AMP or FMOC/TAEA Peptide Synthesis. Five millimeters of a 0.1 M solution of the C-terminal starting amino acid ester, 5 mL of $CHCl_3$ containing 0.75 mmol of the appropriate

$$\text{FMOC-}\overset{\displaystyle X\atop |}{\text{AA}}\text{-Y},$$

(wherein Y is halo, i.e., chloro or fluoro) and 5 mL of 5% $Na_2CO_3$ solution were combined and the two phase mixture stirred vigorously for 10 minutes. The organic phase was separated and treated with 5 mL of 4-AMP or TAEA. After 30 minutes 40 mL of $CHCl_3$ was added and the organic phase washed with five 25-mL portions of 10% phosphate buffer of pH 5.5. Alternatively two 25-mL portions of saturated NaCl solution was used to remove excess 4-AMP or TAEA prior to extractions with the phosphate buffer. The organic phase was concentrated in vacuo to a volume of 5 mL on a rotary evaporator and the resulting $CHCl_3$ solution used analogously for treatment with the next FMOC amino acid halide.

II Preparation of FMOC-Val-Asp($CMe_3$)-Val-Leu-Leu-Ser$(CMe)_3$-Tyr($CMe_3$)-$OCMe_3$. The general procedure described above was followed beginning with 330 mg (1 mmol) of H-Tyr-($CMe_3$)-$OCMe_3$ HCl, 462 mg (1.2 mmol) of FMOC-Ser-($CMe_3$)-F, 10 mL of methylene dichloride and 10 mL of 5% sodium carbonate solution. The mixture was stirred for 15 minutes, the organic layer separated and treated with 7.5 mL of TAEA for 30 minutes. The solution was washed with three 10-mL portions of satd sodium chloride solution and three 15-mL portions of phosphate buffer (pH 5.5). The organic layer was then treated in the same way in sequence with the remaining FMOC amino acid halides (FMOC-Leu-Cl twice, FMOC-Val-Cl, FMOC-Asp($CMe_3$)-F and FMOC-Val-Cl a second time). Evaporation of the organic layer was followed by column chromatorgraphy to give the protected heptapeptide, mp 249-251 °C, in 50-60%

yield MSFAB: 1255 (M+1); calculated 1253.8 (M).

EXAMPLE 60

Coupling of FMOC-Phe-F with H-Leu-OMe. Five millimeters of a 0.1 M solution of H-Leu-OMe in $CHCl_3$ was treated with 5 mL of $CHCl_3$ containing 0.6 mmol of FMOC-Phe-F (mp 118-120°) and 5 mL of 5% $Na_2CO_3$ solution. The two-phase mixture was stirred vigorously for 10 minutes, the layers separated and the organic layer dried over $MgSO_4$ and evaporated with a rotary evaporator. Without purification the crude residue (90%) was examined by HPLC analysis which showed no evidence for the DL-diastereomer ( 0.1%). The two diasteromers are readily separated with a mobile phase consisting of 1% isopropyl alcohol in hexane (retention times: LL, 13.26 minutes; DL-17.13 minutes at flow rate 1.5 mL/minutes) using a Waters Radial Pak normal silica gel column 10 u, Z-module fitting.

EXAMPLE 61

CBZ-alanine Fluoride

CBZ-Ala (1 mmole) in dry $CH_2Cl_2$ is kept under nitrogen and treated with cyannuric fluoride (8 mmol) and pyridine (1 mmole) to form the corresponding CBZ-Ala-F.

EXAMPLE 62

N-formyl-O-(t-butyl) serine Fluoride

N-formyl serine (1 mmole) in dry $CH_2Cl_2$ under nitrogen is treated with cyannuric fluoride (8 mmol) and pyridine (1 mmol) to form the above-identified product.

EXAMPLE 63

2-(t-butylsulfonyl)-2-propenyloxycarbonyl-1-phenylalanine Fluoride

A. t-Butyl Allyl Sulfide. To a solution of 350 mL of anhydrous ethanol maintained under nitrogen was slowly added 22.99 g (1 mol) of sodium spheres. The sodium dissolved within 90 minutes, and to the resulting sodium ethoxide solution was added 90.19 g (1 mol) of t-butyl mercaptan with mechanical stirring. Allyl bromide (120.98 g; 1 mol) was then added dropwise to the mechanically stirred sodium t-butyl thiolate solution. After the addition was complete, the mixture was refluxed for 10 minutes, the solution allowed to cool, the precipitated sodium bromide filtered, and the ethanol removed by distillation at atmospheric pressure. The residue was diluted with 200 mL of water, and the layers separated. The aqueous layer was extracted with five 40-mL portions of ether. The combined organic layers were extracted with 150 mL of water, the organic layer dried over $MgSO_4$, filtered, and the solvent removed in vacuo from a water bath at 45°C to give a yellow liquid. Distillation through a 0.8 x 15-cm fractionating column gave 56.16 g (45%) of the sulfide as a colorless liquid, bp 139-141°C.

B. 1,3-Dibromo-2-(t-butylsulfonyl) Propane. To a stirred solution of 17.37 g (0.13 mol) of t-butyl allyl sulfide in 133 mL of $CCl_4$ at -24°C ($CCl_4$/dry ice) was added dropwise a solution of 21.33 g (0.13 mol) of $Br_2$ in 67 mL of $CCl_4$. A yellow solid precipitated during the addition. The mixture was warmed to room temperature and stirred for 10 minutes following complete solution of the yellow solid. The resulting solution was poured into a mixture of 55.50 g (0.27 mol) of 85% m-chloroperbenzoic acid in 490 mL of $CH_2Cl_2$ kept at -24°C, and the mixture stirred for 30 minutes at this temperature. The cooling bath was then removed and the mixture stirred at room temperature overnight. The precipitated m-chlorobenzoic acid was filtered and the filtrate washed with three 200-mL portions of saturated $NaHCO_3$, followed by 200 mL of water. The organic layer was dried over $MgSO_4$, filtered, and the solvent removed in vacuo from a water bath at 45°C. The crude product was recrystallized from 20% EtOAc/Skelly B to give 32.02 g (75%) of the dibromide, mp 139-140°C.

C. 2-(t-Butylsulfonyl)-2-propenyl Bromide. A mixture of 16.78 g (0.052 mol) of 1,3-dibromo-2-(t-butylsulfonyl) propane and 14 mL (0.12 mol) of 2,6-lutidine in 55 mL of $CH_2Cl_2$ was refluxed for 75 minutes. The solution was allowed to cool to room temperature and extracted with three 80-mL portions of 5% HCl followed by 80 mL of water. The organic layer was dried over $MgSO_4$, filtered, and the solvent removed in vacuo from a water bath at 45°C to give 11.46 g (91%) of the allyl bromide as a white solid, mp 40.5-42.0 ° C, which was used without further purification.

D. 2-(t-Butylsulfonyl-2-propenyl Alcohol. A mixture of 8.55 g (35.3 mmol) of 2- (t-butylsulfonyl)-2-propenyl bromide and 5.31 g (78.1 mmol) of sodium formate in 150 ml of methanol was refluxed overnight. The solution was allowed to cool and concentrated to 50 mL with the aid of a water aspirator, resulting in the precipitation of excess sodium formate. The residue was diluted with 150 mL of water and extracted with five 50-mL portions of $CH_2Cl_2$. The organic layer was dried over $MgSO_4$, filtered, and the solvent removed in vacuo from a water bath at 45 ° C. The crude product was recrystallized from 15% EtOAc/Skelly F to give 4.30 g (68%) of the alcohol as a colorless solid, mp 53.5-54.5 ° C.

E. 2-(t-Butylsulfonyl)-2-propenyl Chloroformate. To a solution of 6.67 g (37.4 mmol) of 2-(t-butylsulfonyl)-2-propenyl alcohol in 27 mL of dry THF at 0 ° C was added in one portion 27 mL of phosgene. The solution was stirred for 1 hour at 0 ° C and allowed to stand at room temperature overnight. Excess phosgene and solvent were removed under reduced pressure. The crude product was recrystallized from 25% ether/Skelly B to give 8.23 g (91%) of the chloroformate as a colorless solid, mp 56.5-57.7.

F. 2-(t-Butylsulfonyl)-2-propenyloxycarbonyl-L-phenylalanine. A solution of 4.57 g (19.0 mmol) of 2- (t-butylsulfonyl)-2-propenyl chloroformate and 5.64 g (18.6 mmol) of t-butyl L-phenylalaninate hydrophosphite in 90 mL of $CH_2Cl_2$ was stirred in the presence of 165 mL of 5% $NaHCO_3$ at room temperature for 2 hours. The aqueous phase was separated, and the organic phase washed with three 75-mL portions of 5% HCl. The organic phase was dried over $MgSO_4$, filtered, and the solvent removed in vacuo from a water bath at 45 ° C. The resulting oil was dissolved in 36 mL of 50% $CH_2Cl_2$/trifluoroacetic acid, and the solution stirred at room temperature for two hours. Excess trifluoracetic acid and solvent were removed in vacuo from a water bath at 150 ° C. The resulting oil was crystallized from ether/Skelly F to give approximately 6.25g (91%) of the colorless acid, mp 88.0-89.5 ° C.

G. 2-(t-butylsulfonyl)-2propenyloxycarbonyl-L-phenylalanine fluoride. The product formed hereinabove in Example 17F (1 mmol) is placed in dry $CH_2Cl_2$ under nitrogen and treated with cyannuric fluoride (8 mmol) and pyridine (1 mmol) to form the above-identified product.

EXAMPLE 64

BOC-Phe-F.

BOC-Phe (1 mmole) in dry $CH_2Cl_2$ under nitrogen is treated with cyannuric fluoride (8 mmol) and pyridine (1 mmole) to form the above-identified product.

EXAMPLE 65

Coupling of Bspoc-Phe-F with H-Leu-OMe

Five millimeter of 0.1 M solution of H-Leu-OMe in $CHCl_3$ was treated with 5 ml of $CHCl_3$ containing 0.6 mmol of Bspoc-phe-F and 5 ml of 5% $Na_2CO_3$ solution. The two phase mixture is stirred vigorously for 10 minutes, the layers separated and the organic layer dried over $MgSO_4$ and evaporated to afford the coupled depeptide.

Bspoc-Phe-Leu-OMe

EXAMPLE 66

BIMOC-Phe-F

A. 1-Bromo-2-bromomethylnaphthalene. To a solution of 103 g of 1-bromo-2-methylnaphthalene (bp 98-120 °/0.5 mm, prepared in 90% yield by the method of Adams and Binder in JACS, 63, 2771(1941) and 82 g or N-bromosuccinimide in 1030 mL of $CCl_4$ was added 0.54 g of dibenzoyl peroxide. The reaction mixture was refluxed with stirring for 3 h. After another 0.54g of dibenzoyl peroxide was added, the mixture was refluxed for 3 hours. The solution was allowed to stand at room temperature overnight, and the resulting suspension was brought to the boiling point and filtered while hot. Concentration of the filtrate gave 139 g (99.4%) of the bromide as a light yellow solid, mp 104-107 ° C, which was recrystallized from $CCl_4$ to give 128 g (91.6%) of pure 1-bromo-2-bromomethylnaphthalene as colorless crystals, mp 106.5-107.5 ° C.

B. Diethyl 2-(1-Bromo-2-naphthylmethyl)malonate. To a solution of NaOEt prepared from 9.66 g of sodium and 210 mL of dry EtOH was added 63.76 mL of diethyl malonate and the reaction mixture

refluxed for 2 h. To the resulting yellow solution was added in small portions 126 g of 1-bromo-2-bromomethylnaphthalene and the reaction mixture refluxed for 16 h. Distillation of ethanol from an oil bath (100-110 °C) through a simple Claisen head gave a yellow suspension, to which was added 350 mL of $CH_2Cl_2$ and 350 mL of $H_2O$. The aqueous layer was extracted twice with 120-mL portions of $CH_2Cl_2$, and the combined organic layer was washed three times with 100-mL portion of $H_2O$, dried ($MgSO_4$) and evaporated to give 152 g (95.6%) of the ester as a yellow solid, mp 60-65 °C. Recrystallization from acetic acid gave 137 g (86.2%) of the pure diester as a colorless solid, mp 77-79 °C.

C. β-(1-Bromo-2-naphthyl)propionic Acid. To a solution of 160 g of the product formed in B hereinabove in 239 mL of methanol was added 538 mL of 5N NaOH solution. The mixture was refluxed for 135 min, and cooled by means of an ice bath to 0 °C. To the reaction mixture was added 320 mL of ice water, and the resulting precipitate was collected by filtration and washed several times with small portions of ice water. To the ice-cold, stirred filtrate was added dropwise 5N HCl solution until the solution was weakly acidic. The precipitate was filtered and washed several times with small portions of water. Drying gave 86 g (63.1%) of crude diacid as a light yellow solid, mp 154-157 °C. Recrystallization from water gave 80 g (58.7%) of pure diacid as colorless crystals, mp 157-159 °C. A suspension of 47.5 g of the crude malonic acid in 968 mL of 6N HCl solution was refluxed for 16 h. The mixture was cooled by means of an ice bath to 0 °C, treated with 650 mL of $CH_2Cl_2$, and stirred for 15 min. The aqueous layer was extracted twice with 100-mL portions of water, dried ($MgSO_4$), and evaporated to give 30.3 g (74%) of yellow solid, mp 116-119 °C, which was recrystallized from alcohol to give 28 g (68%) of the acid as colorless crystalls, mp 123-124 °C.

D. β-(1-Bromo-2-naphthyl)propionyl chloride. To a solution of 40.16 g of the product formed in C hereinabove in 802 mL of $CH_2Cl_2$ was added 19.3 mL of thionyl chloride. The mixture was refluxed for 4 h, cooled to room temperature, and the solvent evaporated from a water bath (40-50 °C) with a rotary evaporator (10 mm) to give a red-brown residue. In order to remove traces of thionyl chloride, small portions of $CH_2Cl_2$ were added and the solution reevaporated three times. Eventually the crude acid chloride was obtained as a red-brown oil. The crude product was used immediately for the next step without further purification.

E. 4-Bromobenz[f]indan-1-one. To an ice cold, stirred solution of the above crude β-(1-bromo-2-naphthyl)propionyl chloride in 802 mL of dry $CH_2Cl_2$ was added 26.11 g of anhydrous $AlCl_3$ carefully. The reaction mixture was refluxed for 2 h, cooled to room temperature and treated carefully while stirring with 900 mL of ice-water followed by 75 mL of conc. HCl. The brown precipitate was filtered and washed five times with small portions of $CH_2Cl_2$. The aqueous layer was separated and extracted twice with 100-mL portions of $CH_2Cl_2$. The combined $CH_2Cl_2$ extracts were washed three times with small portions of $H_2O$, dried ($MgSO_4$) and evaporated from a water bath (50-60 °C) with a rotary evaporator (7 mm) to give 30.1 g (80.1%) of the crude ketone as a yellow solid, mp 146-149 °C, which could be recrystallized from acetic acid to give 28.5 g (76%) of the pure ketone as colorless crystals, mp 149-151 °C.

F. Benz[f]indan-1-ol. To an ice-cold solution of 22 g of the product formed in E hereinabove in 150 mL of dry THF was added carefully in small portions 16.1 g of $LiAlH_4$. Subsequently, another 270 mL of dry THF was added to the suspension and the mixture was refluxed for 8 days. The reaction mixture was cooled to 0 °C by means of an ice-bath and treated dropwise with 50 mL of ice-water followed by 1080 mL of 10% $H_2SO_4$ solution. The mixture was extracted three times with 100-mL portions of ether and the combined ether extracts washed three times with 150-mL portions of $H_2O$, dried ($MgSO_4$), and evaporated from a water bath (60-70 °C) with a rotary evaporator (10 mm) to give 9.2 g (59.3%) of the crude alcohol as a light yellow solid, mp 135-139 °C. Recrystallization from benzene (45 mL) gave 8.6 g (55.4%) of pure alcohol as colorless crystals, mp 139-141 °C.

G. Benz[f]indene. A solution of 8.6 g of benz[f]indan-1-ol in 250 mL of 10% $H_2SO_4$ was refluxed for 24 h. After cooling to room temperature the reaction mixture was extracted with three 150-mL portions of a mixture of benzene and hexane (1:2). The extracts were washed three times with 100-mL portions of water, dried ($MgSO_4$), and evaporated to give 7.6 g (98%) of a colorless solid, mp 160-163 °C. Recrystallization from 340 mL of 95% ethanol gave 6.6 g (85%) of the hydrocarbon as colorless crystals: mp 163-164 °C.

H. Benz[f]indene-1-methanol. A 1.0 M solution of n-butyllithium (44 mL, 44 mmoles) was added dropwise under a nitrogen atmosphere to a stirred solution of 5 g (30.12 mmoles) of benz[f]indene in 140 mL of anhydrous ether and 20 mL of anhydrous THF cooled by means of a Dry Ice-acetone bath to -70 °C. The temperature of the reaction mixture was not allowed to exceed -50 °C. Benzindenyl lithium soon started to precipitate as small red crystals. After completion of the addition (about 2 hours), the reaction mixture was stirred at -70 °C for another 45 minutes before introduction of formaldehyde. Paraformaldehyde, 13.5 g. dried overnight in vacuum over phosphorus pentoxide, was stirred and heated in a dry

flask placed in an oil bath at 175-195 °C. The formaldehyde gas was led through a 7-mm glass tube into the benz[f]indenyl lithium solution (held below -50 °C) by a stream of dry nitrogen. The temperature was not allowed to exceed -50 °C. After completion of the addition, 280 mL of 10% HCl solution was slowly poured into the stirred reaction mixture. The mixture was stirred for 15 minutes at room temperature. After the ether layer was separated, the aqueous solution was extracted twice with 50-mL portions of ether and the combined ether solution was washed with small portions of water until neutral. The ether solution was dried ($MgSO_4$) and evaporated to give a light brown oil (6 g). Storage in the freezer gave a soft yellow solid which was purified by chromatography (100 g of silica gel, 1:1 ethyl acetate/hexane) to give 3.5 g (59%) of the alcohol as a yellow solid. The NMR showed the solid to be a mixture of benz[f]-indene-1-methanol ( 95%) and benz[f]indene-3-methanol. Several recrystallizations from ligroin (bp 88-89 °C) gave 3.0 g (50.6%) of pure, colorless benz[f]indene-1-methanol: mp 115-116 °C.

M. The BIMOC-Phe (1 mmole) formed in L hereinabove in dry $CH_2Cl$ is kept under nitrogen and treated with Cyannuric fluoride (8 mmol) and pyridine (1 mmol) to form the corresponding BIMOC-Phe-F.

## EXAMPLE 67

### Benz [e] indene-3-methyloxycarbonyl-phenylalanine acid fluoride

A. Benz [e]-indene-3-methanol

1-Keto-4,5-benzindane-3-carboxylic acid, [which was prepared in accordance with the procedure described by T. N. Poltabiraman and W. B. Lawson in J.Biol. Chem, 247, 302a (1972) the contents of which are incorporated herein by reference] is reduced by sodium borohydride to form the corresponding 3-carboxylic acid -1-ol. Dehydration with sulfuric acid give the corresponding unsaturated acid. The unsaturated acid was then reduced by $LiAlH_4$ to form the 3-methanol derivative:

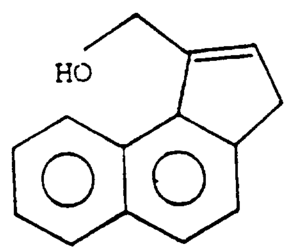

B. N-[Benz[e] indene-3-methyloxycarbony] phenylalanine.

This product is prepared from the product of A hereinabove by following the procedure described in Ex. 20 I-L hereinabove

C. N-[Benz[e] indene 1-methoxycarbonyl] phenylalanine acid fluoride

The product B formed hereinabove (1 mmol) in dry $Ch_2Cl_2$ is kept under nitrogen and treated with cyannuric fluoride (8 mmol) and pyridine (1 mmol) to form the above product.

## EXAMPLE 68

### Benz [e] indene-1-methyloxy carbonylphenylalanine acid fluoride

A. Benz [e]-indene-1-methanol

3-Keto-4,5-benzindan-1-carboxylic acid, which was prepared in accordance with the procedure described by T.N. Paltabiraman and W.B. Lawson, in J. Biol. Chem. 242, 3029(1941) is reduced to the corresponding 1-carboxylic acid-3-ol- by $NaBH_4$. Dehydration with $H_2SO_4$ gave the unsaturated acid which was then reduced to benz (e)-indene-1-methanol by means of $LiAlH_4$.

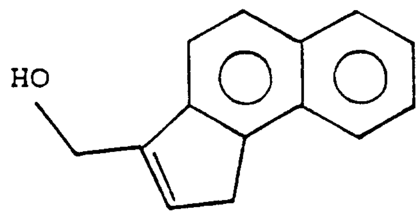

B. N-(Benz[e]indene-1-methyloxycarbonyl) phenylalanine

This product is prepared from the product of A hereinabove by following the procedure described in Ex. 20 I-L hereinabove

C. N-[Benz(e) indene-1-methoxyoxy carbonyl] phenylalanine acid fluoride.

The product B formed hereinabove (1 mmol) in dry $CH_2Cl_2$ is kept under nitrogen and treated with cyannuric fluoride (8 mmol) and pyridine (1 mmol) to form the above product.

EXAMPLE 69

N-[Benz(e) indene 1-methoxy carbonyl]phenylalanine acid fluoride and N[Benz(e) indene 3-methoxy carbonyl] phenylalanine acid fluoride

2($\beta$)-naphthylpropronic acid is treated with thionyl chloride and cyclized in the presence of aluminum chloride to form the corresponding ketone. Reduction of the ketone with sodium borohydride, followed by dehydration with sulfuric acid gives the benz[e] indene. Formylation with ethyl formate and sodium hydride followed by treatment with sodium borohydride gave a mixture of the Benz[e] indene-3-methanol and Benz-[e]-indene-1-methanol. The two alcohols are separated by chromatography.

Following the procedures in Examples 27 B-C and 23 B-C, the above-identified products are prepared.

It is to be noted from the preparations hereinabove that Benz[e]indene 1-methoxycarbonyl and the benz[e] indene-3-methoxy carbonyl can be used to protect the N $\alpha$-amino group of an amino acid.

These groups are formed from the corresponding alcohol, as described in Ex. 21-23. The alcohols are then used as starting materials to form the corresponding chloroformates and azidoformates, as described in Ex. 20. The azidoformate can then be reacted with an amino acid in accordance with the procedure described in Ex. 20-L to form the corresponding N $\alpha$-protected amino acid. This then is treated with cyannuric fluoride to form the corresponding amino acid fluoride.

The above preferred embodiments and examples are given to illustrate the scope and spirit of the present invention. These embodiments and examples will make apparent, to those skilled in the art, other embodiments and examples. These other embodiments and examples are within the contemplation of the present invention. Therefore, the present invention should be limited only by the appended claims.

**Claims**

**Claims for the following Contracting States : AT, BE, DE, DK, FR, GB, IT, NL, SE**

**1.** A compound of the formula:

$$\mathrm{BLK-}\overset{\overset{\displaystyle X}{|}}{\mathrm{AA}}\mathrm{-F}$$

or the acid fluoride salts thereof wherein

BLK is an N-amino protecting group;

AA is an alpha amino residue;

X is - or a side chain protecting group.

**2.** The D or L stereoisomer of the compound of Claim 1 or mixtures thereof.

**3.** The compound according to any of Claims 1-2, wherein BLK is a base labile N-$\alpha$-amino acid protecting group, an acid labile N-$\alpha$-amino acid protecting group, a nucleophile labile N-$\alpha$-amino acid protecting

group or a N-α-amino acid protecting group removable by hydrogenation.

**4.** The compound according to any of the Claims 1-3 wherein BLK has the formula:

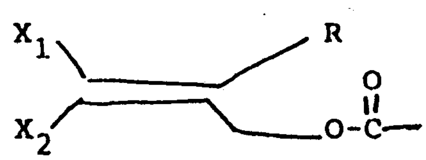

wherein R is an electron withdrawing group,

$X_1$ and $X_2$ are independently H, lower alkyl, aryl, aryl lower alkyl, or a solid support or R and $X_1$ taken together with the carbon which they are attached form a ring containing from 4-15 ring carbon atoms and may contain up to 2 heteroatoms, wherein the heteroatoms are O, S, or N.

**5.** The compound according to Claim 4 wherein R is $S0_2R_2$, $SOR_2$, $COOR_2$, $COR_2$, CHO, $CONR_2\ R_3$, CN, $CF_3$, $NO_2$, aryl, 2-pyridyl or 4-pyridyl, wherein $R_2$ and $R_3$ are independently lower alkyl, aryl, aryl lower alkyl, or polystyrene and the alkyl or aryl groups are unsubstituted or mono or disubstituted with halide $SO_2R_2$, $SOR_2$, $COOR_2$, $COR_2$, CHO, CN, $CF_3$, or $NO_2$.

**6.** The compound according to any of Claims 4-5 wherein R is $SO_2R_2$, $SOR_2$, $COOR_2$, $COR_2$, $CONR_2R_3$, aryl, 2-pyridyl, or 4-pyridyl.

**7.** The compound according to any of Claims 4-6 wherein R is $SO_2\ (CH_3)_3$, $SOC(CH_3)_3$, $SOCC_6H_5$, $SO_2\ CH_3$, COO Et, 2-pyridyl or 4-pyridyl or R and $X_1$ when taken together with the carbon to which they are attached are $SO_2C_6H_4$ or $C_5H_3NCH_2$.

**8.** The compound according to any of Claims 1-7 wherein AA is glycine, alanine, leucine, isoleucine, proline, hydroxyproline, phenylalanine, tyrosine, methionine, norleucine, serine, threonine, cystine, cysteine, tyrosine, aspartic acid, glutamic acid, asparagine, glutamine, lysine, arginine, hyroxylysine, ornithine, histidine or tryptophan and X is -.

**9.** The compound according to any of Claims 1-8 wherein

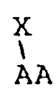

is

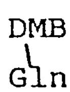, 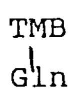, 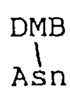, 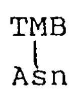,

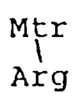, 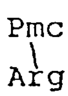, 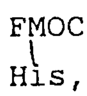,

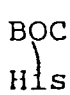, 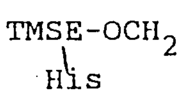 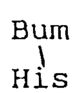, 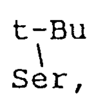, 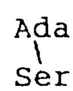, 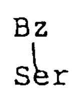, 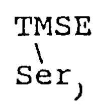)

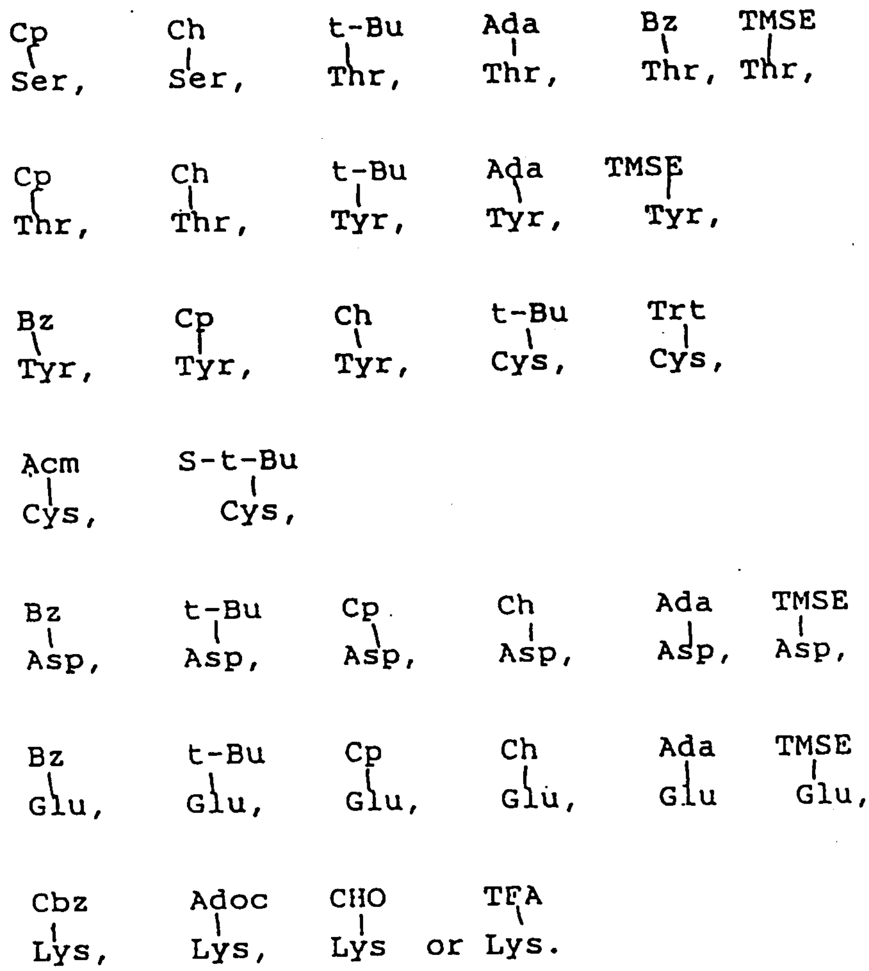

or

10. The compound according to any of Claims 1-9 wherein the side chain of the AA is hydroxy lower alkyl, carboxy lower alkyl, mercapto lower alkyl, hydroxy aryl or hydroxy benzyl and X is t-butyl.

11. The compound according to any one of Claims 1-3 and 8-10 wherein BLK is FMOC, BOC, CLIMOC, BIMOC, Dbd-TMOC, Aoc, Adoc $ArSO_2$, Bpoc, Ddz, HCO, TFA, Teoc, benz[e]indene-1-methoxycarbonyl, Benz-(e)-indene-3-methoxycarbonyl, Bspoc, Bsmoc Mspoc or CBZ.

12. The compound according to Claims 1-11 which is

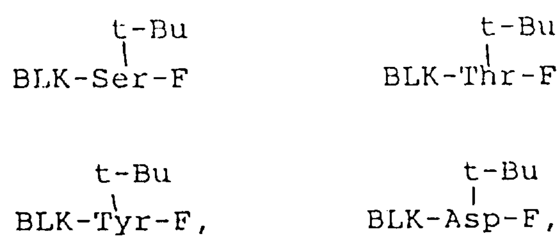

BLK-Trp-F,

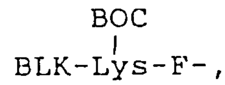

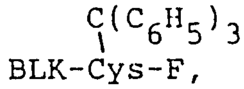

BLK-Gly-F, BLK-Ala-F, BLK-Val-F, BLK-Leu-F, BLK-Ile-F or BLK-Phe-F wherein

BLK is Bspoc, FMOC, Bsmoc, Mspoc or Cbz.

**13.** A process for forming a peptide bond between a first amino acid having a free amino group and a second amino acid or between a peptide having a free amino group and a second amino acid which comprises reacting the first amino acid or peptide with an amino acid fluoride of the formula

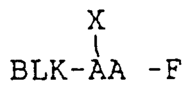

or the acid fluoride salts thereof wherein,

BLK is an N-amino protecting group;

AA is an alpha amino residue and X is - or a side chain protecting group.

**14.** The process according to Claim 13 wherein BLK is a base labile N-$\alpha$ amino acid protecting group, an acid labile N-$\alpha$ amino acid protecting group, a nucleophile labile N-$\alpha$ amino acid protecting group or N-$\alpha$ amino acid protecting group removeable by hydrogenation.

**15.** The process according to any one of Claims 13-14 wherein BLK has the formula:

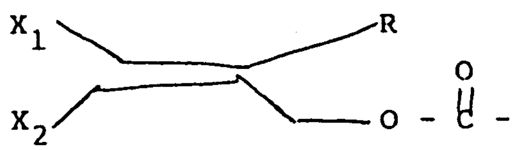

wherein R is an electron with drawing group,

$X_1$ and $X_2$ are independently H, lower alkyl, aryl, aryl lower alkyl or a solid support or R and $X_1$ taken together if the carbon to which they are attached form a ring containing from 4-15 carbon atoms and may contain up to 2 heteroatoms, wherein the heteroatoms are O, S, or N.

**16.** The process according to Claim 15 wherein R is $S0_2R_2$ $SOR_2$, $COOR_2$, $COR_2$, CHO, $CONR_2$ $R_3$, CN, $CF_3$, $NO_2$, aryl, 2-pyridyl or 4-pyridyl, wherein $R_2$ and $R_3$ are independently lower alkyl, aryl, aryl lower alkyl, or polystyrene and the alkyl or aryl groups are unsubstituted or mono or disubstituted with halide $SO_2R_2$, $SOR_2$, $COOR_2$, $COR_2$, CHO, CN, $CF_3$, or $NO_2$.

**17.** The process according to any one of Claims 15 and 16 wherein R is $SO_2R_2$, $SOR_2$, $COOR_2$, $COR_2$, $CONR_2R_3$, aryl, 2-pyridyl, or 4-pyridyl.

**18.** The process according to any one of Claims 15-17 wherein R is $SO_2$ $(CH_3)_3$, $SOC(CH_3)_3$, $SOCC_6H_5$, $SO_2$ $CH_3$, COO Et, 2-pyridyl, or 4-pyridyl or R and $X_1$ when taken together with the carbon to which they are attached are $SO_2C_6H_4$ or $C_5H_3NCH_2$.

**19.** The process according to any one of Claims 13-18 wherein AA is glycine, alanine, leucine, isoleucine, proline, hydroxy proline, phenylalanine, tyrosine, methionine, norleucine, serine, threonine, cysteine, cystine, tyrosine, aspartic acid, glutamic acid, asparagine, glutamine, lysine, arginine, hydroxylysine,

ornithine, histidine or tryptophan.

**20.** The process according to any one of Claims 13-19 wherein

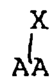

is

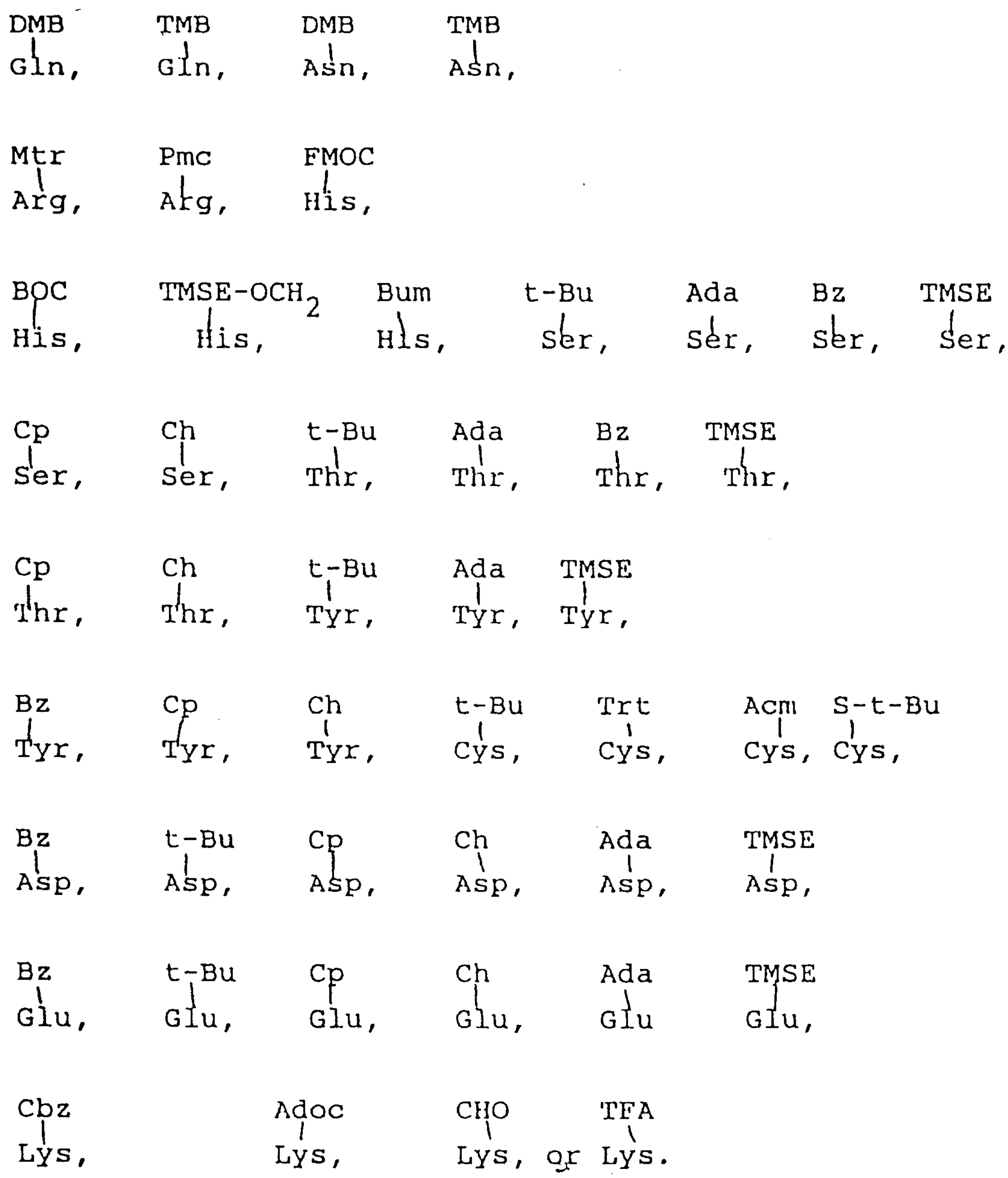

**21.** The process according to Claims 13-20 wherein the side chain of AA is hydroxy lower alkyl, carboxy lower alkyl mercapto lower alkyl, hydroxyaryl or hydroxy carbonyl and X t-butyl.

**22.** The process according to Claims 13-21 wherein the D or L stereoisomer or mixtures of the amino acid fluoride is used.

23. The process according to any of Claims 13-22 wherein BLK is FMOC, BOC, CLIMOC, BIMOC, Dbd-TMOC, Aoc, Adoc, $ArSO_2$, Bpoc, Ddz, HCO, TFA, Teoc, benz(e)indene-1-methoxycarbonyl or benz(e)-indene-3-methoxycarbonyl, Mspoc, Bspoc, Bsmoc or CBZ.

**Claims for the following Contracting State : ES**

1. A process for forming a peptide bond between a first amino acid having a free amino group and a second amino acid or between a peptide having a free amino group and a second amino acid which comprises reacting the first amino acid or peptide with an amino acid fluoride of the formula

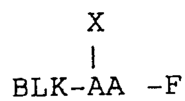

or the acid fluoride salts thereof, wherein,
BLK is an N-amino protecting group;
AA is an alpha amino residue and X is - or a side chain protecting group.

2. The process according to claim 1 wherein BLK is a base labile N-$\alpha$ amino acid protecting group, an acid labile N-$\alpha$ amino acid protecting group, a nucleophile labile N-$\alpha$ amino acid protecting group or N-$\alpha$ amino acid protecting group removeable by hydrogenation.

3. The process according to any one of claims 1-2 wherein BLK has the formula:

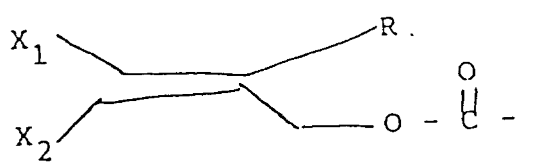

wherein R is an electron with drawing group,
$X_1$ and $X_2$ are independently H, lower alkyl, aryl, aryl lower alkyl or a solid support or R and $X_1$ taken together with the carbon to which they are attached form a ring containing from 4-15 carbon atoms and may contain up to 2 heteroatoms, wherein the heteroatoms are O,S, or N.

4. The process according to claim 3 wherein R is $SO_2R_2$, $SOR_2$, $COOR_2$, $COR_2$, CHO, $CONR_2R_3$, CN, $CF_3$, $NO_2$, aryl, 2-pyridyl or 4-pyridyl, wherein $R_2$ and $R_3$ are independently lower alkyl, aryl, aryl lower alkyl, or polystyrene and the alkyl or aryl groups are unsubstituted or mono or disubstituted with halide $SO_2R_2$, $SOR_2$, $COOR_2$, $COR_2$, CHO, CN, $CF_3$, or $NO_2$.

5. The process according to any one of claims 3 and 4 wherein R is $SO_2R_2$, $SOR_2$, $COOR_2$, $COR_2$, $CONR_2R_3$, aryl, 2-pyridyl, or 4-pyridyl.

6. The process according to any one of claims 3-5 wherein R is $SO_2(CH_3)_3$, $SOC(CH_3)_3$, $SOCC_6H_5$, $SO_2CH_3$, COO Et, 2-pyridyl, or 4-pyridyl or R and $X_1$ when taken together with the carbon to which they are attached are $SO_2C_6H_4$ or $C_5H_3NCH_2$.

7. The process according to any one of claims 1-6 wherein AA is glycine, alanine, leucine, isoleucine, proline, hydroxyproline, phenylalanine, tyrosine, methionine, norleucine, serine, threonine, cysteine, cystine, tyrosine, aspartic acid, glutamic acid, asparagine, glutamine, lysine, arginine, hydroxylysine, ornithine, histidine or tryptophan.

8. The process according to any one of claims 1-7 wherein

| |
|---|
| X |
| \| |
| AA |

is

| | | | |
|---|---|---|---|
| DMB | TMB | DMB | TMB |
| \| | \| | \| | \| |
| Gln, | Gln, | Asn, | Asn, |

| | | |
|---|---|---|
| Mtr | Pmc | FMOC |
| \| | \| | \| |
| Arg, | Arg, | His, |

| | | | | | | |
|---|---|---|---|---|---|---|
| BOC | $TMSE-OCH_2$ | Bum | t-Bu | Ada | Bz | TMSE |
| \| | \| | \| | \| | \| | \| | \| |
| His, | His, | His, | Ser, | Ser, | Ser, | Ser, |

| | | | | | |
|---|---|---|---|---|---|
| Cp | Ch | t-Bu | Ada | Bz | TMSE |
| \| | \| | \| | \| | \| | \| |
| Ser, | Ser, | Thr, | Thr, | Thr, | Thr, |

| | | | | |
|---|---|---|---|---|
| Cp | Ch | t-Bu | Ada | TMSE |
| \| | \| | \| | \| | \| |
| Thr, | Thr, | Tyr, | Tyr, | Tyr, |

| | | | | | | |
|---|---|---|---|---|---|---|
| Bz | Cp | Ch | t-Bu | Trt | Acm | S-t-Bu |
| \| | \| | \| | \| | \| | \| | \| |
| Tyr, | Tyr, | Tyr, | Cys, | Cys, | Cys, | Cys, |

| | | | | | |
|---|---|---|---|---|---|
| Bz | t-Bu | Cp | Ch | Ada | TMSE |
| \| | \| | \| | \| | \| | \| |
| Asp, | Asp, | Asp, | Asp, | Asp, | Asp, |

| | | | | | |
|---|---|---|---|---|---|
| Bz | t-Bu | Cp | Ch | Ada | TMSE |
| \| | \| | \| | \| | \| | \| |
| Glu, | Glu, | Glu, | Glu, | Glu | Glu, |

| | | | |
|---|---|---|---|
| Cbz | Adoc | CHO | TFA |
| \| | \| | \| | \| |
| Lys, | Lys, | Lys, or | Lys. |

9. The process according to claims 1-8 wherein the side chain of AA is hydroxy lower alkyl, carboxy lower alkyl, mercapto lower alkyl, hydroxyaryl or hydroxy carbonyl and X is t-butyl.

10. The process according to claims 1-9 wherein the D or L stereoisomer or mixtures of the amino acid fluoride is used.

11. The process according to any of claims 1-10 wherein BLK is FMOC, BOC, CLIMOC, BIMOC, Dbd-TMOC, Aoc, Adoc, $ArSO_2$, Bpoc, Ddz, HCO, TFA, Teoc, benz(e)indene-1-methoxycarbonyl or benz(e)-indene-3-methoxy-carbonyl, Mspoc, Bspoc, Bsmoc or CBZ.

**Patentansprüche**
**Patentansprüche für folgende Vertragsstaaten : AT, BE, DE, DK, FR, GB, IT, NL, SE**

1. Verbindung der Formel

X
|
BLK-AA-F

oder die Säurefluoridsalze davon, worin
BLK eine N-Aminoschutzgruppe bedeutet;
AA einen α-Aminosäurerest bedeutet;
X die Bedeutung - hat oder eine Seitenketten-Schutzgruppe bedeutet.

2. D- oder L-Stereoisomeres der Verbindung von Anspruch 1 oder Gemische davon.

3. Verbindung nach einem der Ansprüche 1-2, worin BLK eine basenlabile N-α-Aminosäure-Schutzgruppe, eine säurelabile N-α-Aminosäure-Schutzgruppe, eine gegenüber einem nukleophilen Agens labile N-α-Aminosäure-Schutzgruppe oder eine durch Hydrierung entfernbare N-α-Aminosäure-Schutzgruppebedeutet.

4. Verbindung nach einem der Ansprüche 1 bis 3, worin BLK die folgende Formel aufweist:

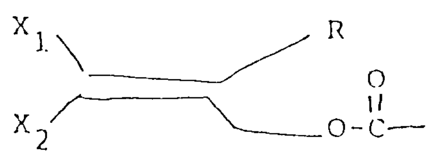

worin R eine elektronenziehende Gruppe bedeutet;
$X_1$ und $X_2$ unabhängig voneinander H, Niederalkyl, Aryl, Aryl-niederalkyl oder einen festen Träger bedeuten oder R und $X_1$ zusammen mit dem Kohlenstoffatom, an das sie gebunden sind, einen Ring mit 4-15 Ringkohlenstoffatomen, der bis zu zwei Heteroatome enthalten kann, bilden, wobei es sich bei den Heteroatomen um O, S oder N handelt.

5. Verbindung nach Anspruch 4, worin R die Bedeutung $SO_2R$, $SOR_2$, $COOR_2$, $COR_2$, CHO, $CONR_2$, $R_3$, CN, $CF_3$, $NO_2$, Aryl, 2-Pyridyl oder 4-Pyridyl hat, worin $R_2$ und $R_3$ unabhängig voneinander Niederalkyl, Aryl, Aryl-niederalkyl oder Polystyrol bedeuten und die Alkyl- oder Arylgruppen unsubstituiert oder einfach oder zweifach durch Halogenid, $SO_2R_2$, $SOR_2$, $COOR_2$, $COR_2$, CHO, CN, $CF_3$ oder $NO_2$ substituiert sind.

6. Verbindung nach einem der Ansprüche 4-5, worin R die Bedeutung $SO_2R_2$, $SOR_2$, $COOR_2$, $COR_2$, $CONR_2R_3$, Aryl, 2-Pyridyl oder 4-Pyridyl hat.

7. Verbindung nach einem der Ansprüche 4-6, worin R die Bedeutung $SO_2(CH_3)_3$, $SOC(CH_3)_3$, $SOCC_6H_5$, $SO_2CH_3$, COOEt, 2-Pyridyl oder 4-Pyridyl hat und R und $X_1$ zusammen mit dem Kohlenstoffatom, an das sie gebunden sind, die Bedeutung $SO_2C_6H_4$ oder $C_5H_3NCH_2$ haben.

8. Verbindung nach einem der Ansprüche 1-7, worin AA die Bedeutung Glycin, Alanin, Leucin, Isoleucin, Prolin, Hydroxyprolin, Phenylalanin, Tyrosin, Methionin, Norleucin, Serin, Threonin, Cystin, Cystein, Tyrosin, Asparaginsäure, Glutaminsäure, Asparagin, Glutamin, Lysin, Arginin, Hydroxylysin, Ornithin,

Histidin oder Tryptophan hat und X die Bedeutung - hat.

**9.** Verbindung nach einem der Ansprüche 1 bis 8, worin

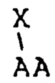

die Bedeutung

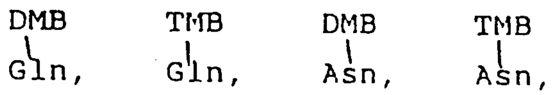

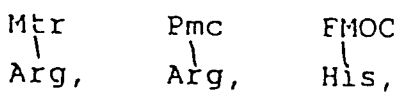

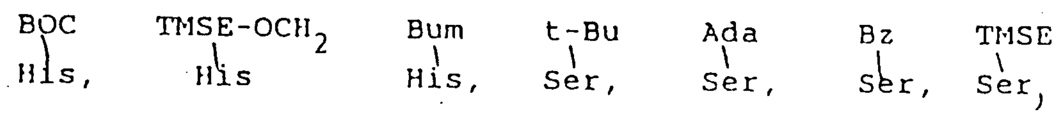

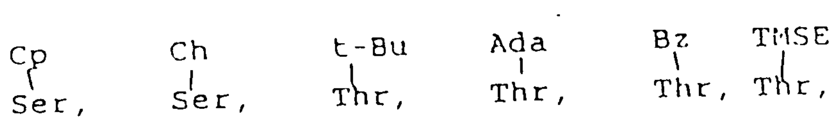

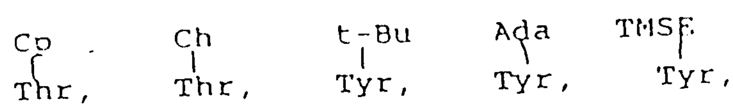

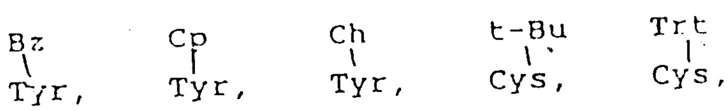

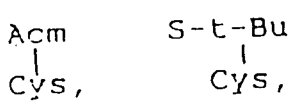

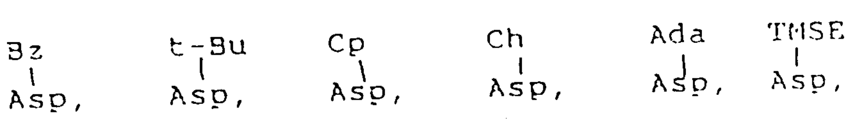

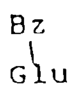, 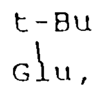, 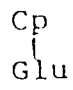, 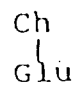, 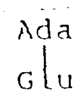 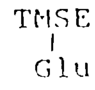,

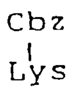, 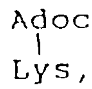, 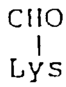 oder 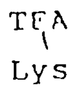

hat.

**10.** Verbindung nach einem der Ansprüche 1-9, worin es sich bei der Seitenkette von AA um Hydroxy-niederalkyl, Carboxy-niederalkyl, Mercaptoniederalkyl, Hydroxyaryl oder Hydroxybenzyl und bei X um tert.-Butyl handelt.

**11.** Verbindung nach einem der Ansprüche 1-3 und 8-10, worin es sich bei BLK um FMOC, BOC, CLIMOC, BIMOC, Dbd-TMOC, Aoc, Adoc, $ArSO_2$, Bpoc, Ddz, HCO, TFA, Teoc, Benz[e]inden-1-methoxycarbonyl, Benz[e]inden-3-methoxycarbonyl, Bspoc, Bsmoc, Mspoc oder CBZ handelt.

**12.** Verbindung nach den Ansprüchen 1-11, wobei es sich um

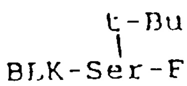 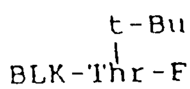,

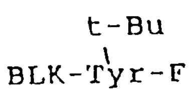, 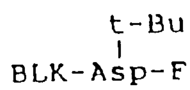,

BLK-Trp-F,

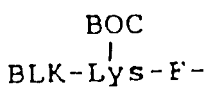,

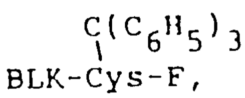,

BLK-Gly-F, BLK-Ala-F, BLK-Val-F, BLK-Leu-F, BLK-Ile-F oder BLK-Phe-F
handelt, worin BLK die Bedeutung Bspoc, FMOC, Bsmoc, Mspoc oder Cbz hat.

**13.** Verfahren zur Bildung einer Peptidbindung zwischen einer ersten Aminosäure mit einer freien Aminogruppe und einer zweiten Aminosäure oder zwischen einem Peptid mit einer freien Aminogruppe und einer zweiten Aminosäure, das die Umsetzung der ersten Aminosäure oder Peptids mit einem Aminosäurefluorid der Formel

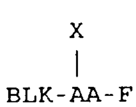

oder den Säurefluoridsalzen davon umfaßt, worin

BLK eine N-Aminoschutzgruppe bedeutet,
AA einen α-Aminosäurerest bedeutet; und
X die Bedeutung - hat oder eine Seitenketten-Schutzgruppe bedeutet.

**14.** Verfahren nach Anspruch 13, wobei BLK eine basenlabile N-α-Aminosäure-Schutzgruppe, eine säurelabile N-α-Aminosäure-Schutzgruppe, eine gegenüber einem nukleophilen Agens labile N-α-Aminosäure-Schutzgruppe oder eine durch Hydrierung entfernbare N-α-Aminosäure-Schutzgruppebedeutet.

**15.** Verfahren nach einem der Ansprüche 13-14, wobei BLK die folgende Formel aufweist:

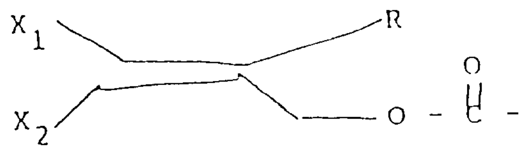

worin R eine elektronenziehende Gruppe bedeutet;
$X_1$ und $X_2$ unabhängig voneinander H, Niederalkyl, Aryl, Aryl-niederalkyl oder einen festen Träger bedeuten oder R und $X_1$ zusammen mit dem Kohlenstoffatom, an das sie gebunden sind, einen Ring mit 4-15 Ringkohlenstoffatomen, der bis zu zwei Heteroatome enthalten kann, bilden, wobei es sich bei den Heteroatomen um O, S oder N handelt.

**16.** Verfahren nach Anspruch 15, wobei R die Bedeutung $SO_2R$, $SOR_2$, $COOR_2$, $COR_2$, CHO, $CONR_2$, $R_3$, CN, $CF_3$, $NO_2$, Aryl, 2-Pyridyl oder 4-Pyridyl hat, worin $R_2$ und $R_3$ unabhängig voneinander Niederalkyl, Aryl, Aryl-niederalkyl oder Polystyrol bedeuten und die Alkyl- oder Arylgruppen unsubstituiert oder einfach oder zweifach durch Halogenid, $SO_2R_2$, $SOR_2$, $COOR_2$, $COR_2$, CHO, CN, $CF_3$ oder $NO_2$ substituiert sind.

**17.** Verfahren nach einem der Ansprüche 15-16, worin R die Bedeutung $SO_2R_2$, $SOR_2$, $COOR_2$, $COR_2$, $CONR_2R_3$, Aryl, 2-Pyridyl oder 4-Pyridyl hat.

**18.** Verfahren nach einem der Ansprüche 15-17, worin R die Bedeutung $SO_2(CH_3)_3$, $SOC(CH_3)_3$, $SOCC_6H_5$, $SO_2CH_3$, COOEt, 2-Pyridyl oder 4-Pyridyl hat und R und $X_1$ zusammen mit dem Kohlenstoffatom, an das sie gebunden sind, die Bedeutung $SO_2C_6H_4$ oder $C_5H_3NCH_2$ haben.

**19.** Verfahren nach einem der Ansprüche 13-18, worin AA die Bedeutung Glycin, Alanin, Leucin, Isoleucin, Prolin, Hydroxyprolin, Phenylalanin, Tyrosin, Methionin, Norleucin, Serin, Threonin, Cystin, Cystein, Tyrosin, Asparaginsäure, Glutaminsäure, Asparagin, Glutamin, Lysin, Arginin, Hydroxylysin, Ornithin, Histidin oder Tryptophan hat und X die Bedeutung - hat.

**20.** Verfahren nach einem der Ansprüche 13-19, worin

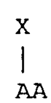

die Bedeutung

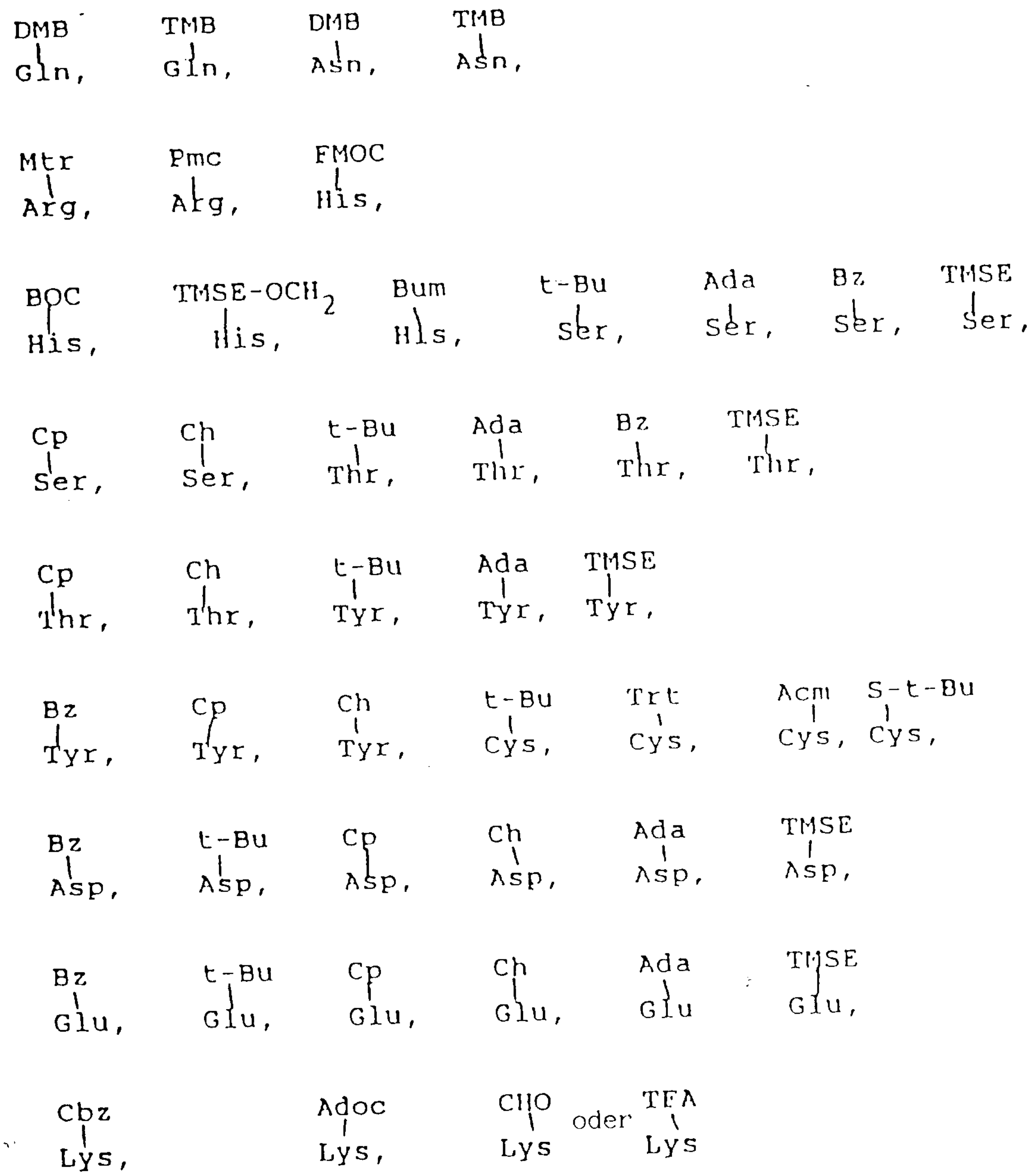

oder

hat.

**21.** Verfahren nach einem der Ansprüche 13-20, worin es sich bei der Seitenkette von AA um Hydroxy-niederalkyl, Carboxy-niederalkyl, Mercaptoniederalyyl, Hydroxyaryl oder Hydroxybenzyl und bei X um tert.-Butyl handelt.

**22.** Verfahren nach einem der Ansprüche 13-21, worin das D- oder L-Stereoisomere oder Gemische der Aminosäurefluoride verwendet werden.

**23.** Verfahren nach einem der Ansprüche 13-22, worin es sich bei BLK um FMOC, BOC, CLIMOC, BIMOC, Dbd-TMOC, Aoc, Adoc, $ArSO_2$, Bpoc, Ddz, HCO, TFA, Teoc, Benz[e]inden-1-methoxycarbonyl, Benz-[e]inden-3-methoxycarbonyl, Mspoc, Bspoc, Bsmoc oder CBZ handelt.

**Patentansprüche für folgenden Vertragsstaat : ES**

1. Verfahren zur Bildung einer Peptidbindung zwischen einer ersten Aminosäure mit einer freien Aminogruppe und einer zweiten Aminosäure oder zwischen einem Peptid mit einer freien Aminogruppe und einer zweiten Aminosäure, das die Umsetzung der ersten Aminosäure oder Peptids mit einem Aminosäurefluorid der Formel

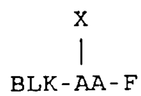

oder den Säurefluoridsalzen davon umfaßt, worin
BLK eine N-Aminoschutzgruppe bedeutet,
AA einen α-Aminosäurerest bedeutet; und
X die Bedeutung - hat oder eine Seitenketten-Schutzgruppe bedeutet.

2. Verfahren nach Anspruch 1, wobei BLK eine basenlabile N-α-Aminosäure-Schutzgruppe, eine säurelabile N-α-Aminosäure-Schutzgruppe, eine gegenüber einem nukleophilen Agens labile N-α-Aminosäure-Schutzgruppe oder eine durch Hydrierung entfernbare N-α-Aminosäure-Schutzgruppebedeutet.

3. Verfahren nach einem der Ansprüche 1-2, wobei BLK die folgende Formel aufweist:

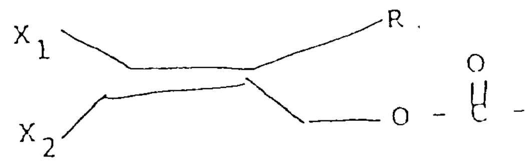

worin R eine elektronenziehende Gruppe bedeutet;
$X_1$ und $X_2$ unabhängig voneinander H, Niederalkyl, Aryl, Aryl-niederalkyl oder einen festen Träger bedeuten oder R und $X_1$ zusammen mit dem Kohlenstoffatom, an das sie gebunden sind, einen Ring mit 4-15 Ringkohlenstoffatomen, der bis zu zwei Heteroatome enthalten kann, bilden, wobei es sich bei den Heteroatomen um O, S oder N handelt.

4. Verfahren nach Anspruch 3, wobei R die Bedeutung $SO_2R$, $SOR_2$, $COOR_2$, $COR_2$, CHO, $CONR_2$, $R_3$, CN, $CF_3$, $NO_2$, Aryl, 2-Pyridyl oder 4-Pyridyl hat, worin $R_2$ und $R_3$ unabhängig voneinander Niederalkyl, Aryl, Aryl-niederalkyl oder Polystyrol bedeuten und die Alkyl- oder Arylgruppen unsubstituiert oder einfach oder zweifach durch Halogenid, $SO_2R_2$, $SOR_2$, $COOR_2$, $COR_2$, CHO, CN, $CF_3$ oder $NO_2$ substituiert sind.

5. Verfahren nach einem der Ansprüche 3 und 4, worin R die Bedeutung $SO_2R_2$, $SOR_2$, $COOR_2$, $COR_2$, $CONR_2R_3$, Aryl, 2-Pyridyl oder 4-Pyridyl hat.

6. Verfahren nach einem der Ansprüche 3-5, worin R die Bedeutung $SO_2(CH_3)_3$, $SOC(CH_3)_3$, $SOCC_6H_5$, $SO_2CH_3$, COOEt, 2-Pyridyl oder 4-Pyridyl hat und R und $X_1$ zusammen mit dem Kohlenstoffatom, an das sie gebunden sind, die Bedeutung $SO_2C_6H_4$ oder $C_5H_3NCH_2$ haben.

7. Verfahren nach einem der Ansprüche 1-6, worin AA die Bedeutung Glycin, Alanin, Leucin, Isoleucin, Prolin, Hydroxyprolin, Phenylalanin, Tyrosin, Methionin, Norleucin, Serin, Threonin, Cystin, Cystein, Tyrosin, Asparaginsäure, Glutaminsäure, Asparagin, Glutamin, Lysin, Arginin, Hydroxylysin, Ornithin, Histidin oder Tryptophan hat und X die Bedeutung - hat.

**8.** Verfahren nach einem der Ansprüche 1-7, worin

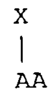

die Bedeutung

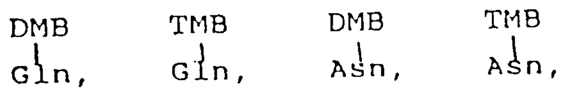

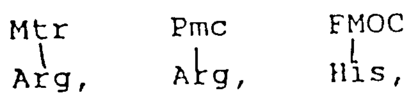

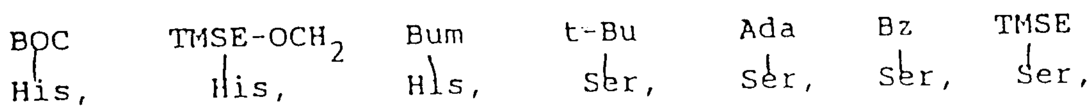

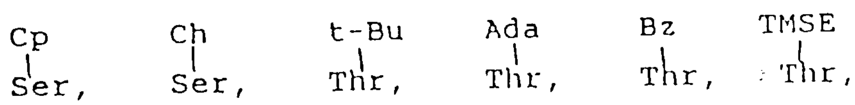

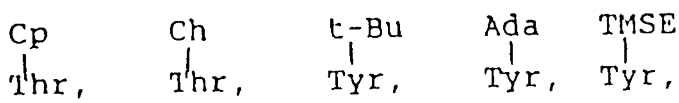

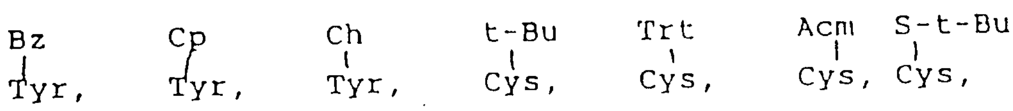

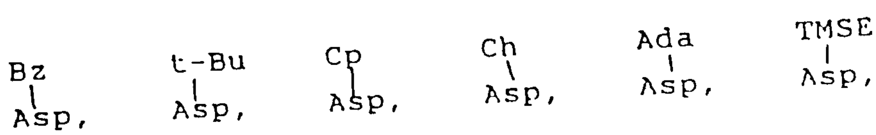

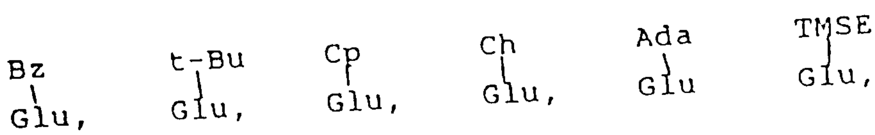

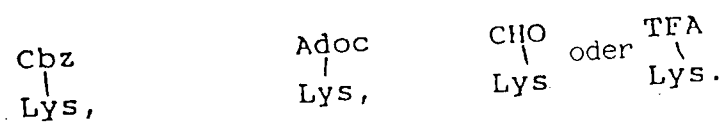

oder

hat.

**9.** Verfahren nach einem der Ansprüche 1-8, worin es sich bei der Seitenkette von AA um Hydroxy-niederalkyl, Carboxy-niederalkyl, Mercaptoniederalkyl, Hydroxyaryl oder Hydroxybenzyl und bei X um

tert.-Butyl handelt.

10. Verfahren nach einem der Ansprüche 1-9, worin das D- oder L-Stereoisomere oder Gemische der Aminosäurefluoride verwendet werden.

11. Verfahren nach einem der Ansprüche 1-10, worin es sich bei BLK um FMOC, BOC, CLIMOC, BIMOC, Dbd-TMOC, Aoc, Adoc, $ArSO_2$, Bpoc, Ddz, HCO, TFA, Teoc, Benz[e]inden-1-methoxycarbonyl, Benz-[e]inden-3-methoxycarbonyl, Mspoc, Bspoc, Bsmoc oder CBZ handelt.

**Revendications**
**Revendications pour les Etats contractants suivants : AT, BE, DE, DK, FR, GB, IT, NL, SE**

1. Composé de formule :

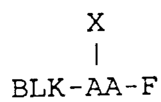

ou ses sels avec l'acide fluorhydrique, dans lequel
BLK est un groupe N-amino-protecteur ;
AA est un résidu alpha-amino ;
X est - ou un groupe protecteur de chaîne latérale.

2. Stéréoisomère D ou L du composé selon la revendication 1, ou leurs mélanges.

3. Composé selon l'une quelconque des revendications 1 ou 2, dans lequel BLK est un groupe protecteur d'acide N-$\alpha$-aminé labile basique, un groupe protecteur d'acide N-$\alpha$-aminé acide labile, un groupe protecteur d'acide N-$\alpha$-aminé nucléophile labile ou un groupe protecteur d'acide N-$\alpha$-amino éliminable par hydrogénation.

4. Composé selon l'une quelconque des revendications 1 à 3, dans lequel BLK a la formule suivante :

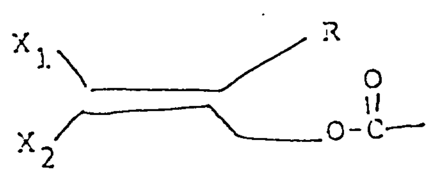

dans laquelle R est un groupe attracteur d'électrons, $X_1$ et $X_2$, indépendamment l'un de l'autre, sont chacun H ou un radical alkyle inférieur, aryle, aryl(alkyle inférieur) ou encore un support solide, ou bien R et $X_1$, pris ensemble avec l'atome de carbone auquel ils sont liés, forment un noyau contenant de 4 à 15 atomes de carbone nucléaire et pouvant contenir jusqu'à 2 hétéroatomes, les hétéroatomes étant O, S ou N.

5. Composé selon la revendication 4, dans lequel R est $SO_2R_2$ , $SOR_2$, $COOR_2$, $COR_2$, CHO, $CONR_2R_3$, CN, $CF_3$, $NO_2$, un radical aryle, 2-pyridyle ou 4-pyridyle, où $R_2$ et $R_3$, indépendamment l'un de l'autre, sont chacun un radical alkyle inférieur, aryle, aryl(alkyle inférieur), ou encore un polystyrène, et les groupes alkyle ou aryle sont non substitués ou encore sont mono- ou disuhstitués par des halogénures ou des substituants $SO_2R_2$, $SOR_2$, $COOR_2$, $COR_2$, CHO, CN, $CF_3$ ou $NO_2$.

6. Composé selon l'une quelconque des revendications 4 ou 5, dans lequel R est $SO_2R_2$, $SOR_2$, $COOR_2$, $COR_2$, $CONR_2R_3$, ou encore un radical aryle, 2-pyridyle ou 4-pyridyle.

7. Composé selon l'une quelconque des revendications 4 à 6, dans lequel R est $SO_2(CH_3)_3$, $SOC(CH_3)_3$, $SOCC_6H_5$, $SO_2CH_3$, COOEt, ou encore un radical 2-pyridyle ou 4-pyridyle, ou encore R et $X_1$, pris

ensemble avec l'atome de carbone auquel ils sont liés, représentent $SO_2C_6H_4$ ou $C_5H_3NCH_2$.

**8.** Composé selon l'une quelconque des revendications 1 à 7, dans lequel AA est la glycine, l'alanine, la leucine, l'isoleucine, la proline, l'hydroxyproline, la phénylalanine, la tyrosine, la méthionine, la norleucine, la sérine, la thréonine, la cystine, la cystéine, la tyrosine, l'acide aspartique, l'acide glutamique, l'asparagine, la glutamine, la lysine, l'arginine, l'hydroxylysine, l'ornithine, l'histidine ou le tryptophane, et X est -.

**9.** Composé selon l'une quelconque des revendications 1 à 8, dans lequel

X
|
AA

est

| | | | | | | |
|---|---|---|---|---|---|---|
| DMB | TMB | DMB | TMB | | | |
| \| | \| | \| | \| | | | |
| Gln, | Gln, | Asn, | Asn, | | | |
| Mtr | Pmc | FMOC | | | | |
| \| | \| | \| | | | | |
| Arg, | Arg, | His, | | | | |
| BOC | TMSE-$OCH_2$ | Bum | t-Bu | Ada | Bz | TMSE |
| \| | \| | \| | \| | \| | \| | \| |
| His, | His, | His, | Ser, | Ser, | Ser, | Ser, |
| Cp | Ch | t-Bu | Ada | Bz | TMSE | |
| \| | \| | \| | \| | \| | \| | |
| Ser, | Ser, | Thr, | Thr, | Thr, | Thr, | |
| Cp | Ch | t-Bu | Ada | TMSE | | |
| \| | \| | \| | \| | \| | | |
| Thr, | Thr, | Tyr, | Tyr, | Tyr, | | |
| Bz | Cp | Ch | t-Bu | Trt | | |
| \| | \| | \| | \| | \| | | |
| Tyr, | Tyr, | Tyr, | Cys, | Cys, | | |
| Acm | S-t-Bu | | | | | |
| \| | \| | | | | | |
| Cys, | Cys, | | | | | |
| Bz | t-Bu | Cp | Ch | Ada | TMSE | |
| \| | \| | \| | \| | \| | \| | |
| Asp, | Asp, | Asp, | Asp, | Asp, | Asp, | |
| Bz | t-Bu | Cp | Ch | Ada | TMSE | |
| \| | \| | \| | \| | \| | \| | |
| Glu, | Glu, | Glu, | Glu, | Glu, | Glu, | |
| Cbz | Adoc | CHO | TFA | | | |
| \| | \| | \| | \| | | | |
| Lys, | Lys, | Lys, ou | Lys. | | | |

**10.** Composé selon l'une quelconque des revendications 1 à 9, dans lequel la chaîne latérale de AA est un groupe hydroxy(alkyle inférieur), carboxy(alkyle inférieur), mercapto(alkyle inférieur), hydroxyaryle ou hydroxybenzyle, et X est le radical tert-butyle.

**11.** Composé selon l'une quelconque des revendications 1 à 3 et 8 à 9, dans lequel BLK est FMOC, BOC, CLIMOC, BIMOC, Dbd-TMOC, Aoc, Adoc, $ArSO_2$, Bpoc, Ddz, HCO, TFA, Teoc, le radical benzo[e]-indène-1-méthoxycarbonyle, benzo[e]indène-3-méthoxycarbonyle, Bspoc, Bsmoc, Mspoc ou Cbz.

**12.** Composé selon les revendications 1 à 11, qui est l'un des composés suivants :

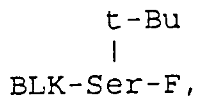

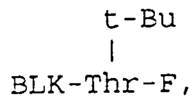

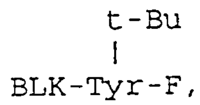

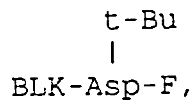

BLK-Trp-F,

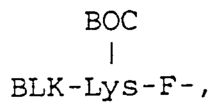

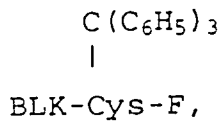

BLK-Gly-F, BLK-Ala-F, BLK-Val-F, BLK-Leu-F, BLK-Ile-F ou BLK-Phe-F, où BLK est Bspoc, FMOC, Msmoc, Mspoc ou Cbz.

**13.** Procédé pour former une liaison peptidique entre un premier acide aminé ayant un groupe amino libre et un deuxième acide aminé ou entre un peptide ayant un groupe amino libre et un deuxième acide aminé, qui consiste à faire réagir le premier acide aminé ou le peptide avec un fluorure d'acide aminé de formule :

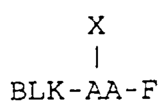

ou ses sels de l'acide fluorhydrique, dans lequel
BLK est un groupe N-amino-protecteur ;
AA est un résidu alpha-amino et X est - ou encore un groupe protecteur en chaîne latérale.

**14.** Procédé selon la revendication 13, dans lequel BLK est un groupe protecteur d'acide N-$\alpha$-aminé labile basique, un groupe protecteur d'acide N-$\alpha$-aminé acide labile, un groupe protecteur d' acide N-$\alpha$-aminé nucléophile labile ou un groupe protecteur d'acide N-$\alpha$-amino éliminable par hydrogénation.

**15.** Procédé selon l'une quelconque des revendications 13 ou 14, dans lequel BLK a la formule suivante :

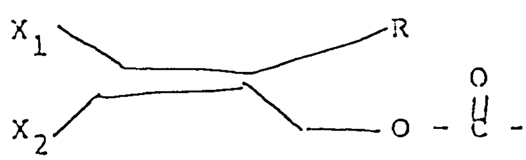

dans laquelle R est un groupe attracteur d'électrons, $X_1$ et $X_2$, indépendamment l'un de l'autre, sont chacun H ou un radical alkyle inférieur, aryle, aryl(alkyle inférieur) ou encore un support solide, ou bien R et $X_1$, pris ensemble avec l'atome de carbone auquel ils sont liés, forment un noyau contenant de 4

à 15 atomes de carbone nucléaire et pouvant contenir jusqu'à 2 hétéroatomes, les hétéroatomes étant O, S ou N.

**16.** Procédé selon la revendication 15, dans lequel R est $SO_2R_2$, $SOR_2$, $COOR_2$, $COR_2$, CHO, $CONR_2R_3$, CN, $CF_3$, $NO_2$ ou un radical aryle, 2-pyridyle ou 4-pyridyle, où $R_2$ et $R_3$, indépendamment l'un de l'autre, sont chacun un radical alkyle inférieur, aryle, aryl(alkyle inférieur), ou encore un polystyrène, et les groupes alkyle ou aryle sont non substitués ou encore sont mono- ou disubstitués par des halogénures ou des substituants $SO_2R_2$, $SOR_2$, $COOR_2$, $COR_2$, CHO, CN, $CF_3$ ou $NO_2$.

**17.** Procédé selon l'une quelconque des revendications 15 et 16, dans lequel R est $SO_2R_2$, $SOR_2$, $COOR_2$, $COR_2$, $CONR_2R_3$, ou encore un radical aryle, 2-pyridyle ou 4-pyridyle.

**18.** Procédé selon l'une quelconque des revendications 15 à 17, dans lequel R est $SO_2(CH_3)_3$, $SOC(CH_3)_3$, $SOCC_6H_5$, $SO_2CH_3$, COOEt, ou encore un radical 2-pyridyle ou 4-pyridyle, ou encore R et $X_1$, pris ensemble avec l'atome de carbone auquel ils sont liés, représentent $SO_2C_6H_4$ ou $C_5H_3NCH_2$.

**19.** Procédé selon l'une quelconque des revendications 13 à 18, dans lequel AA est la glycine, l'alanine, la leucine, l'isoleucine, la proline, l'hydroxyproline, la phénylalanine, la tyrosine, la méthionine, la norleucine, la sérine, la thréonine, la cystine, la cystéine, la tyrosine, l'acide aspartique, l'acide glutamique, l'asparagine, la glutamine, la lysine, l'arginine, l'hydroxylysine, l'ornithine, l'histidine ou le tryptophane.

**20.** Procédé selon l'une quelconque des revendications 13 à 19, dans lequel

X
|
AA

est

| | | | |
|---|---|---|---|
| DMB<br>\|<br>Gln, | TMB<br>\|<br>Gln, | DMB<br>\|<br>Asn, | TMB<br>\|<br>Asn, |
| Mtr<br>\|<br>Arg, | Pmc<br>\|<br>Arg, | FMOC<br>\|<br>His, | |

| | | | | | | |
|---|---|---|---|---|---|---|
| BOC | $TMSE-OCH_2$ | Bum | t-Bu | Ada | Bz | TMSE |
| \| | \| | \| | \| | \| | \| | \| |
| His, | His, | His, | Ser, | Ser, | Ser, | Ser, |

| | | | | | |
|---|---|---|---|---|---|
| Cp | Ch | t-Bu | Ada | Bz | TMSE |
| \| | \| | \| | \| | \| | \| |
| Ser, | Ser, | Thr, | Thr, | Thr, | Thr, |

| | | | | |
|---|---|---|---|---|
| Cp | Ch | t-Bu | Ada | TMSE |
| \| | \| | \| | \| | \| |
| Thr, | Thr, | Tyr, | Tyr, | Tyr, |

| | | | | |
|---|---|---|---|---|
| Bz | Cp | Ch | t-Bu | Trt |
| \| | \| | \| | \| | \| |
| Tyr, | Tyr, | Tyr, | Cys, | Cys, |

| | |
|---|---|
| Acm | S-t-Bu |
| \| | \| |
| Cys, | Cys, |

| | | | | | |
|---|---|---|---|---|---|
| Bz | t-Bu | Cp | Ch | Ada | TMSE |
| \| | \| | \| | \| | \| | \| |
| Asp, | Asp, | Asp, | Asp, | Asp, | Asp, |

| | | | | | |
|---|---|---|---|---|---|
| Bz | t-Bu | Cp | Ch | Ada | TMSE |
| \| | \| | \| | \| | \| | \| |
| Glu, | Glu, | Glu, | Glu, | Glu, | Glu, |

| | | | |
|---|---|---|---|
| Cbz | Adoc | CHO | TFA |
| \| | \| | \| | \| |
| Lys, | Lys, | Lys, ou | Lys. |

**21.** Procédé selon l'une quelconque des revendications 13 à 20, dans lequel la chaîne latérale de AA est un groupe hydroxy(alkyle inférieur), carboxy(alkyle inférieur), mercapto(alkyle inférieur), hydroxyaryle ou hydroxycarbonyle, et X est le radical tert-butyle.

**22.** Procédé selon les revendications 13 à 21, dans lequel on utilise le stéréoisomère D ou L ou des mélanges de fluorure d'acide aminé.

**23.** Procédé selon l'une quelconque des revendications 13 à 22, dans lequel BLK est FMOC, BOC, CLIMOC, BIMOC, Dbd-TMOC, Aoc, Adoc, $ArSO_2$, Bpoc, Ddz, HCO, TFA, Teoc, le radical benzo[e]-indène-1-méthoxycarbonyle, benzo[e]indène-3-méthoxycarbonyle, Mspoc, Bspoc, Bsmoc ou Cbz.

**Revendications pour l'Etat contractant suivant : ES**

**1.** Procédé pour former une liaison peptidique entre un premier acide aminé ayant un groupe amino libre et un deuxième acide aminé ou entre un peptide ayant un groupe amino libre et un deuxième acide aminé, qui consiste à faire réagir le premier acide aminé ou le peptide avec un fluorure d'acide aminé de formule :

X
|
BLK-AA-F

ou ses sels de l'acide fluorhydrique, dans lequel
BLK est un groupe N-amino-protecteur ;
AA est un résidu alpha-amino et X est - ou encore un groupe protecteur en chaîne latérale.

**2.** Procédé selon la revendication 1, dans lequel BLK est un groupe protecteur d'acide N-α-aminé labile basique, un groupe protecteur d'acide N-α-aminé acide labile, un groupe protecteur d' acide N-α-aminé nucléophile labile ou un groupe protecteur d'acide N-α-amino éliminable par hydrogénation.

**3.** Procédé selon l'une quelconque des revendications 1 ou 2, dans lequel BLK a la formule suivante :

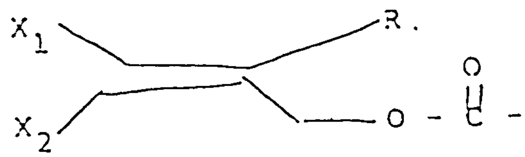

dans laquelle R est un groupe attracteur d'électrons, $X_1$ et $X_2$, indépendamment l'un de l'autre, sont chacun H ou un radical alkyle inférieur, aryle, aryl(alkyle inférieur) ou encore un support solide, ou bien R et $X_1$, pris ensemble avec l'atome de carbone auquel ils sont liés, forment un noyau contenant de 4 à 15 atomes de carbone nucléaire et pouvant contenir jusqu'à 2 hétéroatomes, les hétéroatomes étant O, S ou N.

**4.** Procédé selon la revendication 3, dans lequel R est $SO_2R_2$, $SOR_2$, $COOR_2$, $COR_2$, CHO, $CONR_2R_3$, CN, $CF_3$, $NO_2$ ou un radical aryle, 2-pyridyle ou 4-pyridyle, où $R_2$ et $R_3$, indépendamment l'un de l'autre, sont chacun un radical alkyle inférieur, aryle, aryl(alkyle inférieur), ou encore un polystyrène, et les groupes alkyle ou aryle sont non substitués ou encore sont mono- ou disubstitués par des halogénures ou des substituants $SO_2R_2$, $SOR_2$, $COOR_2$, $COR_2$, CHO, CN, $CF_3$ ou $NO_2$.

**5.** Procédé selon l'une quelconque des revendications 3 et 4, dans lequel R est $SO_2R_2$, $SOR_2$, $COOR_2$, $COR_2$, $CONR_2R_3$, ou encore un radical aryle, 2-pyridyle ou 4-pyridyle.

**6.** Procédé selon l'une quelconque des revendications 3 à 5, dans lequel R est $SO_2(CH_3)_3$, $SOC(CH_3)_3$, $SOCC_6H_5$, $SO_2CH_3$, COOEt, ou encore un radical 2-pyridyle ou 4-pyridyle, ou encore R et $X_1$, pris ensemble avec l'atome de carbone auquel ils sont liés, représentent $SO_2C_6H_4$ ou $C_5H_3NCH_2$.

**7.** Procédé selon l'une quelconque des revendications 1 à 6, dans lequel AA est la glycine, l'alanine, la leucine, l'isoleucine, la proline, l'hydroxyproline, la phénylalanine, la tyrosine, la méthionine, la norleucine, la sérine, la thréonine, la cystine, la cystéine, la tyrosine, l'acide aspartique, l'acide glutamique, l'asparagine, la glutamine, la lysine, l'arginine, l'hydroxylysine, l'ornithine, l'histidine ou le tryptophane.

**8.** Procédé selon l'une quelconque des revendications 1 à 7, dans lequel

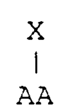

est

<table>
<tr><td>DMB<br>|<br>Gln,</td><td>TMB<br>|<br>Gln,</td><td>DMB<br>|<br>Asn,</td><td>TMB<br>|<br>Asn,</td><td></td><td></td><td></td></tr>
<tr><td>Mtr<br>|<br>Arg,</td><td>Pmc<br>|<br>Arg,</td><td>FMOC<br>|<br>His,</td><td></td><td></td><td></td><td></td></tr>
<tr><td>BOC<br>|<br>His,</td><td>TMSE-OCH$_2$<br>|<br>His,</td><td>Bum<br>|<br>His,</td><td>t-Bu<br>|<br>Ser,</td><td>Ada<br>|<br>Ser,</td><td>Bz<br>|<br>Ser,</td><td>TMSE<br>|<br>Ser,</td></tr>
<tr><td>Cp<br>|<br>Ser,</td><td>Ch<br>|<br>Ser,</td><td>t-Bu<br>|<br>Thr,</td><td>Ada<br>|<br>Thr,</td><td>Bz<br>|<br>Thr,</td><td>TMSE<br>|<br>Thr,</td><td></td></tr>
<tr><td>Cp<br>|<br>Thr,</td><td>Ch<br>|<br>Thr,</td><td>t-Bu<br>|<br>Tyr,</td><td>Ada<br>|<br>Tyr,</td><td>TMSE<br>|<br>Tyr,</td><td></td><td></td></tr>
<tr><td>Bz<br>|<br>Tyr,</td><td>Cp<br>|<br>Tyr,</td><td>Ch<br>|<br>Tyr,</td><td>t-Bu<br>|<br>Cys,</td><td>Trt<br>|<br>Cys,</td><td></td><td></td></tr>
<tr><td>Acm<br>|<br>Cys,</td><td>S-t-Bu<br>|<br>Cys,</td><td></td><td></td><td></td><td></td><td></td></tr>
<tr><td>Bz<br>|<br>Asp,</td><td>t-Bu<br>|<br>Asp,</td><td>Cp<br>|<br>Asp,</td><td>Ch<br>|<br>Asp,</td><td>Ada<br>|<br>Asp,</td><td>TMSE<br>|<br>Asp,</td><td></td></tr>
<tr><td>Bz<br>|<br>Glu,</td><td>t-Bu<br>|<br>Glu,</td><td>Cp<br>|<br>Glu,</td><td>Ch<br>|<br>Glu,</td><td>Ada<br>|<br>Glu,</td><td>TMSE<br>|<br>Glu,</td><td></td></tr>
<tr><td>Cbz<br>|<br>Lys,</td><td>Adoc<br>|<br>Lys,</td><td>CHO<br>|<br>Lys, ou</td><td>TFA<br>|<br>Lys.</td><td></td><td></td><td></td></tr>
</table>

**9.** Procédé selon l'une quelconque des revendications 1 à 8, dans lequel la chaîne latérale de AA est un groupe hydroxy(alkyle inférieur), carboxy (alkyle inférieur), mercapto(alkyle inférieur), hydroxyaryle ou hydroxycarbonyle, et X est le radical tert-butyle.

**10.** Procédé selon les revendications 1 à 9, dans lequel on utilise le stéréoisomère D ou L ou des mélanges de fluorure d'acide aminé.

**11.** Procédé selon l'une quelconque des revendications 1 à 10, dans lequel BLK est FMOC, BOC, CLIMOC, BIMOC, Dbd-TMOC, Aoc, Adoc, $ArSO_2$, Bpoc, Ddz, HCO, TFA, Teoc, le radical benzo[e]-indène-1-méthoxycarbonyle, benzo[e]indène-3-méthoxycarbonyle, Mspoc, Bspoc, Bsmoc ou Cbz.